(19) Europäisches Patentamt
European Patent Office
Office européen des brevets

(11) EP 3 149 143 B1

(12) EUROPEAN PATENT SPECIFICATION

(45) Date of publication and mention of the grant of the patent:
**06.08.2025 Bulletin 2025/32**

(21) Application number: **15724701.6**

(22) Date of filing: **28.05.2015**

(51) International Patent Classification (IPC):
***C11D 3/386*** *(2006.01)*

(52) Cooperative Patent Classification (CPC):
**C11D 3/38636; C11D 3/0036; C11D 3/0068; C12Y 301/21001; D06L 4/40**

(86) International application number:
**PCT/EP2015/061828**

(87) International publication number:
**WO 2015/181286 (03.12.2015 Gazette 2015/48)**

# (54) USE OF POLYPEPTIDE

VERWENDUNG EINES POLYPEPTIDS

UTILISATION D'UN POLYPEPTIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.05.2014 EP 14170238**
**16.06.2014 EP 14172548**
**10.02.2015 EP 15154473**

(43) Date of publication of application:
**05.04.2017 Bulletin 2017/14**

(73) Proprietor: **Novozymes A/S**
**2880 Bagsvaerd (DK)**

(72) Inventors:
- **BALTSEN, Lilian Eva Tang**
  **DK-2880 Bagsvaerd (DK)**
- **ALLESEN-HOLM, Marie**
  **DK-2880 Bagsvaerd (DK)**
- **GORI, Klaus**
  **DK-2880 Bagsvaerd (DK)**

(74) Representative: **NVS EPO Representatives**
**Krogshoejvej 36**
**2880 Bagsvaerd (DK)**

(56) References cited:
**WO-A1-2011/098579 WO-A1-2014/087011**
**US-A1- 2010 021 587 US-A1- 2013 072 413**

- **SIGNE MUNK ET AL: "Microbial survival and odor in laundry", JOURNAL OF SURFACTANTS AND DETERGENTS, vol. 4, no. 4, 1 October 2001 (2001-10-01), pages 385 - 394, XP055095395, ISSN: 1097-3958, DOI: 10.1007/s11743-001-0192-2**
- **AMBERG CAROLINE ET AL: "Odour formation ontextiles- Why dotextilesaccumulate Malodour?", 1 February 2014 (2014-02-01), pages 1 - 27, XP055928051, Retrieved from the Internet <URL:https://www.swissatest.ch/files/downloads/90d632725e685a48967925bd44cda783/Odour%20formation%20on%20textiles%20-%20Fresenius.pdf> [retrieved on 20220603]**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Reference to a Sequence Listing

This application contains a Sequence Listing in computer readable form.

### Field of the Invention

The present invention concerns the use of a polypeptide having deoxyribonuclease (DNase) activity for preventing or reducing redeposition of soil on an item during a subsequent cleaning or laundering process.

### Background of invention

Microorganisms generally live attached to surfaces in many natural, industrial, and medical environments, encapsulated by extracellular substances including biopolymers and macromolecules. The resulting layer of slime encapsulated microorganism is termed a biofilm. Biofilms are the predominant mode of growth of bacteria in the natural environment, and bacteria growing in biofilms exhibit distinct physiological properties. Compared to their planktonically grown counterparts, the bacteria in a biofilm are more resistant to antibiotics, UV irradiation, detergents and the host immune response.

It has for many years been a known problem that laundry items like shirts and blouses become more and more grey as time goes by. For normal day clothing as well as sportswear sweat and the resulting odor is a challenge. These stains usually consist of a lot of different components adhering to the textile of the clothing and can be difficult to dissolve and remove. When laundry items like T-shirts or sportswear are used, they are contacted to sweat and bacteria from the body of the user and for other kinds of dirt from the rest of the environment in which they are used. Some of these bacteria are capable of adhering to the laundry item and form a biofilm on the item. The presence of bacteria implies that the laundry items become sticky and therefore soil adheres to the sticky areas. This soil has shown difficult to remove by commercially available detergent compositions. Further, when very dirty laundry items are washed together with less dirty laundry items the dirt present in the wash liquor tend to stick to the biofilm. As a result hereof the laundry item is more "soiled" and more greyed after washing than before washing.

Sportswear is a good example because there is often soil, clay and traffic dirt on the clothes washed together with very sweaty shirts. From wash to wash the clothes become greyer and greyer and they eventually appear as developed spots. This kind of dirt is one reason why people discard their clothes. Although the problem is well known in most garments the problem is very pronounced for mixed fabrics. There is a European political desire to conserve resources for laundry which has led to their adoption of a labeling law for washing machines in the EU to exclude machines with high energy consumption. This means that cold water washing is much more prevalent in the EU and thus come to resemble the rest of the world wash circumstances better. However, the saving of energy by washing at lower temperature may lead to consumers discarding clothes and buying new because the sweat stains are not properly removed. There is an urgent need to solve the problem of removing sweat stains effectively.

International patent application WO 2011/098579 concerns bacterial deoxyribonuclease compounds and methods for biofilm disruption and prevention.

WO 2014/087011 discloses detergent compositions comprising a DNase, a washing method for textile using a DNase, and use of a DNase for reducing malodor from laundry and/or textile, for anti-redeposition and for maintaining or improving the whiteness of a textile.

Munk et al., "Microbial survival and odor in laundry", Journal of Surfactants and Detergents, vol. 4, no. 4, October 2001, pages 385-394, describes a study of the survival and distribution of microflora and adhesion of odorants during laundering at 30 or 40°C in commercial US and EU detergents.

Amberg et al., "Odour formation on textiles - Why do textiles accumulate Malodour?", presented at the 8th International Fresenius Conference ’Detergents and Cleaning products’, February 2014, is a presentation discussing why textiles accumulate malodour, with emphasis on the role of microorganisms.

### Summary of the Invention

The present invention concerns the use of a polypeptide having DNase activity for preventing or reducing redeposition of soil on an item during a subsequent cleaning or laundering process, wherein the item is a textile, and wherein no polypeptide having DNase activity is present in the subsequent cleaning or laundering process.

## Definitions

The term alkyl means a hydrocarbyl moiety which is straight or branched, saturated or unsaturated. Unless otherwise specified, alkyl moieties are preferably saturated or unsaturated with double bonds, preferably with one or two double bonds. Included in the term "alkyl" is the alkyl portion of acyl groups.

The term "automatic dishwashing composition" refers to compositions intended for cleaning dishware such as plates, cups, glasses, bowls, cutlery such as spoons, knives, forks, serving utensils, ceramics, plastics, metals, china, glass and acrylics in a dishwashing machine. The terms encompass any materials/compounds selected for domestic or industrial washing applications and the form of the product can be liquid, powder or granulate. In addition to lipase, the automatic dishwashing composition contains detergent components such as polymers, bleaching systems, bleach activators, bleach catalysts, silicates, dyestuff and metal care agents.

Allelic variant: The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

Bacterial: In the context of the present invention, the term "bacterial" in relation to polypeptide (such as an enzyme, e.g. a DNAse) refers to a polypeptide encoded by and thus directly derivable from the genome of a bacteria, where such bacteria has not been genetically modified to encode said polypeptide, e.g. by introducing the encoding sequence in the genome by recombinant DNA technology. In the context of the present invention, the term "bacterial DNAse" or "polypeptide having DNAse activity obtained from a bacterial source" or "polypeptide is of bacterial origin" thus refers to a DNAse encoded by and thus directly derivable from the genome of a bacterial species, where the bacterial species has not been subjected to a genetic modification introducing recombinant DNA encoding said DNAse. Thus, the nucleotide sequence encoding the bacterial polypeptide having DNAse activity is a sequence naturally in the genetic background of a bacterial species. The bacterial polypeptide having DNAse activity encoding by such sequence may also be referred to a wildtype DNAse (or parent DNAse). Polypeptides having DNase activity may be substantially homologous to a bacterial DNase. In the context of the present invention, the term "substantially homologous" denotes a polypeptide having DNase activity which is at least 80%, preferably at least 85%, more preferably at least 90%, more preferably at least 95%, even more preferably at least 96%, 97%, 98%, and most preferably at least 99% identical to the amino acid sequence of a selected bacterial DNase.

Biofilm: A biofilm is any group of microorganisms in which cells stick to each other or stick to a surface, such as a textile, dishware or hard surface or another kind of surface. These adherent cells are frequently embedded within a self-produced matrix of extracellular polymeric substance (EPS). Biofilm EPS is a polymeric conglomeration generally composed of extracellular DNA, proteins, and polysaccharides. Biofilms may form on living or non-living surfaces. The microbial cells growing in a biofilm are physiologically distinct from planktonic cells of the same organism, which, by contrast, are single-cells that may float or swim in a liquid medium.

Bacteria living in a biofilm usually have significantly different properties from planktonic bacteria of the same species, as the dense and protected environment of the film allows them to cooperate and interact in various ways. One benefit of this environment for the microorganisms is increased resistance to detergents and antibiotics, as the dense extracellular matrix and the outer layer of cells protect the interior of the community.

On laundry, biofilm-producing bacteria can be found among the following species: Acinetobacter sp., Aeromicrobium sp., Brevundimonas sp., Microbacterium sp., Micrococcus luteus, Pseudomonas sp., Staphylococcus epidermidis, and Stenotrophomonas sp. On hard surfaces, biofilm producing bacteria can be found among the following species: Acinetobacter sp., Aeromicrobium sp., Brevundimonas sp., Microbacterium sp., Micrococcus luteus, Pseudomonas sp., Staphylococcus epidermidis, and Stenotrophomonas sp In one embodiment the biofilm producing strain is *Brevundimonas* sp.. In one embodiment the biofilm producing strain is *Pseudomonas alcaliphila* or *Pseudomonas fluorescens.*

cDNA: The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

Coding sequence: The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

Color difference (L value): A Lab color space is a color-opponent space with dimension L for lightness. L value, L* represents the darkest black at L* = 0, and the brightest white at L* = 100. In the context of the present invention L value is also referred to as color difference. The color difference method is used in the examples of the present patent application.

Control sequences: The term "control sequences" means nucleic acid sequences necessary for expression of a

polynucleotide encoding a mature polypeptide of the present invention. Each control sequence may be native (*i.e.*, from the same gene) or foreign (*i.e.*, from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. As a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

By the term "deep cleaning" is meant disruption, reducing or removal of a biofilm or components of a biofilm such as polysaccharides, proteins, DNA, soil or other components present in the biofilm. Any cleaning which is does not disrupt, reduce or remove biofilm is not deep cleaning.

Detergent components: the term "detergent components" is defined herein to mean the types of chemicals which can be used in detergent compositions. Examples of detergent components are alkalis, surfactants, hydrotropes, builders, co-builders, chelators or chelating agents, bleaching system or bleach components, polymers, fabric hueing agents, fabric conditioners, foam boosters, suds suppressors, dispersants, dye transfer inhibitors, fluorescent whitening agents, perfume, optical brighteners, bactericides, fungicides, soil suspending agents, soil release polymers, anti-redeposition agents, enzyme inhibitors or stabilizers, enzyme activators, antioxidants and solubilizers.

Detergent Composition: The term "detergent composition" refers to compositions that find use in the removal of undesired compounds from items to be cleaned, such as textiles. The detergent composition may be used to e.g. clean textiles for both household cleaning and industrial cleaning. The terms encompass any materials/compounds selected for the particular type of cleaning composition desired and the form of the product (e.g., liquid, gel, powder, granulate, paste, or spray compositions) and includes, but is not limited to, detergent compositions (e.g., liquid and/or solid laundry detergents and fine fabric detergents; fabric fresheners; fabric softeners; and textile and laundry pre-spotters/pretreatment). In addition to containing the enzyme of the invention, the detergent formulation may contain one or more additional enzymes (such as proteases, amylases, lipases, cutinases, cellulases, endoglucanases, xyloglucanases, pectinases, pectin lyases, xanthanases, peroxidaes, haloperoxygenases, catalases and mannanases, or any mixture thereof), and/or detergent adjunct ingredients such as surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anti-corrosion agents, enzyme inhibitors or stabilizers, enzyme activators, transferase(s), hydrolytic enzymes, oxido reductases, bluing agents and fluorescent dyes, antioxidants, and solubilizers.

Dishware: The term "dishware" is intended to mean any form of kitchen utensil, dinner set or tableware such as but not limited to pans, crockery, cutlery, such as plates, cups, knives, forks, spoons, porcelain etc.

Dish washing composition: The term "dish washing composition" refers to compositions comprising detergent components, which composition is suitable and intended for cleaning dishware, table ware, crockery, pots, pans, cutlery. The dish washing composition can be used for cleaning hard surfaces areas in kitchens.

DNase (deoxyribonuclease): The term "DNase" means a polypeptide with DNase activity activity that catalyzes the hydrolytic cleavage of phosphodiester linkages in the DNA backbone, thus degrading DNA. For purposes of the present invention, DNase activity is determined according to the procedure described in Assay I. The polypeptides may have at least 20%, e.g., at least 40%, at least 50%, at least 60%, at least 70%, at least 80%, at least 90%, at least 95%, or at least 100% of the DNase activity of the mature polypeptide of SEQ ID NO: 2. For purposes of the present invention, DNase activity imay be determined according to the procedure described in the Assay I. The DNAse activity of a polypeptide may be at least 105%, e.g., at least 110%, at least 120%, at least 130%, at least 140%, at least 160%, at least 170%, at least 180%, or at least 200% with reference to the DNase activity of the mature polypeptide of SEQ ID NO: 2, a polypeptide comprising or consisting of the sequence set forth in SEQ ID NO: 3, a polypeptide comprising or consisting of the sequence set fort in SEQ ID NO: 5, a polypeptide comprising or consisting of the mature polypeptide of SEQ ID NO: 6, a polypeptide comprising or consisting of the mature polypeptide of SEQ ID NO: 7 or a polypeptide comprising or consisting of the mature polypeptide of SEQ ID NO: 8.

Enzyme Detergency benefit: The term "enzyme detergency benefit" is defined herein as the advantageous effect an enzyme may add to a detergent compared to the same detergent without the enzyme. Important detergency benefits which can be provided by enzymes are stain removal with no or very little visible soils after washing and/or cleaning, prevention or reduction of redeposition of soils released in the washing process (an effect that also is termed anti-redeposition), restoring fully or partly the whiteness of textiles which originally were white but after repeated use and wash have obtained a greyish or yellowish appearance (an effect that also is termed whitening). Textile care benefits, which are not directly related to catalytic stain removal or prevention of redeposition of soils, are also important for enzyme detergency benefits. Examples of such textile care benefits are prevention or reduction of dye transfer from one fabric to another fabric or another part of the same fabric (an effect that is also termed dye transfer inhibition or anti-backstaining), removal of protruding or broken fibers from a fabric surface to decrease pilling tendencies or remove already existing pills or fuzz (an effect that also is termed anti-pilling), improvement of the fabric-softness, colour clarification of the fabric and

removal of particulate soils which are trapped in the fibers of the fabric or garment. Enzymatic bleaching is a further enzyme detergency benefit where the catalytic activity generally is used to catalyze the formation of bleaching components such as hydrogen peroxide or other peroxides.

Expression: The term "expression" includes any step involved in the production of a polypeptide including, but not limited to, transcription, post-transcriptional modification, translation, post-translational modification, and secretion.

Expression vector: The term "expression vector" means a linear or circular DNA molecule that comprises a polynucleotide encoding a polypeptide and is operably linked to control sequences that provide for its expression.

Fragment: The term "fragment" means a polypeptide having one or more (*e.g.*, several) amino acids absent from the amino and/or carboxyl terminus of a mature polypeptide or domain; wherein the fragment has DNase activity. In one aspect, a fragment contains at least 206 amino acid residues (*e.g.*, amino acids 1 to 206 of SEQ ID NO: 2), at least 205 amino acid residues (*e.g.*, amino acids 2 to 206 of SEQ ID NO: 2), or at least 204 amino acid residues (*e.g.*, amino acds 3 to 206 of SEQ ID NO: 2). In one aspect, a fragment contains at least 139 amino acid residues (*e.g.*, amino acids 50 to 188 of SEQ ID NO: 5), or at least 188 amino acid residues (*e.g.*, amino acids 1 to 188 of SEQ ID NO: 5).

Fungal: In the context of the present invention the term "fungal" in relation to polypeptide (such as an enzyme, e.g. a DNAse) refers to a polypeptide encoded by and thus directly derivable from the genome of a fungus, where such fungus has not been genetically modified to encode said polypeptide, e.g. by introducing the encoding sequence in the genome by recombinant DNA technology. In the context of the present invention, the term "fungal DNAse" or "polypeptide having DNAse activity obtained from a fungal source" or "polypeptide is of fungal origin" thus refers to a DNAse encoded by and thus directly derivable from the genome of a fungal species, where the fungal species has not been subjected to a genetic modification introducing recombinant DNA encoding said DNAse. Thus, the nucleotide sequence encoding the fungal polypeptide having DNAse activity is a sequence naturally in the genetic background of a fungal species. The fungal polypeptide having DNAse activity encoding by such sequence may also be referred to a wildtype DNAse (or parent DNAse). Polypeptides having DNase activity may be substantially homologous to a fungal DNase. In the context of the present invention, the term "substantially homologous" denotes a polypeptide having DNase activity which is at least 80%, preferably at least 85%, more preferably at least 90%, more preferably at least 95%, even more preferably at least 96%, 97%, 98%, and most preferably at least 99% identical to the amino acid sequence of a selected fungal DNase.

Hard surface: The term "hard surface" is defined herein as a surface that does not absorb water. In particular, the term "hard surface" does not encompass a textile or fabric.

Items having a hard surface and falling within the intended meaning of the term therefore include household surfaces, surfaces in hospitals/institutions and outdoor surfaces such as floors, walls, roofs etc. as well as surfaces of hard objects such as cars (car wash) tables and other furniture, and dishware. Dishware includes but is not limited to crockery such as plates, cups, glasses, bowls, cutlery such as spoons, knives, forks, serving utensils, and other items made from ceramics, plastics, metals, china, glass and acrylics, etc.

Hard surface detergent composition: The term "hard surface detergent composition" refers to compositions comprising detergent components, which composition is suitable and intended for cleaning hard surfaces areas.

Host cell: The term "host cell" means any cell type that is susceptible to transformation, transfection, transduction, or the like with a nucleic acid construct or expression vector comprising a polynucleotide. The term "host cell" encompasses any progeny of a parent cell that is not identical to the parent cell due to mutations that occur during replication.

Isolated: The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (e.g., recombinant production in a host cell; multiple copies of a gene encoding the substance; and use of a stronger promoter than the promoter naturally associated with the gene encoding the substance). An isolated substance may be present in a fermentation broth sample; e.g. a host cell may be genetically modified to express a polypeptide. The fermentation broth from that host cell will comprise the isolated polypeptide.

Laundering: The term "laundering" relates to both household laundering and industrial laundering and means the process of treating textiles with a solution containing a cleaning or detergent composition. The laundering process can for example be carried out using e.g. a household or an industrial washing machine or can be carried out by hand.

By the term "malodor" is meant an odor which is not desired on clean items. The cleaned item should smell fresh and clean without malodors adhered to the item. One example of malodor is compounds with an unpleasant smell, which may be produced by microorganisms. Another example is unpleasant smells can be sweat or body odor adhered to an item which has been in contact with human or animal. Another example of malodor can be the odor from spices, which sticks to items for example curry or other exotic spices which smells strongly. One way of measuring the ability of an item to adhere malodor is by using Assay II as in examples 5 and 6.

Mature polypeptide: The term "mature polypeptide" means a polypeptide in its final form following translation and any post-translational modifications, such as N-terminal processing, C-terminal truncation, glycosylation, phosphorylation, etc. In one aspect, the mature polypeptide is amino acids 1 to 206 of SEQ ID NO: 2 and amino acids -37 to -16 of SEQ ID NO: 2 are a signal peptide and amino acids -15 to -1 of SEQ ID NO: 2 are a propeptide. In one aspect, the mature polypeptide is amino acids 1 to 188 of SEQ ID NO: 5 and amino acids -17 to -1 of SEQ ID NO: 2 are a signal peptide. In one aspect, the mature polypeptide is amino acids 1 to 110 of SEQ ID NO: 6, the mature polypeptide is amino acids 1 to 109 of SEQ ID NO: 7 or the mature polypeptide is amino acids 1 to 206 of SEQ ID NO: 8. It is known in the art that a host cell may produce a mixture of two of more different mature polypeptides (*i.e.*, with a different C-terminal and/or N-terminal amino acid) expressed by the same polynucleotide. It is also known in the art that different host cells process polypeptides differently, and thus, one host cell expressing a polynucleotide may produce a different mature polypeptide (*e.g.*, having a different C-terminal and/or N-terminal amino acid) as compared to another host cell expressing the same polynucleotide. In one aspect, a mature polypeptides contains up to 206 amino acid residues and of SEQ ID NO: 2, SEQ ID NO: 3 or SEQ ID NO: 8 (*e.g.,* amino acids 1 to 206 of SEQ ID NO: 2), or up to 204 amino acid residues (*e.g.*, amino acids 3 to 206 of SEQ ID NO: 2).

Mature polypeptide coding sequence: The term "mature polypeptide coding sequence" means a polynucleotide that encodes a mature polypeptide having DNase activity. In one aspect, the mature polypeptide coding sequence is nucleotides 1 to 242, 309 to 494, 556 to 714 and 766 to 907 of SEQ ID NO: 1. In one aspect, the mature polypeptide coding sequence is nucleotides 52 to 864 of SEQ ID NO: 4, where three introns are predicted in the sequence in amino acids in position 76-164, 289-362 and 520-615 of SEQ ID NO: 4. A secretion signal is present at amino acids in positions 1-51 of SEQ ID NO: 4.

Nucleic acid construct: The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

Operably linked: The term "operably linked" means a configuration in which a control sequence is placed at an appropriate position relative to the coding sequence of a polynucleotide such that the control sequence directs expression of the coding sequence.

Sequence identity: The relatedness between two amino acid sequences or between two nucleotide sequences is described by the parameter "sequence identity". For purposes of the present invention, the sequence identity between two amino acid sequences imay be determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), pref-erably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present invention, the sequence identity between two deoxyribonucleotide sequences imay be determined using the Needleman-Wunsch algorithm (Needleman and Wunsch, 1970, supra) as implemented in the Needle program of the EMBOSS package (EM-BOSS: The European Molecular Biology Open Software Suite, Rice et al., 2000, supra), prefer-ably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment - Total Number of Gaps in Alignment).

Stringency conditions: The term "very low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 45°C.

The term "low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 50°C.

The term "medium stringency conditions" means for probes of at least 100 nucleotides in length, prehybridization and hybridization at 42°C in 5X SSPE, 0.3% SDS, 200 micrograms/ml sheared and denatured salmon sperm DNA, and

35% formamide, following standard Southern blotting procedures for 12 to 24 hours. The carrier material is finally washed three times each for 15 minutes using 2X SSC, 0.2% SDS at 55°C.

The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 60°C.

The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 65°C.

The term "very high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 70°C.

Subsequence: The term "subsequence" means a polynucleotide having one or more (*e.g.*, several) nucleotides absent from the 5' and/or 3' end of a mature polypeptide coding sequence; wherein the subsequence encodes a fragment having DNase activity. In one aspect, a subsequence contains at least 796 nucleotides (*e.g.*, nucleotides 112 to 907 of SEQ ID NO: 1), at least 793 nucleotides (*e.g.*, nucleotides 115 to 907 of SEQ ID NO: 1), or at least 790 nucleotides (*e.g.*, nucleotides 118 to 907 of SEQ ID NO: 1). In one aspect, a subsequence contains at least 587 nucleotides (*e.g.*, nucleotides 278 to 864 of SEQ ID NO: 4), at least 650 nucleotides (*e.g.*, nucleotides 215 to 864 of SEQ ID NO: 4), or at least 816nucleotides (*e.g.*, nucleotides 52 to 864 of SEQ ID NO: 4).

Textile: The term "textile" means any textile material including yarns, yarn intermediates, fibers, non-woven materials, natural materials, synthetic materials, and any other textile material, fabrics made of these materials and products made from fabrics (e.g., garments and other articles). The textile or fabric may be in the form of knits, wovens, denims, non-wovens, felts, yarns, and towelling. The textile may be cellulose based such as natural cellulosics, including cotton, flax/linen, jute, ramie, sisal or coir or manmade cellulosics (e.g. originating from wood pulp) including viscose/rayon, cellulose acetate fibers (tricell), lyocell or blends thereof. The textile or fabric may also be non-cellulose based such as natural polyamides including wool, camel, cashmere, mohair, rabbit and silk or synthetic polymers such as nylon, aramid, polyester, acrylic, polypropylene and spandex/elastane, or blends thereof as well as blends of cellulose based and non-cellulose based fibers. Examples of blends are blends of cotton and/or rayon/viscose with one or more companion material such as wool, synthetic fiber (e.g. polyamide fiber, acrylic fiber, polyester fiber, polyvinyl chloride fiber, polyurethane fiber, polyurea fiber, aramid fiber), and/or cellulose-containing fiber (e.g. rayon/viscose, ramie, flax/linen, jute, cellulose acetate fiber, lyocell). Fabric may be conventional washable laundry, for example stained household laundry. When the term fabric or garment is used it is intended to include the broader term textiles as well.

Variant: The term "variant" means a polypeptide having same activity as the parent enzyme comprising an alteration, *i.e.*, a substitution, insertion, and/or deletion, at one or more (*e.g.*, several) positions. A substitution means replacement of the amino acid occupying a position with a different amino acid; a deletion means removal of the amino acid occupying a position; and an insertion means adding an amino acid adjacent to and immediately following the amino acid occupying a position. In the context of the present invention, a variant of an identified DNAse has the enzymatic activity of the parent, *i.e.* the capacity of catalyzing the hydrolytic cleavage of phosphodiester linkages in the DNA backbone (deoxyribonuclease activity). In one embodiment, the deoxyribonuclease activity of the variant is increased with reference to the parent DNAse, e.g. the mature polypeptide of SEQ ID NO: 2.

Wash cycle: The term "wash cycle" is defined herein as a washing operation wherein textiles are immersed in the wash liquor, mechanical action of some kind is applied to the textile in order to release stains and to facilitate flow of wash liquor in and out of the textile and finally the superfluous wash liquor is removed. After one or more wash cycles, the textile is generally rinsed and dried.

Wash liquor: The term "wash liquor" is intended to mean the solution or mixture of water and detergents optionally including enzymes used for laundrering textiles, for hard surface cleaning or for dishwashing.

## Detailed Description of the Invention

As described above, it has for many years been a known problem that laundry items like shirts and blouses lose whiteness or become more and more grey as time goes by. It is believed that part of this problem is due to redeposition of soil during washing so that when very dirty laundry items are washed together with less dirty laundry items the dirt present in the wash liquor tends to stick to the laundry items. As a result the laundry item becomes more "soiled" and greyer after washing than it was before washing. The inventors have surprisingly found that the use of polypeptides having DNase activity can be used for preventing or reducing this redeposition of soil during a subsequent cleaning or laundering process. The item can be a textile.

The inventors have found that when contacting an item such as a laundry item with a polypeptide having DNase activity, the laundry item is cleaner and less grey after a subsequent wash without DNase present than a similar laundry item, which has not been contacted with a polypeptide having DNase activity, see for example examples 1 and 2. The inventors have found that a polypeptide having DNase activity can be used for maintaining or improving whiteness of the item, see for example examples 1 and 2.

Further, the present invention concerns the use of a polypeptide having DNase activity for preventing or reducing adherence of soil to an item during a subsequent cleaning or laundering process where no DNase is used, see examples 1-7.

The inventors have found that the use of a polypeptide having DNase activity can prevent or reduce malodour from an item such as textile (see Example 3 til 7). The malodour may be caused by E-2-nonenal.

In conventional laundry methods, the malodor may even survive the laundry process and the drying process. This has the effect that malodor can be sensed when the textile is used. This is not very pleasant for the user of the textile, i.e. when wearing sportswear that smells even before the sport activity has started. This can be embarrassing for the user of the textile who chooses to buy new sportswear instead of the bad smelling sportswear. By the use of the present invention this is avoided and the environment is thereby saved for use of limited resources such as raw material for new textiles, water, energy and pollution of the environment. The amount of E-2-nonenal present on a dry item after wash may be prevented, reduced or removed.

One advantage is that this malodor does not appear from the wet laundry items i.e. when opening the washing machine. This makes the washing process a more attractive task both in domestic and industrial applications.

Another advantage is that, when receiving the wet laundry directly from the washing machine or wash liquor, the laundry items do not have a malodor and are perceived as clean. Thereby time, money and energy for an unnecessary second or even third wash is saved. This is of huge advantage for the environment.

The item is contacted with a polypeptide having DNase activity. The polypeptide having DNase activity can for example be sprayed onto the item. The deposition of soil can thereby be reduced or removed by spraying the polypeptide onto for example carpets, floors or sheets.

Another way of preventing or reducing redeposition is contacting the item with a liquid solution comprising a polypeptide having DNase activity. The item can be contacted to the liquid solution for example by immersing the item into the liquid solution. The liquid solution can be a wash liquor and the item may be washed at the same time. This embodiment of the invention is preferred for textiles such as laundry textiles which can be washed at the same time. In one embodiment of the invention the textile is newly produced and is pretreated by washing, immersing or impregnating the textile with the polypeptide having DNase activity after production of the textile. By impregnating the item, e.g. the textile the item may be more resistant to deposition of soil and/or odour on the item.

The wash liquor can also be used for washing floors or other hard surfaces which need anti-redeposition treatment.

The liquid solution can also be an impregnation liquid which liquid prevents the item from building up dirt during a subsequent cleaning or laundering process. The liquid solution for impregnation may serve as detergent and anti-redeposition solution at the same time

The polypeptide having DNase activity is used for cleaning or laundering the item at least one time before the subsequent cleaning or laundering process, wherein the subsequent laundering process does not comprise the use of a DNase

In one embodiment, the polypeptide having DNase activity is used for cleaning or laundering the item at least two times, three times, four times, five times, six times, seven times, eight times, nine times or ten times before the subsequent cleaning or laundering process. In one embodiment, the polypeptide having DNase activity is used for cleaning or laundering the item at least two times, three times, four times, five times, six times, seven times, eight times, nine times or ten times before the subsequent cleaning or laundering process where no polypeptide having DNase activity is used.

A detergent composition comprising a polypeptide having deoxyribonuclease (DNase) activity and a surfactant may be one wherein the composition fullfils at least one of a) or b):

a) the composition further comprises a odor control agent; and/or
b) the surfactant is not a cationic surfactant.

The odor control agent is selected from the group consisting of cyclodextrins and mixtures thereof, odor blockers, reactive aldehydes, flavanoids, metallic salts, zeolites, activated carbon, hydrophobically modified malodour control polymers (HMP's), derivativs of isothiazolinone such as benzisothiazolinone, and/or volatile aldehydes.

The composition may comprise benzisothiazolinone and be used for preventing, reducing or removing biofilm, for reducing malodour, for improving the whiteness and/or for reducing redeposition. Benzisothiazolinone is a widely used biocide and belongs to the group of isothiazolinones.

The surfactant may be a non-ionic surfactant, an anionic surfactant, a zwitterionic surfactant or a semipolar

surfactant.

The surfactant can be an anionic surfactant selected from the group consisting of: sulfates and sulfonates, such asl inear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof.

The amount of the anionic surfactant is from about 1% to about 40% by weight, such as from about 5% to about 30%, including from about 5% to about 15%, or from about 15% to about 20%, or from about 20% to about 25%.

The surfactant can be a non-ionic surfactant selected from the group consisting of alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), SPAN™, TWEEN™ and combinations thereof

The amount of the non-ionic surfactant is from about 0.2% to about 40% by weight of a nonionic surfactant, for example from about 0.5% to about 30%, in particular from about 1% to about 20%, from about 3% to about 10%, such as from about 3% to about 5%, from about 8% to about 12%, or from about 10% to about 12%.

The surfactant can be a semipolar surfactant selected from the group consisting of amine oxides (AO) such as alkyldimethylamineoxide, N-(coco alkyl)-N,N-dimethylamine oxide and N-(tallow-alkyl)-N,N-bis(2-hydroxyethyl)amine oxide, and combinations thereof.

The surfactant can be a zwitterionic surfactant selected from the group consisting of betaines such as alkyldimethylbetaines, sulfobetaines.

The surfactant may be selected from the group consisting of sodium alcoholethoxy sulfate, linear alkylbenzene sulfonate, sodium fatty acid, sodium alkyl sulfate, lauramine oxide, linear alkylbenzene sulfonate (MEA salt), linear alkylbenzene sulfonate (sodium salt), alcohol ethoxylate.

The present detergent composition can be used for preventing or reducing redeposition of soil on an item during a subsequent cleaning or laundering process and for preventing or reducing malodour.

The composition can further comprise builders, flocculating aid, chelating agents, dye transfer inhibitors, enzymes, enzyme stabilizers, enzyme inhinitors, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, builders and co-builders, fabric huing agents, anti-foaming agents, dispersants, processing aids, and/or pigments.

The composition can comprise one or more enzymes selected from the group consisting of proteases, lipases, cutinases, amylases, carbohydrases, cellulases, pectinases, mannanases, arabinases, galactanases, xylanases and oxidases.

The composition may be a detergent composition suitable for laundering, dish washing or hard surface cleaning.

A liquid detergent composition may comprise a surfactant and a detergent and a detergent builder in a total concentration of at least 3% by weight, and a detergent enzyme containing microcapsule, wherein the membrane of the microcapsule is produced by cross-linking of a polybranched polyamine having a molecular weight of more than 1 kDa. The inventors have found that encapsulating enzymes in a microcapsule with a semipermeable membrane, and having a water activity inside these capsules (prior to addition to the liquid detergent) higher than in the liquid detergent, the capsules will undergo a (partly) collapse when added to the detergent (water is oozing out), thus leaving a more concentrated and more viscous enzyme containing interior in the capsules. The collapse of the membrane may also result in a reduced permeability. This can be further utilized by addition of stabilizers/polymers, especially ones that are not permeable through the membrane. The collapse and resulting increase in viscosity will reduce/hinder the diffusion of hostile components (e.g., surfactants or sequestrants) into the capsules, and thus increase the storage stability of the enzyme in the liquid detergent. Components in the liquid detergent that are sensitive to the enzyme (e.g., components that act as substrate for the enzyme) are also protected against degradation by the enzyme. During wash the liquid detergent is diluted by water, thus increasing the water activity. Water will now diffuse into the capsules (osmosis). The capsules will swell and the membrane will either become permeable to the enzyme so they can leave the capsules, or simply burst and in this way releasing the enzyme. The concept is very efficient in stabilizing the enzymes against hostile components in liquid detergent, and vice versa also protects enzyme sensitive components in the liquid detergent from enzymes.

Examples of detergent components which are sensitive to, and can be degraded by, enzymes include (relevant

enzyme in parenthesis): xanthan gum (xanthanase), polymers with ester bonds (lipase), hydrogenated castor oil (lipase), perfume (lipase), methyl ester sulfonate surfactants (lipase), cellulose and cellulose derivatives (e.g. CMC) (cellulase), and dextrin and cyclodextrin (amylase).

Also sensitive detergent ingredients can be encapsulated, and thus stabilized, in the microcapsules. Sensitive detergent ingredients are prone to degradation during storage. Such detergent ingredients include bleaching compounds, bleach activators, perfumes, polymers, builder, surfactants, etc.

Generally, the microcapsules can be used to separate incompatible components/compounds in detergents.

Addition of the microcapsules to detergents can be used to influence the visual appearance of the detergent product, such as an opacifying effect (small microcapsules) or an effect of distinctly visible particles (large microcapsules). The microcapsules may also be colored.

The microcapsules can be used to reduce the enzyme dust levels during handling and processing of enzyme products.

Unless otherwise indicated, all percentages are indicated as percent by weight (% w/w) throughout the application.

Microcapsule: The microcapsules are typically produced by forming water droplets into a continuum that is non-miscible with water - i.e., typically by preparing a water-in-oil emulsion - and subsequently formation of the membrane by interfacial polymerization via addition of a cross-linking agent. After eventual curing the capsules can be harvested and further rinsed and formulated by methods known in the art. The capsule formulation is subsequently added to the detergent.

The payload, the major membrane constituents and eventual additional component that are to be encapsulated are found in the water phase. In the continuum is found components that stabilize the water droplets towards coalescence (emulsifiers, emulsion stabilizers, surfactants etc.) and the cross linking agent is also added via the continuum.

The emulsion can be prepared be any methods known in the art, e.g., by mechanical agitation, dripping processes, membrane emulsification, microfluidics, sonication etc. In some cases simple mixing of the phases automatically will result in an emulsion, often referred to as self-emulsification. Using methods resulting in a narrow size distribution is an advantage.

The cross-linking agent(s) is typically subsequently added to the emulsion, either directly or more typically by preparing a solution of the crosslinking agent in a solvent which is soluble in the continuous phase. The emulsion and cross-linking agent or solution hereof can be mixed by conventional methods used in the art, e.g., by simple mixing or by carefully controlling the flows of the emulsion and the cross-linking agent solution through an in-line mixer.

In some cases, curing of the capsules is needed to complete the membrane formation. Curing is often simple stirring of the capsules for some time to allow the interfacial polymerization reaction to end. In other cases the membrane formation can be stopped by addition of reaction quencher.

The capsules may be post modified, e.g., by reacting components onto the membrane to hinder or reduce flocculation of the particles in the detergent as described in WO 99/01534.

The produced capsules can be isolated or concentrated by methods known in the art, e.g., by filtration, centrifugation, distillation or decantation of the capsule dispersion.

The resulting capsules can be further formulated, e.g., by addition of surfactants to give the product the desired properties for storage, transport and later handling and addition to the detergent. Other microcapsule formulation agents include rheology modifiers, biocides (e.g., Proxel), acid/base for adjustment of pH (which will also adjust inside the microcapsules), and water for adjustment of water activity.

The capsule forming process may include the following steps:

- Preparation of the initial water and oil phase(s),
- Forming a water-in-oil emulsion,
- Membrane formation by interfacial polymerization,
- Optional post modification,
- Optional isolation and/or formulation,
- Addition to detergent.

The process can be either a batch process or a continuous or semi-continuous process.

A microcapsule is a small aqueous sphere with a uniform membrane around it. The material inside the microcapsule is referred to as the core, internal phase, or fill, whereas the membrane is sometimes called a shell, coating, or wall. The microcapsules have diameters between 0.5 μm and 2 millimeters. Preferably, the mean diameter of the microcapsules is in the range of 1 μm to 1000 μm, more preferably in the range of 5 μm to 500 μm, even more preferably in the range of 10 μm to 500 μm, even more preferably in the range of 50 μm to 500 μm, and most preferably in the range of 50 μm to 200 μm. Alternatively, the diameter of the microcapsules is in the range of 0.5 μm to 30 μm; or in the range of 1 μm to 25 μm. The diameter of the microcapsule is measured in the oil phase after polymerization is complete.

The diameter of the capsule may change depending on the water activity of the surrounding chemical environment.

Microencapsulation of enzymes may be carried out by interfacial polymerization, wherein the two reactants in a polymerization reaction meet at an interface and react rapidly. The basis of this method is a reaction of a polyamine with an acid derivative, usually an acid halide, acting as a crosslinking agent. The polyamine is preferably substantially water-soluble (when in free base form). Under the right conditions, thin flexible membranes form rapidly at the interface. One way of carrying out the polymerization is to use an aqueous solution of the enzyme and the polyamine, which are emulsified with a non-aqueous solvent (and an emulsifier), and a solution containing the acid derivative is added. An alkaline agent may be present in the enzyme solution to neutralize the acid formed during the reaction. Polymer (polyamide) membranes form instantly at the interface of the emulsion droplets. The polymer membrane of the microcapsule is typically of a cationic nature, and thus bind/complex with compounds of an anionic nature.

The diameter of the microcapsules is determined by the size of the emulsion droplets, which is controlled, for example by the stirring rate.

Emulsion: An emulsion is a temporary or permanent dispersion of one liquid phase within a second liquid phase. The second liquid is generally referred to as the continuous phase. Surfactants are commonly used to aid in the formation and stabilization of emulsions. Not all surfactants are equally able to stabilize an emulsion. The type and amount of a surfactant needs to be selected for optimum emulsion utility especially with regard to preparation and physical stability of the emulsion, and stability during dilution and further processing. Physical stability refers to maintaining an emulsion in a dispersion form. Processes such as coalescence, aggregation, adsorption to container walls, sedimentation and creaming, are forms of physical instability, and should be avoided. Examples of suitable surfactants are described in WO 97/24177, page 19-21; and in WO 99/01534.

Emulsions can be further classified as either simple emulsions, wherein the dispersed liquid phase is a simple homogeneous liquid, or a more complex emulsion, wherein the dispersed liquid phase is a heterogeneous combination of liquid or solid phases, such as a double emulsion or a multiple-emulsion. For example, a water-in-oil double emulsion or multiple emulsion may be formed wherein the water phase itself further contains an emulsified oil phase; this type of emulsion may be specified as an oil-in-water-in oil (o/w/o) emulsion. Alternatively, a water-in-oil emulsion may be formed wherein the water phase contains a dispersed solid phase often referred to as a suspension-emulsion. Other more complex emulsions can be described. Because of the inherent difficulty in describing such systems, the term emulsion is used to describe both simple and more complex emulsions without necessarily limiting the form of the emulsion or the type and number of phases present

Polyamine: The rigidity/flexibility and permeability of the membrane is mainly influenced by the choice of polyamine. The polyamine is a polybranched polyamine. Each branch, preferably ending with a primary amino group serves as a tethering point in the membrane network, thereby giving the favorable properties . A polybranched polyamine is a polyamine having more than two branching points and more than two reactive amino groups (capable of reacting with the crosslinking agent, i.e., primary and secondary amino groups). The polybranched polyamine is used as starting material when the emulsion is prepared - it is not formed in situ from other starting materials. To obtain the attractive properties , the polybranched structure of the polyamine must be present as starting material.

There is a close relation between number of branching points and number of primary amines, since primary amines will always be positioned at the end of a branch: A linear amine can only contain two primary amines. For each branching point hypothetically introduced in such a linear di-amine will allow one or more primary amine(s) to be introduced at the end of the introduced branch(es). In this context we understand the primary amino group as part of the branch, i.e., the endpoint of the branch. For example, we consider both tris(2-aminoethyl)amine and 1,2,3-propanetriamine as molecules having one branching point. The polyamine has at least four primary amines. Branching points can be introduced from an aliphatic hydrocarbon chain as in the previously stated examples or from unsaturated carbon bonds, such as in, e.g., 3,3'-diaminobenzidine, or from tertiary amino groups, such as in N,N,N',N'-tetrakis-(2-aminoethyl) ethylenediamine.

In addition to the number of branching points, we have found that the compactness of the reactive amino groups is of high importance. A substance such as, e.g., N,N,N',N'-tetrakis-(12-aminododecyl)ethylenediamine would not be suitable. Neither would a peptide or protein, such as an enzyme, be suitable for membrane formation. Thus, the polybranched polyamine is not a peptide or protein.

In an embodiment, the reactive amino groups constitute at least 15% of the molecular weight of the polybranched polyamine, such as more than 20%, or more than 25%. Preferably, the molecular weight of the polybranched polyamine is at least 1 kDa; more preferably, the molecular weight of the polybranched polyamine is at least 1.3 kDa.

In a preferred embodiment, the polybranched polyamine is a polyethyleneimine (PEI), and modifications thereof, having more than two branching points and more than two reactive amino groups; wherein the reactive amino groups constitute at least 15% of the molecular weight of the PEI, such as more than 20%, or more than 25%. Preferably, the molecular weight of the PEI is at least 1 kDa.

Combinations of different polybranched polyamines may be used for preparing the microcapsule.

The advantageous properties (e.g., enzyme storage stability, reduced enzyme leakage, reduced in-flux of

detergent ingredients) of the microcapsule may be improved by adding one or more small amines with a molecular weight of less than 1 kDa. The small amine is preferably substantially water-soluble (when in free base form) and can be a material such as ethylene diamine, hexamethylene diamine, hexane diamine, diethylene tetramine, ethylene tetramine, diamino benzene, piperazine, tetramethylene pentamine or, preferably, diethylene triamine (DETA). The small amines may be added in an amount of up to 50%, preferably up to 40%, up to 30%, up to 20%, up to 10%, or up to 5%, by weight of the total content of small amine and polybranched polyamine, when preparing the microcapsule .

Crosslinking agent: The crosslinking agent is a molecule with at least two groups/sites capable of reacting with amines to form covalent bonds.

The crosslinking agent is preferably oil soluble and can be in the form of an acid anhydride or acid halide, preferably an acid chloride. For example, it can be adipoyl chloride, sebacoyl chloride, dodecanedioc acid chloride, phthaloyl chloride, terephthaloyl chloride, isophthaloyl chloride, or trimesoyl chloride; but preferably, the crosslinking agent is terephthaloyl chloride or trimesoyl chloride.

The composition or the polypeptide having DNase activity can be contacted with the item for example by spraying, coating, impregnating, washing or immersing the item with the composition or the liquid solution. The item may be contacted to the item for a short period of time such as a 1-60 seconds or for a longer period of time such as 1-60 minutes or even longer such as 1-12 hours.

The liquid solution can further comprise surfactants, builders, flocculating aid, chelating agents, dye transfer inhibitors, enzymes, enzyme stabilizers, enzyme inhinitors, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, builders and co-builders, fabric huing agents, anti-foaming agents, dispersants, processing aids, and/or pigments.

In one embodiment, the liquid solution further comprises one or more enzymes selected from the group consisting of proteases, lipases, cutinases, amylases, carbohydrases, cellulases, pectinases, mannanases, arabinases, galactanases, xylanases and oxidases.

The pH of the liquid solution is in the range of 1 to 11, such as in the range 5.5 to 11, such as in the range of 7 to 9, in the range of 7 to 8 or in the range of 7 to 8.5.

The temperature of the liquid solution can be in the range of 5°C to 95°C, or in the range of 10°C to 80°C, in the range of 10°C to 70°C, in the range of 10°C to 60°C, in the range of 10°C to 50°C, in the range of 15°C to 40°C or in the range of 20°C to 30°C. In one embodiment the temperature of the liquid solution is 30°C.

In one embodiment, the item is rinsed after being contacted to the liquid solution. The item can be rinsed with water or with water comprising a conditioner.

The concentration of the DNase is typically in the range of 0.00004-100 ppm enzyme protein, such as in the range of 0.00008-100, in the range of 0.0001-100, in the range of 0.0002-100, in the range of 0.0004-100, in the range of 0.0008-100, in the range of 0.001-100 ppm enzyme protein, 0.01-100 ppm enzyme protein, preferably 0.05-50 ppm enzyme protein, more preferably 0.1-50 ppm enzyme protein, more preferably 0.1-30 ppm enzyme protein, more preferably 0.5-20 ppm enzyme protein, and most preferably 0.5-10 ppm enzyme protein.

The DNase of the present invention may be added to a detergent composition in an amount corresponding to at least 0.002 mg of DNase protein, such as at least 0.004 mg of DNase protein, at least 0.006 mg of DNase protein, at least 0.008 mg of DNase protein, at least 0.01 mg of DNase protein, at least 0.1 mg of protein, preferably at least 1 mg of protein, more preferably at least 10 mg of protein, even more preferably at least 15 mg of protein, most preferably at least 20 mg of protein, and even most preferably at least 25 mg of protein. Thus, the detergent composition may comprise at least 0.00008% DNase protein, preferably at least 0.002%, 0.003%, 0.004%, 0.005%, 0.006%, 0.008%, 0.01%, 0.02%, 0.03%, 0.05%, 0.1%, 0.2%, 0.3%, 0.4%, 0.6%, 0.7%, 0.8%, 0.9% or 1.0% of DNase protein.

The polypeptide having DNase activity can be of animal, vegetable, microbial origin. In one embodiment the polypeptide is of human or bovine origin. In one embodiment the polypeptide is obtained from a plant such as mung bean. In one embodiment the polypeptide is of bacterial or fungal origin.

A polypeptide of fungal origin may be selected from the group consisting of:

a. a polypeptide having at least 60% sequence identity to the mature polypeptide of SEQ ID NO: 2, a polypeptide having at least 60% sequence identity to the mature polypeptide of SEQ ID NO: 3 or a polypeptide having at least 60% sequence identity to the mature polypeptide of SEQ ID NO: 5 or a polypeptide having at least 60% sequence identity to the mature polypeptide of SEQ ID NO: 8;

b. a polypeptide encoded by a polynucleotide that hybridizes under low stringency conditions with

   i. the mature polypeptide coding sequence of SEQ ID NO: 1 or the mature polypeptide coding sequence of SEQ ID NO: 4

   ii. the cDNA sequence thereof, or

   iii. the full-length complement of (i) or (ii);

c. a polypeptide encoded by a polynucleotide having at least 60% sequence identity to the mature polypeptide coding sequence of SEQ ID NO: 1 or the cDNA sequence thereof or a polypeptide encoded by a polynucleotide having at least 60% sequence identity to the mature polypeptide coding sequence of SEQ ID NO: 4 or the cDNA sequence thereof;
d. a variant of the mature polypeptide of SEQ ID NO: 2 comprising a substitution, deletion, and/or insertion at one or more positions, a variant of the mature polypeptide of SEQ ID NO: 3 comprising a substitution, deletion, and/or insertion at one or more positions or a variant of the mature polypeptide of SEQ ID NO: 5 comprising a substitution, deletion, and/or insertion at one or more positions or a variant of the mature polypeptide of SEQ ID NO: 8 comprising a substitution, deletion, and/or insertion at one or more positions; and
e. a fragment of the polypeptide of (a), (b), (c), or (d) that has DNase activity.

European patent application number 14164424.5 discloses in examples 1 to 3 how the polypeptide of SEQ ID NO: 2, SEQ ID NO: 3 and SEQ ID NO: 8 are produced. European patent application number 14164429.4 discloses in examples 1 to 2 how the polypeptide of SEQ ID NO: 5 is produced.

A polypeptide of bacterial origin may be selected from the group consisting of:

a. a polypeptide having at least 60% sequence identity to the mature polypeptide of SEQ ID NO: 6 or a polypeptide having at least 60% sequence identity to the mature polypeptide of SEQ ID NO: 7;
b. a variant of the mature polypeptide of SEQ ID NO: 6 comprising a substitution, deletion, and/or insertion at one or more positions or a variant of the mature polypeptide of SEQ ID NO: 7 comprising a substitution, deletion, and/or insertion at one or more positions; and
c. a fragment of the polypeptide of (a) or (b) that has DNase activity;

The polypeptide can have at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the mature polypeptide of SEQ ID NO: 2, to the mature polypeptide of SEQ ID NO: 3, or to the mature polypeptide of SEQ ID NO: 5, or to the mature polypeptide of SEQ ID NO: 6 or to the mature polypeptide of SEQ ID NO: 7.

International patent application published under number WO2011098579 discloses in example 3 how to clone and express the polypeptide of SEQ ID NO: 6.

The polypeptide can comprise or consist of SEQ ID NO: 2 or the mature polypeptide of SEQ ID NO: 2, the polypeptide comprises or consists of SEQ ID NO: 3 or the mature polypeptide of SEQ ID NO: 3, the polypeptide comprises or consists of SEQ ID NO: 5 or the mature polypeptide of SEQ ID NO: 5, the polypeptide comprises or consists of SEQ ID NO: 6 or the mature polypeptide of SEQ ID NO: 6, the polypeptide comprises or consists of SEQ ID NO: 7 or the mature polypeptide of SEQ ID NO: 7 or the polypeptide comprises or consists of SEQ ID NO: 8 or the mature polypeptide of SEQ ID NO: 8..

The mature polypeptide can comprise amino acids 1 to 206 of SEQ ID NO: 2, amino acids 1 to 206 of SEQ ID NO: 3, amino acids 1 to 188 of SEQ ID NO: 5, amino acids 1 to 110 of SEQ ID NO: 6, amino acids 1 to 109 of SEQ ID NO: 7 or amino acids 1 to 206 of SEQ ID NO: 8.

The polypeptide can be a variant of the mature polypeptide of SEQ ID NO: 2, SEQ ID NO: 3, SEQ ID NO: 5, SEQ ID NO: 6, SEQ ID NO: 7 or SEQ ID NO: 8, wherein the variant comprises a substitution, deletion, and/or insertion at one or more positions or a variant of the mature polypeptide of SEQ ID NO: 2, SEQ ID NO: 3, SEQ ID NO: 5, SEQ ID NO: 6, SEQ ID NO: 7 or SEQ ID NO: 8 which comprises a substitution, deletion, and/or insertion at one or more positions.

The polypeptide can be a fragment of of SEQ ID NO: 2, SEQ ID NO: 3, SEQ ID NO: 5, SEQ ID NO: 6, SEQ ID NO: 7 or SEQ ID NO: 8, wherein the fragment has DNase activity.

The polypeptide having DNase activity can be obtained from *Aspergillus,* for example from *Aspergillus oryzae.*

The polypeptides may have a sequence identity to the mature polypeptide of SEQ ID NO: 2 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have DNase activity. In one aspect, the polypeptides differ by up to 10 amino acids, e.g., 1, 2, 3, 4, 5, 6, 7, 8, 9, or 10, from the mature polypeptide of SEQ ID NO: 2.

The polypeptides may have a sequence identity to the mature polypeptide of SEQ ID NO: 3 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have DNase activity. In one aspect, the polypeptides differ by up to 10 amino acids, e.g., 1, 2, 3, 4, 5, 6, 7, 8, 9, or 10, from the mature polypeptide of SEQ ID NO: 3.

The polypeptides may have a sequence identity to the mature polypeptide of SEQ ID NO: 8 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have DNase activity. In one

aspect, the polypeptides differ by up to 10 amino acids, e.g., 1, 2, 3, 4, 5, 6, 7, 8, 9, or 10, from the mature polypeptide of SEQ ID NO: 8.

A polypeptide for use in the present invention preferably comprises or consists of the amino acid sequence of SEQ ID NO: 2 or an allelic variant thereof; or is a fragment thereof having DNase activity. In another aspect, the polypeptide comprises or consists of the mature polypeptide of SEQ ID NO: 2. In another aspect, the polypeptide comprises or consists of amino acids 1 to 206 of SEQ ID NO: 2. The polypeptide may have been isolated.

A polypeptide for use in the present invention preferably comprises or consists of the amino acid sequence of SEQ ID NO: 3 or an allelic variant thereof; or is a fragment thereof having DNase activity. In another aspect, the polypeptide comprises or consists of the mature polypeptide of SEQ ID NO: 3. In another aspect, the polypeptide comprises or consists of amino acids 1 to 206 of SEQ ID NO: 3. The polypeptide may have been isolated.

A polypeptide for use in the present invention preferably comprises or consists of the amino acid sequence of SEQ ID NO: 8 or an allelic variant thereof; or is a fragment thereof having DNase activity. In another aspect, the polypeptide comprises or consists of the mature polypeptide of SEQ ID NO: 8. In another aspect, the polypeptide comprises or consists of amino acids 1 to 206 of SEQ ID NO: 8. The polypeptide may have been isolated.

An isolated polypeptide having DNase activity may be encoded by a polynucleotide that hybridizes under low stringency conditions with (i) the mature polypeptide coding sequence of SEQ ID NO: 1, (ii) the cDNA sequence thereof, or (iii) the full-length complement of (i) or (ii) (Sambrook et al., 1989, Molecular Cloning, A Laboratory Manual, 2d edition, Cold Spring Harbor, New York). In an embodiment, the polypeptide has been isolated.

A polypeptide having DNase activity may be encoded by a polynucleotide having a sequence identity to the mature polypeptide coding sequence of SEQ ID NO: 1 or the cDNA sequence thereof of at least 60%, *e.g.,* at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

The polypeptide may be a variant of the mature polypeptide of SEQ ID NO: 2 comprising a substitution, deletion, and/or insertion at one or more (e.g., several) positions. In an embodiment, the number of amino acid substitutions, deletions and/or insertions introduced into the mature polypeptide of SEQ ID NO: 2 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

The polypeptide may be a variant of the mature polypeptide of SEQ ID NO: 3 comprising a substitution, deletion, and/or insertion at one or more (*e.g.*, several) positions. In an embodiment, the number of amino acid substitutions, deletions and/or insertions introduced into the mature polypeptide of SEQ ID NO: 3 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

The polypeptide having DNase activity can also be obtained from from *Trichoderma,* for example from *Trichoderma harzianum.* In an embodiment, the polypeptides may have a sequence identity to the mature polypeptide of SEQ ID NO: 5 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have DNase activity. In one aspect, the polypeptides differ by up to 10 amino acids, e.g., 1, 2, 3, 4, 5, 6, 7, 8, 9, or 10, from the mature polypeptide of SEQ ID NO: 5.

A polypeptide for use in the present invention may comprise or consist of the amino acid sequence of SEQ ID NO: 5 or an allelic variant thereof; or be a fragment thereof having DNase activity. The polypeptide may comprise or consist of the mature polypeptide of SEQ ID NO: 5, or may comprise or consist of amino acids 1 to 188 of SEQ ID NO: 5. The polypeptide may have been isolated.

An isolated polypeptide having DNase activity may be encoded by a polynucleotide that hybridizes under low stringency conditions with (i) the mature polypeptide coding sequence of SEQ ID NO: 4, (ii) the cDNA sequence thereof, or (iii) the full-length complement of (i) or (ii) (Sambrook et al., 1989, Molecular Cloning, A Laboratory Manual, 2d edition, Cold Spring Harbor, New York). In an embodiment, the polypeptide has been isolated.

Hybridization indicates that the polynucleotide hybridizes to a labeled nucleic acid probe corresponding to (i) SEQ ID NO: 1 or SEQ ID NO: 4; (ii) the mature polypeptide coding sequence of SEQ ID NO: 1 or SEQ ID NO: 4; (iii) the cDNA sequence thereof; (iv) the full-length complement thereof; or (v) a subsequence thereof; under very low to very high stringency conditions. Molecules to which the nucleic acid probe hybridizes under these conditions can be detected using, for example, X-ray film or any other detection means known in the art.

A polypeptide having DNase activity may be encoded by a polynucleotide having a sequence identity to the mature polypeptide coding sequence of SEQ ID NO: 1 or SEQ ID NO: 4 or the cDNA sequence thereof of at least 60%, *e.g.,* at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

The polypeptide may be a variant of the mature polypeptide of SEQ ID NO: 2, SEQ ID NO: 3 or SEQ ID NO: 5 comprising a substitution, deletion, and/or insertion at one or more (*e.g.*, several) positions. In an embodiment, the number of amino acid substitutions, deletions and/or insertions introduced into the mature polypeptide of SEQ ID NO: 2, SEQ ID NO: 3 or SEQ ID NO: 5 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

The polypeptide having DNase activity can also be obtained from from *Bacillus,* for example from *Bacillus subtilis or Bacillus licheniformis.*

The polypeptide may have a sequence identity to the mature polypeptide of SEQ ID NO: 6 or SEQ ID NO: 7 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have DNase activity. In one aspect, the polypeptides differ by up to 10 amino acids, e.g., 1, 2, 3, 4, 5, 6, 7, 8, 9, or 10, from the mature polypeptide of SEQ ID NO: 6 or SEQ ID NO: 7.

A polypeptide for use in the present invention may comprises or consists of the amino acid sequence of SEQ ID NO: 6, SEQ ID NO: 7 or an allelic variant thereof; or is a fragment thereof having DNase activity. The polypeptide may comprise or consist of the mature polypeptide of SEQ ID NO: 6 or SEQ ID NO: 7. The polypeptide may comprise or consist of amino acids 1 to 110 of SEQ ID NO: 6 or amino acids 1 to 109 of SEQ ID NO: 7. The polypeptide may have been isolated.

The polypeptide having DNase activity may be encoded by a polynucleotide having a sequence identity to the mature polypeptide coding sequence of SEQ ID NO: 6, SEQ ID NO: 7 or the cDNA sequence thereof of at least 60%, *e.g.,* at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%. The polypeptide may have been isolated.

The polypeptide having DNase activity may be encoded by a polynucleotide having a sequence identity to the mature polypeptide coding sequence of SEQ ID NO: 6, SEQ ID NO: 7 or the cDNA sequence thereof of at least 60%, *e.g.,* at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% and wherein the polypeptide is used for preventing or reducing re-deposition of soil on an item during a subsequent cleaning or laundering process

The polypeptide may be a variant of the mature polypeptide of SEQ ID NO: 6 or SEQ ID NO: 7 comprising a substitution, deletion, and/or insertion at one or more (*e.g.*, several) positions. The number of amino acid substitutions, deletions and/or insertions introduced into the mature polypeptide of SEQ ID NO: 6 or SEQ ID NO: 7 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for DNase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance,

crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.*, Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

The polypeptide may be a hybrid polypeptide in which a region of one polypeptide is fused at the N-terminus or the C-terminus of a region of another polypeptide.

The polypeptide may be a fusion polypeptide or cleavable fusion polypeptide in which another polypeptide is fused at the N-terminus or the C-terminus of the polypeptide of the present invention. A fusion polypeptide is produced by fusing a polynucleotide encoding another polypeptide to a polynucleotide encoding a DNase. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

**Deoxyribonuclease (DNase)**

A polypeptide having DNase activity or a deoxyribonuclease (DNase) is any enzyme that catalyzes the hydrolytic cleavage of phosphodiester linkages in the DNA backbone, thus degrading DNA. The two terms polypeptide having DNase activity and DNase are used interchangeably.

According to the present invention, a DNase which is obtainable from a fungus is preferred; in particular a DNase which is obtainable from *Aspergillus* is preferred; in particular a DNase which is obtainable from *Aspergillus oryzae* is preferred. In one embodiment of the present invention, the polypeptide having deoxyribonuclease activity is not the S1 nuclease from *Aspergillus oryzae*.

The DNase used in the present invention preferably includes the mature polypeptide of SEQ ID NO: 2, shown as amino acids 1 to 206 of SEQ ID NO: 2, which is obtained from *Aspergillus oryzae.* The polypeptide having DNase activity can be obtained from *Aspergillus,* for example from *Aspergillus oryzae.*

Isolated polypeptides may have a sequence identity to the mature polypeptide of SEQ ID NO: 2 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have DNase activityThe polypeptides may differ by up to 10 amino acids, e.g., 1, 2, 3, 4, 5, 6, 7, 8, 9, or 10, from the mature polypeptide of SEQ ID NO: 2.

Isolated polypeptides may have a sequence identity to the mature polypeptide of SEQ ID NO: 3 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have DNase activity. The polypeptides may differ by up to 10 amino acids, e.g., 1, 2, 3, 4, 5, 6, 7, 8, 9, or 10, from the mature polypeptide of SEQ ID NO: 3.

Isolated polypeptides may have a sequence identity to SEQ ID NO: 8 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have DNase activity. The polypeptides may differ by up to 10 amino acids, e.g., 1, 2, 3, 4, 5, 6, 7, 8, 9, or 10, from the mature polypeptide of SEQ ID NO: 8.

A polypeptide for use in the present invention preferably comprises or consists of the amino acid sequence of SEQ ID NO: 2 or an allelic variant thereof; or is a fragment thereof having DNase activity. In another aspect, the polypeptide comprises or consists of the mature polypeptide of SEQ ID NO: 2. In another aspect, the polypeptide comprises or consists of amino acids 1 to 206 of SEQ ID NO: 2.

In an embodiment, the polypeptide has been isolated. A polypeptide for use in the present invention preferably comprises or consists of the amino acid sequence of SEQ ID NO: 3 or an allelic variant thereof; or is a fragment thereof having DNase activity. In another aspect, the polypeptide comprises or consists of the mature polypeptide of SEQ ID NO: 3. In another aspect, the polypeptide comprises or consists of amino acids 1 to 204 of SEQ ID NO: 3. A composition may comprise or consist of a polypeptide consisting of the amino acid sequence of SEQ ID NO: 8 and a polypeptide consisting of the amino acid sequence of SEQ ID NO: 3.

An isolated polypeptide having DNase activity may be encoded by a polynucleotide that hybridizes under low stringency conditions with (i) the mature polypeptide coding sequence of SEQ ID NO: 1, (ii) the cDNA sequence thereof, or (iii) the full-length complement of (i) or (ii) (Sambrook et al., 1989, Molecular Cloning, A Laboratory Manual, 2d edition, Cold Spring Harbor, New York). In an embodiment, the polypeptide has been isolated.

An isolated polypeptide having DNase activity may be encoded by a polynucleotide that hybridizes under low-medium stringency conditions with (i) the mature polypeptide coding sequence of SEQ ID NO: 1, (ii) the cDNA sequence thereof, or (iii) the full-length complement of (i) or (ii). In an embodiment, the polypeptide has been isolated.

An isolated polypeptide having DNase activity may be encoded by a polynucleotide that hybridizes under medium stringency conditions with (i) the mature polypeptide coding sequence of SEQ ID NO: 1, (ii) the cDNA sequence thereof, or (iii) the full-length complement of (i) or (ii). In an embodiment, the polypeptide has been isolated.

An isolated polypeptide having DNase activity may be encoded by a polynucleotide that hybridizes under medium-high stringency conditions with (i) the mature polypeptide coding sequence of SEQ ID NO: 1, (ii) the cDNA sequence thereof, or (iii) the full-length complement of (i) or (ii). In an embodiment, the polypeptide has been isolated.

An isolated polypeptide having DNase activity may be encoded by a polynucleotide that hybridizes under high stringency conditions with (i) the mature polypeptide coding sequence of SEQ ID NO: 1, (ii) the cDNA sequence thereof, or (iii) the full-length complement of (i) or (ii). In an embodiment, the polypeptide has been isolated.

An isolated polypeptide having DNase activity may be encoded by a polynucleotide that hybridizes under very high stringency conditions with (i) the mature polypeptide coding sequence of SEQ ID NO: 1, (ii) the cDNA sequence thereof, or (iii) the full-length complement of (i) or (ii). In an embodiment, the polypeptide has been isolated.

A polypeptide having DNase activity may be encoded by a polynucleotide having a sequence identity to the mature polypeptide coding sequence of SEQ ID NO: 1 or the cDNA sequence thereof of at least 60%, *e.g.,* at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%. The polypeptide may have been isolated.

Variants of the mature polypeptide of SEQ ID NO: 2 may comprise a substitution, deletion, and/or insertion at one or more (e.g., several) positions. In an embodiment, the number of amino acid substitutions, deletions and/or insertions introduced into the mature polypeptide of SEQ ID NO: 2 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Variants of the mature polypeptide of SEQ ID NO: 3 may comprise a substitution, deletion, and/or insertion at one or more (*e.g.*, several) positions. In an embodiment, the number of amino acid substitutions, deletions and/or insertions introduced into the mature polypeptide of SEQ ID NO: 3 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain

The DNase enzyme may comprise or consist of the amino acid sequence shown as amino acids -37 to 206 of SEQ ID NO: 2 or a fragment thereof that has DNase activity, such as the mature polypeptide. Or the DNase enzyme may comprise or consist of a fragment of amino acids -37 to 206 of SEQ ID NO: 2 or amino acids 1 to 206 of SEQ ID NO: 2 for which fragment one or more amino acids is deleted from the amino and/or carboxyl terminus of SEQ ID NO: 2.

The DNase enzyme may comprise or consist of the amino acid sequence shown as amino acids 1 to 206 of SEQ ID NO: 3 or a fragment thereof that has DNase activity, such as the mature polypeptide. Or the DNase enzyme may comprise or consist of a fragment of amino acids 1 to 206 of SEQ ID NO: 3 or amino acids 1 to 206 of SEQ ID NO: 3 for which fragment one or more amino acids is deleted from the amino and/or carboxyl terminus of SEQ ID NO: 3.

The DNase enzyme may comprise or consist of the amino acid sequence shown as amino acids 1 to 206 of SEQ

ID NO: 8 or a fragment thereof that has DNase activity, such as the mature polypeptide. Or the DNase enzyme may comprise or consist of a fragment of amino acids 1 to 206 of SEQ ID NO: 8 or amino acids 1 to 206 of SEQ ID NO: 8 for which fragment one or more amino acids is deleted from the amino and/or carboxyl terminus of SEQ ID NO: 8.

DNase polypeptides may also be substantially homologous to the polypeptides above, and species homologs (paralogs or orthologs) thereof. The term "substantially homologous" is used herein to denote polypeptides being at least 80%, preferably at least 85%, more preferably at least 90%, more preferably at least 95%, even more preferably at least 97% identical, and most preferably at least 99% or more identical to the amino acid sequence of SEQ ID NO: 2 or to the amino acid sequence of SEQ ID NO: 3, or a fragment thereof that has DNase activity, or its orthologs or paralogs.

The DNase of SEQ ID NO: 2 may comprise a substitution, deletion, and/or insertion at one or more (*e.g.*, several) positions. The DNase of SEQ ID NO: 3 may comprise a substitution, deletion, and/or insertion at one or more (*e.g.*, several) positions. The number of amino acid substitutions, deletions and/or insertions introduced into the mature polypeptide of SEQ ID NO: 2 or into the mature polypeptide of SEQ ID NO: 3 may be not more than 10, *e.g.,* 1, 2, 3, 4, 5, 6, 7, 8 or 9. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

According to the present invention, a DNase which is obtainable from a fungus is preferred; in particular a DNase which is obtainable from *Trichoderma* is preferred; in particular a DNase which is obtainable from *Trichoderma harzianum* is preferred.

The DNase used in the present invention includes the mature polypeptide of SEQ ID NO: 5, shown as amino acids 1 to 188 of SEQ ID NO: 5, which is obtained from *Trichoderma harzianum.*

The DNase enzyme may comprise or consist of the amino acid sequence shown as amino acids -17 to 188 of SEQ ID NO: 5 or a fragment thereof that has DNase activity, such as the mature polypeptide. Or the DNase enzyme may comprise or consist of a fragment of amino acids -17 to 188 of SEQ ID NO: 5 or amino acids 1 to 188 of SEQ ID NO: 5 for which fragment one or more amino acids is deleted from the amino and/or carboxyl terminus of SEQ ID NO: 5.

DNase polypeptides may be substantially homologous to the polypeptides above, and species homologs (paralogs or orthologs) thereof. The term "substantially homologous" is used herein to denote polypeptides being at least 80%, preferably at least 85%, more preferably at least 90%, more preferably at least 95%, even more preferably at least 97% identical, and most preferably at least 99% or more identical to the amino acid sequence of SEQ ID NO: 5, or a fragment thereof that has DNase activity, or its orthologs or paralogs.

According to the present invention, a DNase which is obtainable from a bacterium is preferred; in particular a DNase which is obtainable from a *Bacillus* is preferred; in particular a DNase which is obtainable from *Bacillus subtilis* or *Bacillus licheniformis* is preferred.

The DNase used in the present invention includes the mature polypeptide of SEQ ID NO: 6, shown as amino acids 1 to 110 of SEQ ID NO: 6, which is derived from *Bacillus subtilis;* or the mature polypeptide of SEQ ID NO: 7, shown as amino acids 1 to 109 of SEQ ID NO: 7, which is derived from *Bacillus licheniformis.*

The DNase enzyme may comprise or consist of the amino acid sequence shown as amino acids -26 to 110 of SEQ ID NO: 6 or amino acids -33 to 109 of SEQ ID NO: 7, or a fragment thereof that has DNase activity, such as the mature polypeptide. A fragment of amino acids -26 to 110 of SEQ ID NO: 6, or amino acids 1 to 110 of SEQ ID NO: 6 is a polypeptide, which has one or more amino acids deleted from the amino and/or carboxyl terminus of SEQ ID NO: 6. A fragment of or amino acids -33 to 109 of SEQ ID NO: 7, or 1 to 109 of SEQ ID NO: 7 is a polypeptide, which has one or more amino acids deleted from the amino and/or carboxyl terminus of SEQ ID NO: 7.

DNase polypeptides may be substantially homologous to the polypeptides above, and species homologs (paralogs or orthologs) thereof. The term "substantially homologous" is used herein to denote polypeptides being at least 80%, preferably at least 85%, more preferably at least 90%, more preferably at least 95%, even more preferably at least 97% identical, and most preferably at least 99% or more identical to the amino acid sequence of SEQ ID NO: 6 or SEQ ID NO: 7, or a fragment thereof that has DNase activity, or its orthologs or paralogs.

The DNase in a detergent composition may be stabilized using conventional stabilizing agents, *e.g.* a polyol such as propylene glycol or glycerol, a sugar or sugar alcohol, lactic acid, boric acid, or a boric acid derivative, *e.g.* an aromatic borate ester, or a phenyl boronic acid derivative such as 4-formylphenyl boronic acid, and the composition may be formulated as described in, for example, WO92/19709 and WO92/19708.

**Detergent compositions**

Detergent compositions comprising an enzyme of the present invention comprise one or more additional cleaning composition components. The choice of additional components is within the skill of the artisan and includes conventional ingredients, including the exemplary non-limiting components set forth below.

## Odor control agents

Odor control agents are agents that reduces the malodour from an item, Odor control agents are agents that reduce, neutralize or remove malodor from an item when the odor control agent is used during washing or laundering of the item. The odor control agent is different from DNase.

Odor control agents may include, but are not limited to HMP, volatile aldehydes or cyclodextrins or mixtures thereof.

In general, the present malodor-controlling compositions will comprise one or more odor control agent (s) at a level of from about 0.001% to about 99. 99%, preferably from about 0.002% to about 99.9%, and more preferably from about 0.005% to about 99%, by weight of the malodor- controlling composition. When the compositions are aqueous liquid compositions (especially non- aerosol compositions) to be sprayed onto surfaces, such as fabrics, the compositions will preferably comprise less than about 20%, preferably less than about 10%, more preferably less than about 5%, by weight of the composition, of odor control agent. The odor control agent serves to reduce or remove malodor from the surfaces or objects being treated with the present compositions. The odor control agent is preferably selected from the group consisting of : uncomplexed cyclodextrin; odor blockers; reactive aldehydes; flavanoids; zeolites; activated carbon; and mixtures thereof.

## Uncomplexed Cyclodextrin

As used herein, the term"uncomplexed cyclodextrin"includes any of the known cyclodextrins in uncomplexed form such as unsubstituted cyclodextrins containing from six to twelve glucose units, especially, alpha-cyclodextrin, beta-cyclodextrin, gamma-cyclodextrin and/or their derivatives and/or mixtures thereof. The alpha-cyclodextrin consists of six glucose units, the beta-cyclodextrin consists of seven glucose units, and the gamma-cyclodextrin consists of eight glucose units arranged in donut-shaped rings. The specific coupling and conformation of the glucose units give the cyclodextrins a rigid, conical molecular structure with hollow interiors of specific volumes. The"lining"of each internal cavity is formed by hydrogen atoms and glycosidic bridging oxygen atoms; therefore, this surface is fairly hydrophobic. The unique shape and physical-chemical properties of the cavity enable the cyclodextrin molecules to absorb (form inclusion complexes with) organic molecules or parts of organic molecules which can fit into the cavity. Many odorous molecules can fit into the cavity including many malodorous molecules and perfume molecules. Therefore, cyclodextrins, and especially mixtures of cyclodextrins with different size cavities, can be used to control odors caused by a broad spectrum of organic odoriferous materials, which may, or may not, contain reactive functional groups. The complexation between cyclodextrin and odorous molecules occurs rapidly in the presence of water. However, the extent of the complex formation also depends on the polarity of the absorbed molecules. In an aqueous solution, strongly hydrophilic molecules (those which are highly water-soluble) are only partially absorbed, if at all. Therefore, cyclodextrin does not complex effectively with some very low molecular weight organic amines and acids when they are present at low levels on surfaces. The cavities within the cyclodextrin in the deodorizing composition should remain essentially unfilled (the cyclodextrin remains uncomplexed) while in solution, in order to allow the cyclodextrin to absorb various odor molecules when the solution is applied to a surface. Non-derivatised (normal) beta-cyclodextrin can be present at a level up to its solubility limit of about 1.85% (about 1.85g in 100 grams of water) under the conditions of use at room temperature.

Preferably, the cyclodextrin used in the present invention is highly water-soluble such as, alpha-cyclodextrin and/or derivatives thereof, gamma-cyclodextrin and/or derivatives thereof, derivatised beta-cyclodextrins, and/or mixtures thereof. The derivatives of cyclodextrin consist mainly of molecules wherein some of the OH groups are converted to OR groups. Cyclodextrin derivatives include, e. g. , those with short chain alkyl groups such as methylated cyclodextrins, and ethylated cyclodextrins, wherein R is a methyl or an ethyl group; those with hydroxyalkyl substituted groups, such as hydroxypropyl cyclodextrins and/or hydroxyethyl cyclodextrins, wherein R is a -CH2-CH-(OH)-CH3 or a -CH2CH2-OH group; branched cyclodextrins such as maltose-bonded cyclodextrins; cationic cyclodextrins such as those containing 2-hydroxy-3- (dimethylamino) propyl ether, wherein R is CH2-CH (OH)-CH2-N-(CH3)2 which is cationic at low pH; quaternary ammonium, e. g., 2-hydroxy-3-(trimethylammonio) propyl ether chloride groups, wherein R is CH2-CH-(OH)-CH2-$N^+$(CH3) 3Cl-; anionic cyclodextrins such as carboxymethyl cyclodextrins, cyclodextrin sulfates, and cyclodextrin succinylates; amphoteric cyclodextrins such as carboxymethyl/quatemary ammonium cyclodextrins; cyclodextrins wherein at least one glucopyranose unit has a 3-6-anhydro-cyclomalto structure, e. g. , the mono-3-6-anhydrocyclodextrins, as disclosed in "Optimal Performances with Minimal Chemical Modification of Cyclodextrins", F. Diedaini-Pilard and B. Perly, The 7th International Cyclodextrin Symposium Abstracts, April 1994, p. 49; and mixtures thereof. Other cyclodextrin derivatives are disclosed in U. S. Pat. Nos: 3,426, 011, Parmerter et al. , issued Feb. 4,1969 ; 3,453, 257; 3,453, 258; 3,453, 259; and 3,453, 260, all in the names of Parmerter et al. , and all issued July 1,1969 ; 3,459, 731, Gramera et al. , issued Aug. 5,1969 ; 3,553, 191, Parmerter et al., issued Jan. 5, 1971 ; 3,565, 887, Parmerter et al. , issued Feb. 23,1971 ; 4,535, 152, Szejtli et al., issued Aug. 13, 1985; 4,616, 008, Hirai et al. , issued Oct. 7,1986 ; 4,678, 598, Ogino et al. , issued Jul. 7,1987 ; 4,638, 058, Brandt et al. , issued Jan. 20,1987 ; and 4,746, 734, Tsuchiyama et al.,

issued May 24,1988. Further cyclodextrin derivatives suitable herein include those disclosed in V. T. D'Souza and K. B. Lipkowitz, CHEMICAL REVIEWS: CYCLODEXTRINS, Vol. 98, No. 5 (American Chemical Society, July/August 1998).

Highly water-soluble cyclodextrins are those having water solubility of at least about 10 g in 100 ml of water at room temperature, preferably at least about 20 g in 100 ml of water, more preferably at least about 25 g in 100 ml of water at room temperature. Solubilized, water-soluble cyclodextrin can exhibit more efficient odor control performance than non-water-soluble cyclodextrin when deposited onto surfaces, especially carpeted surfaces.

Examples of preferred water-soluble cyclodextrin derivatives suitable for use herein are hydroxypropyl alpha-cyclodextrin, methylated alpha-cyclodextrin, methylated beta-cyclodextrin, hydroxyethyl beta-cyclodextrin, and hydroxypropyl beta-cyclodextrin. Hydroxyalkyl cyclodextrin derivatives preferably have a degree of substitution of from about 1 to about 14, more preferably from about 1.5 to about 7, wherein the total number of OR groups per cyclodextrin is defined as the degree of substitution. Methylated cyclodextrin derivatives typically have a degree of substitution of from about 1 to about 18, preferably from about 3 to about 16. A known methylated beta-cyclodextrin is heptakis-2, 6-di-O-methyl--cyclodexhin, commonly known as DIMEB, in which each glucose unit has about 2 methyl groups with a degree of substitution of about 14. A preferred, more commercially available, methylated beta- cyclodextrin is a randomly methylated beta-cyclodextrin, commonly known as RAMEB, having different degrees of substitution, normally of about 12.6. RAMEB is more preferred than DIMEB, since DIMEB affects the surface activity of the preferred surfactants more than RAMEB. The preferred cyclodextrins are available, e.g. from Cerestar USA, Inc. and Wacker Chemicals (USA), Inc.

It is also preferable to use a mixture of cyclodextrins. Such mixtures absorb odors more broadly by complexing with a wider range of odoriferous molecules having a wider range of molecular sizes. Preferably at least a portion of the cyclodextrin is alpha-cyclodextrin and its derivatives thereof, gamma-cyclodextrin and its derivatives thereof, and/or derivatised beta- cyclodextrin, more preferably a mixture of alpha-cyclodextrin, or an alpha-cyclodextrin derivative, and derivatised beta-cyclodextrin, even more preferably a mixture of derivatised alpha- cyclodextrin and derivatised beta-cyclodextrin, most preferably a mixture of hydroxypropyl alpha-cyclodextrin and hydroxypropyl beta-cyclodextrin, and/or a mixture of methylated alpha- cyclodextrin and methylated beta-cyclodextrin. Since cyclodextrin can be a prime breeding ground for certain microorganisms, especially when in aqueous compositions, it is preferable to include a water-soluble preservative, as described infra, which is effective for inhibiting and/or regulating microbial growth, to increase storage stability of aqueous odor-absorbing solutions containing water-soluble cyclodextrin.

Odor Blockers "Odor blockers" can be used as an odor control agent to mitigate the effects of malodors.

In order to be effective, the odor blockers normally have to be present at all times. If the odor blocker evaporates before the source of the odor is gone, it is less likely to control the odor. Also, the odor blockers can tend to adversely affect aesthetics by blocking desirable odors like perfumes. Non-limiting examples of odor blockers suitable as odor control agents in the present compositions include 4-cyclohexyl-4-methyl-2-pentanone, 4-ethylcyclohexyl methyl ketone, 4-isopropylcyclohexyl methyl ketone, cyclohexyl methyl ketone, 3-methylcyclohexyl methyl ketone, 4-tert.-butylcyclohexyl methyl ketone, 2-methyl-4-tert. butylcyclohexyl methyl ketone, 2- methyl-5-isopropylcyclohexyl methyl ketone, 4-methylcyclohexyl isopropyl ketone, 4- methylcyclohexyl sec. butyl ketone, 4-methylcyclohexyl isobutyl ketone, 2,4-dimethylcyclohexyl methyl ketone, 2,3-dimethylcyclohexyl methyl ketone, 2,2-dimethylcyclohexyl methyl ketone, 3, 3-dimethylcyclohexyl methyl ketone, 4,4-dimethylcyclohexyl methyl ketone, 3,3, 5- trimethylcyclohexyl methyl ketone, 2,2, 6-trimethylcyclohexyl methyl ketone, 1-cyclohexyl-l- ethyl formate, 1-cyclohexyl-l-ethyl acetate, 1-cyclohexyl-l-ethyl propionate, 1-cyclohexyl-1-ethyl isobutyrate, 1-cyclohexyl-l-ethyl n-butyrate, 1-cyclohexyl-l-propyl acetate, 1-cyclohexyl-l- propyl n-butyrate, l-cyclohexyl-2-methyl-1-propyl acetate, 2-cyclohexyl-2-propyl acetate, 2- cyclohexyl-2-propyl propionate, 2-cyclohexyl-2-propyl isobutyrate, 2-cyclohexyl-2-propyl n- butyrate, 5, 5-dimethyl-1, 3-cyclohexanedione (dimedone), 2, 2-dimethyl-1, 3-dioxane-4,6-dione (Meldrum's acid), spiro- [4. 5]-6,10-dioxa-7, 9-dioxodecane, spiro- [5. 5]-1, 5-dioxa-2,4-dioxoundecane, 2, 2-hydroxymethyl-1, 3-dioxane-4,6-dione and 1,3-cyclohexadione. Odor blockers are disclosed in more detail in U. S. Patent Nos. 4,009, 253; 4,187, 251; 4,719, 105; 5,441, 727; and 5,861, 371.

**Reactive Aldehydes**

As an optional odor control agent, reactive aldehydes can be used as an odor control agent to mitigate the effects of malodors. Non-limiting examples of suitable reactive aldehydes include Class I aldehydes, Class II aldehydes, and mixtures thereof. Non-limiting examples of Class I aldehydes include anisic aldehyde, o-allyl-vanillin, benzaldehyde, cuminic aldehyde, ethyl- aubepin, ethyl-vanillin, heliotropin, tolyl aldehyde, and vanillin. Non-limiting examples of Class II aldehydes include 3- (4'-tert. butylphenyl) propanal, 2-methyl-3- (4'-tert. butylphenyl) propanal, 2- methyl-3- (4'-isopropylphenyl) propanal, 2, 2-dimethyl-3- (4-ethylphenyl) propanal, cinnamic aldehyde, a-amyl-cinnamic aldehyde, and a-hexyl-cinnamic aldehyde. These reactive aldehydes are described in more detail in U. S. Patent No. 5,676, 163.

Reactive aldehydes, when used, can include a combination of at least two aldehydes, with one aldehyde being selected from acyclic aliphatic aldehydes, non-terpenic aliphatic aldehydes, non-terpenic alicyclic aldehydes, terpenic aldehydes, aliphatic aldehydes substituted by an aromatic group and bifunctional aldehydes; and the second aldehyde being selected from aldehydes possessing an unsaturation alpha to the aldehyde function conjugated with an aromatic

ring, and aldehydes in which the aldehyde group is on an aromatic ring. This combination of at least two aldehydes is described in more detail in International Patent Application Pub. No. WO 00/49120.

As used herein, the term "reactive aldehydes" further encompasses deodorizing materials that are the reaction products of (i) an aldehyde with an alcohol, (ii) a ketone with an alcohol, or (iii) an aldehyde with the same or different aldehydes. Such deodorizing materials can be: (a) an acetal or hemiacetal produced by means of reacting an aldehyde with a carbinol; (b) a ketal or hemiketal produced by means of reacting a ketone with a carbinol; (c) a cyclic triacetal or a mixed cyclic triacetal of at least two aldehydes, or a mixture of any of these acetals, hemiacetals, ketals, hemiketals, or cyclic triacetals. These deodorizing perfume materials are described in more detail in International Patent Application Pub. No. WO 01/07095.

**Flavanoids**

Flavanoids can also be used as an odor control agent. Flavanoids are compounds based on t the Ca Q Q; ftavan skeleton. Flavanoids can be found in typical essential oils. Such oils include essential oil extracted by dry distillation from needle leaf trees and grasses such as cedar, Japanese cypress, eucalyptus, Japanese red pine, dandelion, low striped bamboo and cranesbill and can contain terpenic material such as alpha-pinene, beta-pinene, myrcene, phencone and camphene. Also included are extracts from tea leaf. Descriptions of such materials can be found in JP 02284997 and JP 04030855.

**Metallic Salts**

The odor control agent can include metallic salts for malodor control benefits. The metallic salts are selected from the group consisting of copper salts, zinc salts, and mixtures thereof.

The preferred zinc salts possess malodor control abilities. Zinc has been used most often for its ability to ameliorate malodor, e.g. in mouth wash products, as disclosed in U. S. Patent Nos. 4,325, 939 and 4,469, 674. Highly-ionized and soluble zinc salts such as zinc chloride, provide the best source of zinc ions. Preferred zinc salts are selected from the group consisting of zinc borate, zinc caprylate, zinc chloride, zinc ricinoleate, zinc sulfate heptahydrate, zinc undecylenate, and mixtures thereof.

Preferably the metallic salts are water-soluble zinc salts, copper salts or mixtures thereof, and more preferably zinc salts, especially ZnC12. These salts are preferably present as an odor control agent primarily to absorb amine and sulfur-containing compounds. Low molecular weight sulfur-containing materials, e.g. sulfide and mercaptans, are components of many types of malodors, e.g. food odors (garlic, onion), body/perspiration odor, breath odor, etc. Low molecular weight amines are also components of many malodors, e.g. food odors, body odors, urine, etc.

Zinc salts, when used, can be combined with an anionic surfactant having the formula R- (O-CH2-CH2)-X-O-CH2COO-, wherein R is a fatty alcohol substituent or an alkylaryl substituent and X is at least 2. Such anionic surfactants can act as a control release agent for the zinc salts to improve the malodor control properties of the composition. This combination of zinc salts and anionic surfactant is described in more detail in U. S. Patent No. 6,358, 469.

Zinc salts, when used, can also be combined with carbonate and/or bicarbonate to improve the malodor control properties of the composition. When zinc salts are combined with carbonate and/or bicarbonate, the composition preferably further comprises a stabilizing anion selected from phosphates having more than one- (P=O)- group and organic acids having more than one acid functionality. This combination of zinc salts, carbonate and/or bicarbonate, and stabilizing anions is described in more detail in U. S. Patent No. 6,015, 547.

Copper salts possess some malodor control abilities. See U. S. Patent No. 3,172, 817, Leupold, et al. , which discloses deodorizing compositions for treating disposable articles, comprising at least slightly water-soluble salts of acylacetone, including copper salts and zinc salts. When metallic salts are added to the composition as an odor control agent, they are typically present at a level of from about 0.001% to an effective amount to provide a saturated salt solution, preferably from about 0.002% to about 25%, more preferably from about 0. 003% to about 8%, still more preferably from about 0.1% to about 5% by weight of the composition.

**Zeolites**

The odor control agents herein can also be zeolites. A preferred class of zeolites is characterized as"intermediate"silicate/aluminate zeolites. The intermediate zeolites are characterized by SiOx/A10z molar ratios of less than about 10. Preferably the molar ratio of Si02 /A102 ranges from about 2 to about 10. The intermediate zeolites can have an advantage over the "high"zeolites. The intermediate zeolites have a higher affinity for amine-type odors, they are more weight efficient for odor absorption because they have a larger surface area, and they are more moisture tolerant and retain more of their odor absorbing capacity in water than the high zeolites. A wide variety of intermediate zeolites suitable for use herein are commercially available as Valfor® CP301-68, Valfor® 300-63, Valfor® CP300-35, and Valfor®CP300-56,

available from PQ Corporation, and the CBV100® series of zeolites from Conteka.

Zeolite materials marketed under the trade name Absents® and Smellrite®, available from The Union Carbide Corporation and UOP are also preferred. Such materials are preferred over the intermediate zeolites for control of sulfur-containing odors, e.g. thiols, mercaptans. When zeolites are used as odor control agents in compositions that are to be sprayed onto surfaces, the zeolite material preferably has a particle size of less than about 10 microns and is present in the composition at a level of less than about 1% by weight of the composition.

**Activated Carbon**

Activated carbon is another suitable odor control agent for incorporation in the present compositions. The carbon material suitable for use in the present invention is the material well known in commercial practice as an absorbent for organic molecules and/or for air purification purposes. Often, such carbon material is referred to as "activated" carbon or "activated" charcoal.

Such carbon is available from commercial sources under such trade names as; Calgon-Type CPG®; Type PCB®; Type SGL®; Type CAL®; and Type OL®.

When activated carbon is used as an odor control agent in compositions that are to be sprayed onto surfaces, the activated carbon preferably has a particle size of less than about 10 microns and is present in the composition at a level of less than about 1% by weight of the composition.

To the extent any material described herein as an odor control agent might also be classified as another component described herein, for purposes of the present invention, such material shall be classified as an odor control agent.

**Hydrophobically Modified Malodor Control Polymers (HMP)**

The odor control agent can be a Hydrophobically Modified Malodor Control Polymers as described in WO 2012097034.

The composition includes a hydrophobically modified malodor control polymer (HMP). A HMP is formed from a polyamine polymer having a primary, secondary, and/or tertiary amine group that is modified with a hydrophobic group such as an alkyl, alkenyl, alkyloxide, or amide. Although the amine group has been modified, a HMP has at least one free and unmodified primary, secondary, and/or tertiary amine group, to react with malodorous components. Not wishing to be bound by theory, hydrophobic modification may increase a polymer's affinity for hydrophobic odors, thus enabling interactions between the odor molecules and active amine sites. In turn, HMPs may improve the breadth of malodor removal efficacy.

A HMP has the general formula (I):

$$P(R)x \qquad (I)$$

wherein:

P is a polyamine polymer;
R is a C2 to C26 hydrophobic group; and
x is the total degree of substitution, which is less than 100%, of amine sites on the polymer.

1. Polyamine Polymer

HMPs may include a polyamine polymer backbone that can be either linear or cyclic. HMPs can also comprise polyamine branching chains. The polyamine polymer has a general formula (I1):

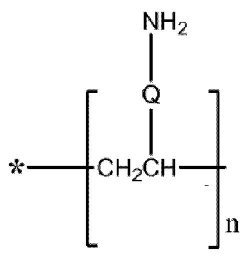

(I1)

where Q is an integer having values between 0-3.

Non-limiting examples of polyamine polymers include polyvinylamines (PVams), polyethyleneimines (PEIs) that are linear or branched, polyamidoamines (PAMams), polyallyamines (PAams), polyetheramines (PEams) or other nitrogen containing polymers, such as lysine, or mixtures of these nitrogen containing polymers.

a. PVams

In one embodiment, the HMP includes a PVam backbone. A PVam is a linear polymer with pendent, primary amine groups directly linked to the main chain of alternating carbons. PVams are manufactured from hydrolysis of poly(N-vinylformamide) (PVNF) which results in the con no groups as described by the following formula (I1a):

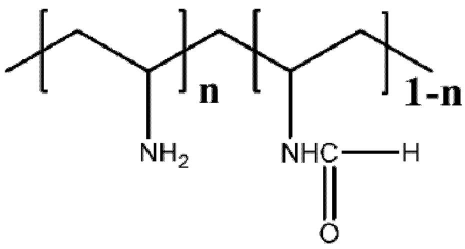

(I1a)

where n is a number from 0.1 to 0.99 depending on the degree of hydrolysis. For instance, in 95% hydrolyzed PVam, n will be 0.95 while 5% of the polymer will have vinylformamide units. PVams may be partially hydrolyzed meaning that 1% to 99%, alternatively 30% to 99%, alternatively 50% to 99%, alternatively 70% to 99%, alternatively 80% to 99%, alternatively 85% to 99%, alternatively 90% to 99%, alternatively 95% to 99%, alternatively 97% to 99%, alternatively 99% of the PVam is hydrolyzed. It has been found that high degree of hydrolysis of PVam increases the resulting polymer's ability to mitigate the odors. PVams that can be hydrolyzed may have an average molecular weight (MW) of 5,000 to 350,000. Suitable hydrolyzed PVams are commercially available from BASF. Some examples include Lupamin(TM) 9095, 9030, 5095, and 1595.

Such hydrolyzed PVams may then be hydrophobically modified. Hydrophobic modification, as described below may further improve malodor removal efficacy,

b. Polyalkylenimine/PEIs

In another embodiment, the HMP includes a polyalkylenimine backbone. Polyalkylenimines include PEIs and polypropylenimines as well as the C4-C12 alkylenimines. PEI is a suitable polyalkylenimine. The chemical structure of a PEI follows a simple principle: one amine function and two carbons. PEIs have the following general formula (11b): $-(CH_2-CH_2-NH)_n-$ (I1b):

where n = 10 - 105

PEIs constitute a large family of water-soluble polyamines of varying molecular weight, structure, and degree of modification. They may act as weak bases and may exhibit a cationic character depending on the extent of protonation driven by pH.

PEIs are produced by the ring-opening cationic polymerization of ethyleneimine as shown below.

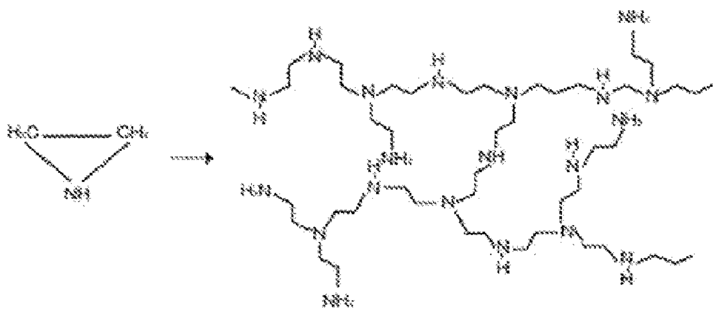

PEIs are believed to be highly branched containing primary, secondary, and tertiary amine groups in the ratio of about 1:2: 1. PEIs may comprise a primary amine range from about 30% to about 40%, alternatively from about 32% to about 38%, alternatively from about 34% to about 36%. PEIs may comprise a secondary amine range from about 30% to about 40%, alternatively from about 32% to about 38%, alternatively from about 34% to about 36%. PEIs may comprise a

tertiary amine range from about 25% to about 35%, alternatively from about 27% to about 33%, alternatively from about 29% to about 31%.

Other routes of synthesis may lead to products with a modified branched chain structure or even to linear chain PEIs. Linear PEIs contain amine sites in the main chain while the branched PEIs contain amines on the main and side chains. Below is an example of a linear PEI

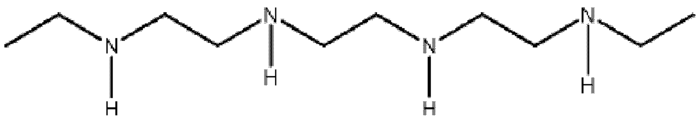

The composition may comprise PEIs having a MW of about 800 to about 2,000,000, alternatively about 1,000 to about 2,000,000, alternatively about 1,200 to about 25,000, alternatively about 1,300 to about 25,000, alternatively about 2,000 to about 25,000, alternatively about 10,000 to about 2,000,000, alternatively about 25,000 to about 2,000,000, alternatively about 25,000.

In one embodiment, the PEI may have a specific gravity of 1.05 and/or an amine value of 18 (mmol/g, solid). For clarity, such specific gravity and/or amine value of the PEI describes the PEI before it is modified or added as part of an aqueous composition. One skilled in the art will appreciate, for example, the primary and secondary amino groups may react with other components of the composition.

Exemplary PEIs include those that are commercially available under the tradename Lupasol® from BASF or the tradename Epomine™ from Nippon Shokubia.

In some embodiments, less than 100% of the active amine sites are substituted with hydrophobic functional groups, alternatively about 0.5% to about 90%, alternatively about 0.5% to about 80%, alternatively about 0.5% to about 70%, alternatively about 0.5% to about 60%, alternatively about 0.5% to about 50%, alternatively about 0.5% to about 40%, alternatively about 0.5% to about 35%, alternatively about 0.5% to about 30%, alternatively about 1% to about 30%, alternatively about alternatively about 1% to about 25%, alternatively about 1% to about 20%, alternatively about 5% to about 20%, alternatively about 10% to about 30%, alternatively about 20% to about 30%, alternatively about 20% of the active amine sites are substituted with hydrophobic functional groups. When a PEI has active amine sites that are fully substituted with hydrophobic functional groups, such hydrophobically modified PEI may have no activity for malodor control.

c. PAMams

In another embodiment, the HMP includes a PAMam backbone. PAMams are polymers whose backbone chain contains both amino functionalities (NH) and amide functionalities (NH- C(O)). PAMams also contain primary amine groups and/or carboxyl groups at the termini of polymer chain. The general structure of a PAMam is below (I1e):

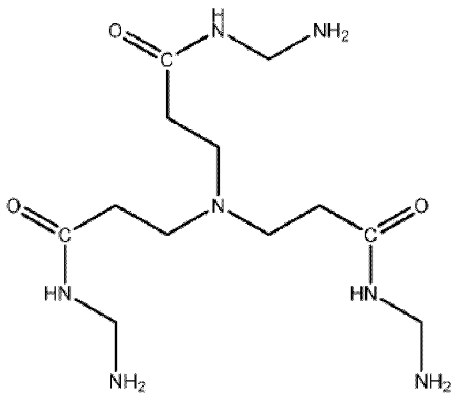

(I1e)

d. PAams

In another embodiment, the HMP includes a PAam backbone. PAams are prepared from polymerization of allyamine- C3H5NH2. Unlike PEIs, they contain only primary amino groups that are for a PAAm is shown below (I1d):

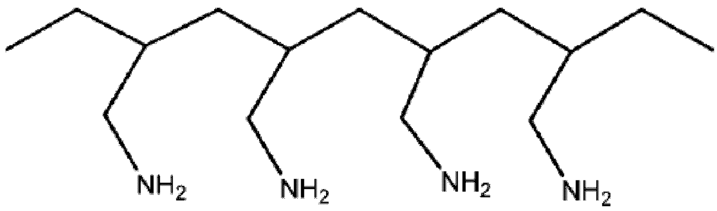

(I1d)

e. PEams

In yet another embodiment, the HMP includes a PEam backbone. PEams contain a primary amino groups attached to the end of a polyether backbone. The polyether backbone may be based on propylene oxide (PO), ethylene oxide (EO), or mixed PO/EO. The general formula for a PEam is shown below (I1e):

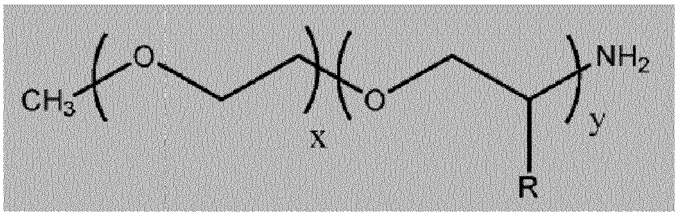

(I1e)

R = H f or (EO) or CH3 for (PO)

These so-called monoamines, M-series, are commercially available from Hunstman under the tradename Jeffamine® monoamines. In another embodiment, the HMP includes a PEam backbone having diamines as shown below (I1f):

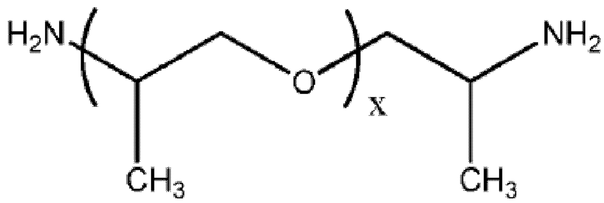

Diamines are commercially available from Hunstman under the tradename Jeffamine<(R)> diamines (e.g. D, ED, and EDR series). The HMP may also include a PEam backbone having triamines (e.g. Jeffamine® triamine T-series).

2. Other Polymer Units

HMPs may include a copolymer of nitrogen-containing polymers having the formula (I2):

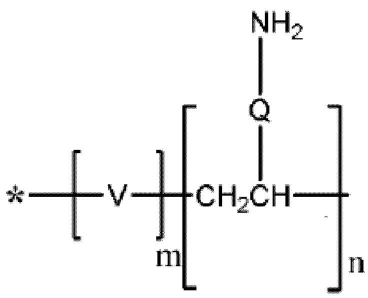

(I2)

where Q is an integer having values between 0-3 and V is a co-monomer. Non-limiting examples of (I2) unmodified polymers include vinylamides, vinyl pyrrolidone, vinylimidazole, vinylesters, vinylalcohols, and mixtures thereof.

3. Hydrophobic Group

The hydrophobic group of the HMP may be linear, branched, or cyclic alkyl, hydroxyalkyl, alkenyl, hydroxyalkenyl, alkyl carboxyl, alkyloxide, alkanediyl, amide, or aryl. In some embodiments, the hydrophobic group is a C2 to C26, alternatively a C2 to C12, alternatively a C2 to CIO, alternatively a C4 to CIO, alternatively a C16 to C26, alternatively a C6. Where cyclodextrin is included in a formulation, it may be desirous to use a HMP that has been modified with a C2 to CIO alkyl group, alternatively a C16-C26 alkyl group, alternatively a C6 alkyl group, since such alkyl groups are cyclodextrin compatible.

4. Hydrophobic Modification

The polyamine backbones are hydrophobically modified in such a manner that at least one nitrogen, alternatively each nitrogen, of the polyamine chain is thereafter described in terms of a unit that is substituted, quaternized, oxidized, or combinations thereof.

There are many ways of hydrophobically modifying polyamine polymers. Generally, the modification is one directed to the primary, secondary, and/or tertiary amines of the polymer. By reacting the unmodified polyamine backbone with appropriate reagents, one can render the polyamine polymer hydrophobic, thereby increasing efficacy for malodor removal. The following are non limiting examples of the ways to prepare the HMPs disclosed herein,

a. Alkoxylation

The reaction of polyamine polymer with an epoxide containing hydrocarbons (R) results in substitution of one or more nitrogen moities on the polymer.

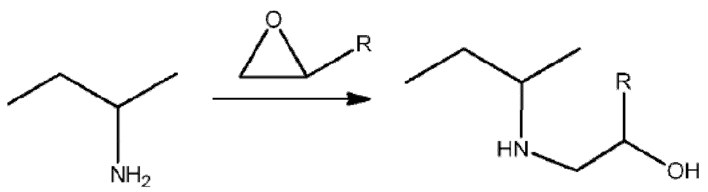

wherein R>C2.

Non-limiting example of such hydrocarbons include C2-C26 chain that is substituted or unsubstituted, branched or unbranched. For example, a reaction of dodeceneoxide with PEI polymer results in C6-HMP disclosed herein having a structure shown below.

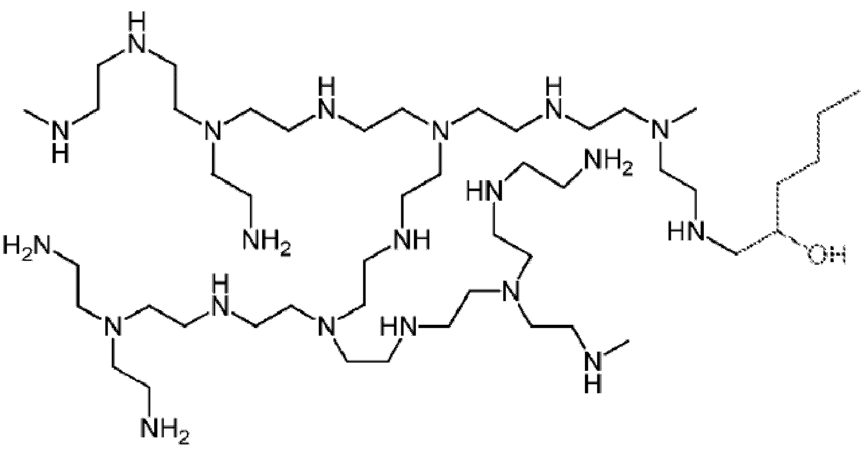

Alternatively, one can modify the base polymer by reacting with EO first and then finish it by alkylation. Additional modifications might also include capping the modified polymer with EO groups if more water solubility is desired. Alternatively, hydroxyl groups can be substituted by further reacting the alkoxylated polymers as described in subparagraph c below,

b. Amidation

Reaction of polyamine polymers with amide-forming reagents such as anhydrides, lactones, isocyanates, or carboxylic acids results in substitution of one or more nitrogen moieties on the polymer rendering hydrophobic character. Prior to amidation, one can begin with partial substitution of amine sites with EO or PO and then carry out amidation on the remaining amine moieties. Reaction of anhydrides with polyamine polymers leads to the formation of amide units of the polymer by partial substitution of the primary/secondary amine sites. Non-limiting examples include non-cyclic carboxylic anhydrides such as acetic anhydride or cyclic carboxylic anhydrides such as maleic anhydride, succinic anhydride or phthalic anhydride. For example, the reaction of a polyamine with acetic anhydride introduces amide units onto the polymer.

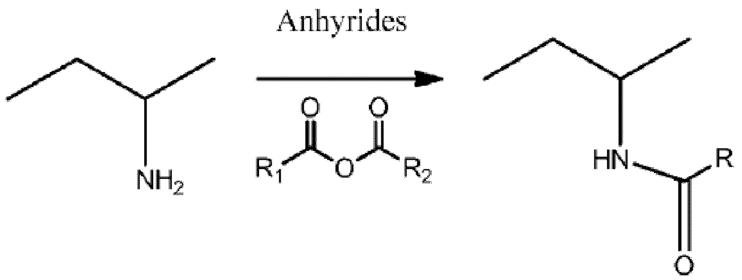


wherein R>C2.

On the other hand, the reaction of polyamine polymer with cyclic anhydrides introduces amido acid units onto the polymer.

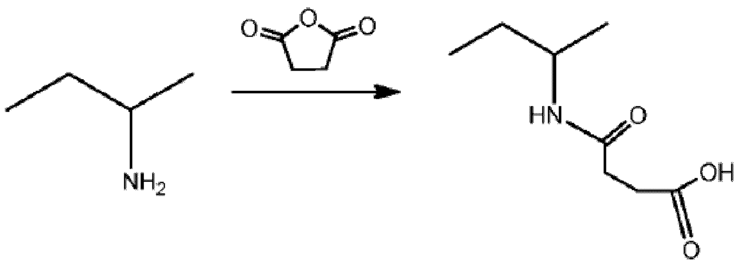

More hydrophobically modified derivatives can be prepared by the use of cyclic anhydrides such as alkylene succinic anhydrides, dodecenyl succinic anhydride or polyisobutane succinic anhydride.

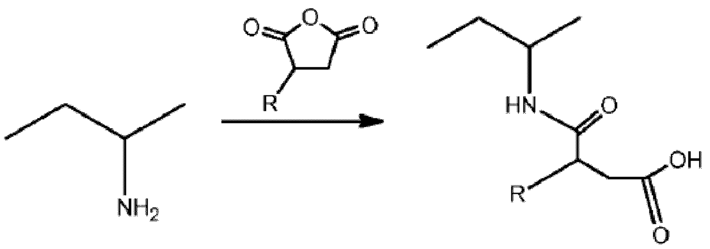

wherein R>C2.

Polyamine polymers containing hydroxyl-terminated polyamido units can be prepared by reacting the polymers with lactones. The use of more hydrophobic alkyl substituted lactones may introduce more hydrophobicity. Optionally, hydroxyl-end groups can be further substituted with functional groups as described in the subparagraph c below.

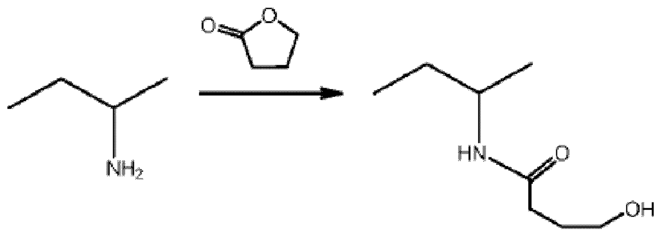

Isocyanate reactions with polyamine polymers result in the formation of urea derivatives shown below.

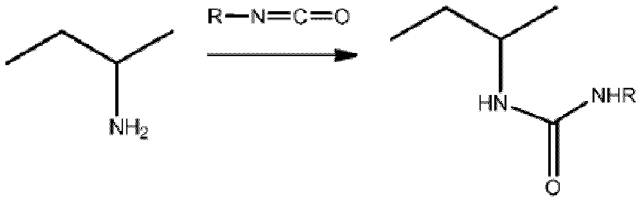


wherein R>C2.

c. Alkoxylation followed by substitution of hydroxyl groups

Additional functional groups can be covalently bonded to an OH group on the alkoxylated polyamine polymers ("x" in formula (I)). This can be achieved by further reacting the alkoxylated polymers with bifunctional compounds such as epihalohydrins such as epichlorohydrin, 2-halo acid halides, isocyanataes or disocyanates such as trimethylhexane diisocyanate, or cyclic carboxylic anhydrides such as maleic anhydride or phthalic anhydride. For example, the reaction of alkoxylated PEI with isocyanates yields:

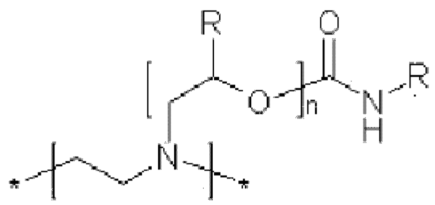

wherein R>C2.

Reaction products of alkoxylated PEI and alk(en)ylsuccinic anhydrides yield

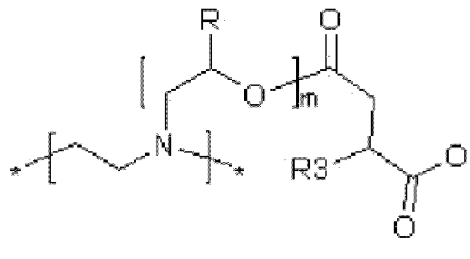

wherein R>C2.

All these HMPs disclosed herein can be optionally capped with hydrophilic groups, such as EO, to render water solubility if necessary.

In some embodiments, about 0.5% to about 90% of the amine groups on the entire unmodified polyamine polymer may be substituted with a hydrophobic group, alternatively about 0.5% to about 80%, alternatively about 0.5% to about 70%, alternatively about 0.5% to about 60%, alternatively about 0.5% to about 50%, alternatively about 0.5% to about 40%, alternatively about 0.5% to about 35%, alternatively about 0.5% to about 30%, alternatively about 1% to about 30%, alternatively about alternatively about 1% to about 25%, alternatively about 1% to about 20%, alternatively about 5% to about 20%, alternatively about 10% to about 30%, alternatively about 20% to about 30%, alternatively about 20% of the amine groups on the entire unmodified polyamine polymer may be substituted with a hydrophobic group. The level of substitution of the amine units can be as low as 0.01 mol percent of the theoretical maximum where all primary, secondary,

and/or tertiary amine units have been replaced. HMPs for use herein may have a MW from about 150 to about $2*10^6$, alternatively from about 400 to about $10^6$, alternatively from about 5000 to about $10^6$.

Malodor control polymers suitable for use in the present invention are water-soluble or dispersible. In some embodiments, the primary, secondary, and/or tertiary amines of the polyamine chain are partially substituted rendering hydrophobicity while maintaining the desired water solubility. The minimum solubility index of a HMP may be about 2% (i.e. 2g/100ml of water). A suitable HMP for an aqueous fabric refresher formulation may have a water solubility percentage of greater than about 0.5% to 100%, alternatively greater than about 5%, alternatively greater than about 10%, alternatively greater than about 20%. The water solubility index can by determined by the Water Solubility test on page 13 of WO 2012/097034.

**Volatile Aldehydes**

The malodor control composition includes volatile aldehydes that neutralize malodors in vapor and/or liquid phase via chemical reactions. Aldehydes that are partially volatile may be considered a volatile aldehyde as used herein. Volatile aldehydes may react with amine-based odors, following the path of Schiff-base formation. Volatiles aldehydes may also react with sulfur-based odors, forming thiol acetals, hemi thiolacetals, and thiol esters in vapor and/or liquid phase. It may be desirable for these vapor and/or liquid phase volatile aldehydes to have virtually no negative impact on the desired perfume character of a product.

Suitable volatile aldehydes may have a vapor pressure (VP) in the range of about 0.0001 torr to 100 torr, alternatively about 0.0001 torr to about 10 torr, alternatively about 0.001 torr to about 50 torr, alternatively about 0.001 torr to about 20 torr, alternatively about 0.001 torr to about 0.100 torr, alternatively about 0.001 torr to 0.06 torr, alternatively about 0.001 torr to 0.03 torr, alternatively about 0.005 torr to about 20 torr, alternatively about 0.01 torr to about 20 torr, alternatively about 0.01 torr to about 15 torr, alternatively about 0.01 torr to about 10 torr, alternatively about 0.05 torr to about 10 torr, measured at 25°C. The volatile aldehydes may also have a certain boiling point (B.P.) and octanol/water partition coefficient (P). The B.P. referred to herein is measured under normal standard pressure of 760 mmHg. The B.P. of many volatile aldehydes, at standard 760 mm Hg are given in, for example, "Perfume and Flavor Chemicals (Aroma Chemicals)," written and published by Steffen Arctander, 1969.

The octanol/water partition coefficient of a volatile aldehyde is the ratio between its equilibrium concentrations in octanol and in water. The partition coefficients of the volatile aldehydes used in the malodor control composition may be more conveniently given in the form of their logarithm to the base 10, logP. The logP values of many volatile aldehydes have been reported. See, e.g., the Pomona92 database, available from Daylight Chemical Information Systems, Inc. (Daylight CIS), Irvine, California. However, the logP values are most conveniently calculated by the "CLOGP" program, also available from Daylight CIS. This program also lists experimental logP values when they are available in the Pomona92 database. The "calculated logP" (ClogP) is determined by the fragment approach of Hansch and Leo (cf. , A. Leo, in Comprehensive Medicinal Chemistry, Vol. 4, C. Hansch, P. G. Sammens, J. B. Taylor and C. A. Ramsden, Eds., p. 295, Pergamon Press, 1990). The fragment approach is based on the chemical structure of each volatile aldehyde, and takes into account the numbers and types of atoms, the atom connectivity, and chemical bonding. The ClogP values, which are the most reliable and widely used estimates for this physicochemical property, are preferably used instead of the experimental logP values in the selection of volatile aldehydes for the malodor control composition.

The ClogP values may be defined by four groups and the volatile aldehydes may be selected from one or more of these groups. The first group comprises volatile aldehydes that have a B.P. of about 250 °C or less and ClogP of about 3 or less. The second group comprises volatile aldehydes that have a B.P. of 250°C or less and ClogP of 3.0 or more. The third group comprises volatile aldehydes that have a B.P. of 250°C or more and ClogP of 3.0 or less. The fourth group comprises volatile aldehydes that have a B.P. of 250°C or more and ClogP of 3.0 or more. The malodor control composition may comprise any combination of volatile aldehydes from one or more of the ClogP groups.

In some embodiments, the malodor control component may comprise, by weight of the malodor control component, from about 0% to about 30% of volatile aldehydes from group 1, alternatively about 25%; and/or about 0% to about 10% of volatile aldehydes from group 2, alternatively about 10%; and/or from about 10% to about 30% of volatile aldehydes from group 3, alternatively about 30%; and/or from about 35% to about 60% of volatile aldehydes from group 4, alternatively about 35%.

The amount of volatile aldehydes that may be formulated in the freshening composition may be from about 0.015% to about 1%, alternatively from about 0.01% to about 0.5%, alternatively from about 0.015% to about 0.3%, by weight of the freshening composition.

Exemplary volatile aldehydes which may be used in a malodor control component include, but are not limited to, Adoxal (2,6,10-Trimethyl-9-undecenal), Bourgeonal (4-t-butylbenzenepropionaldehyde), Lilestralis 33 (2-methyl-4-t-butylphenyl)propanal), Cinnamic aldehyde, cinnamaldehyde (phenyl propenal, 3-phenyl-2-propenal), Citral, Geranial, Neral (dimethyloctadienal, 3,7-dimethyl-2,6-octadien-I-al), Cyclal C (2,4-dimethyl-3-cyclohexen-I- carbaldehyde), Florhydral (3-(3-Isopropyl-phenyl)-butyraldehyde), Citronellal (3,7-dimethyl 6- octenal), Cymal, cyclamen aldehyde, Cyclosal, Lime aldehyde (Alpha-methyl-p-isopropyl phenyl propyl aldehyde), Methyl Nonyl Acetaldehyde, aldehyde C12 MNA (2-methyl-I-undecanal), Hydroxycitronellal, citronellal hydrate (7-hydroxy-3,7-dimethyl octan-I-al), Helional (alpha-methyl-3,4-(methylenedioxy)-hydrocinnamaldehyde, hydrocinnamaldehyde (3-phenylpropanal, 3-phenylpropionaldehyde), Intreleven aldehyde (undec-10-en-I-al), Ligustral, Trivertal (2,4- dimethyl-3-cyclohexene-I-carboxaldehyde), Jasmorange, satinaldehyde (2-methyl-3- tolylproionaldehyde, 4-dimethylbenzenepropanal), Lyral (4-(4-hydroxy-4-methyl pentyl)-3-cyclohexene-I-carboxaldehyde), Melonal (2,6-Dimethyl-5-Heptenal), Methoxy Melonal (6- methoxy-2,6-dimethylheptanal), methoxycinnamaldehyde (trans-4-methoxycinnamaldehyde), Myrac aldehyde isohexenyl cyclohexenyl-carboxaldehyde, trifernal ((3-methyl-4-phenyl propanal, 3-phenyl butanal), lilial, P.T. Bucinal, lysmeral, benzenepropanal (4-tert-butyl-alpha- methyl-hydrocinnamaldehyde), Dupical, tricyclodecylidenebutanal (4-Tricyclo5210- 2,6decylidene-8butanal), Melafleur (1,2,3,4,5, 6,7, 8-octahydro-8,8-dimethyl-2-naphthaldehyde), Methyl Octyl Acetaldehyde, aldehyde C-I I MOA (2-mehtyl deca-I-al), Onicidal (2,6,10- trimethyl-5,9-undecadien-I-al), Citronellyl oxyacetaldehyde, Muguet aldehyde 50 (3,7-dimethyl- 6-octenyl) oxyacetaldehyde), phenylacetaldehyde, Mefranal (3 -methyl- 5 - phenyl pentanal), Triplal, Vertocitral dimethyl tetrahydrobenzene aldehyde (2,4-dimethyl-3-cyclohexene-I-carboxaldehyde), 2-phenylproprionaldehyde, Hydrotropaldehyde, Canthoxal, anisylpropanal 4-methoxy-alpha-methyl benzenepropanal (2-anisylidene propanal), Cylcemone A (1,2,3,4,5,6,7,8-octahydro-8,8-dimethyl-2-naphthaldehyde), and Precylcemone B (1-cyclohexene-I- carboxaldehyde) .

Still other exemplary aldehydes include, but are not limited to, acetaldehyde (ethanal), pentanal, valeraldehyde, amylaldehyde, Scentenal (octahydro-5-methoxy-4,7-Methano-IH-indene-2-carboxaldehyde), propionaldehyde (propanal), Cyclocitral, beta-cyclocitral, (2,6,6- trimethyl-I-cyclohexene-I-acetaldehyde), Iso Cyclocitral (2,4,6-trimethyl-3-cyclohexene-I- carboxaldehyde), isobutyraldehyde, butyraldehyde, isovaleraldehyde (3 -methyl butyraldehyde), methylbutyraldehyde (2-methyl butyraldehyde, 2-methyl butanal), Dihydrocitronellal (3,7- dimethyl octan-I-al), 2-Ethylbutyraldehyde, 3-Methyl-2-butenal, 2-Methylpentanal, 2-Methyl Valeraldehyde, Hexenal (2-hexenal, trans-2-hexenal), Heptanal, Octanal, Nonanal, Decanal, Laurie aldehyde, Tridecanal, 2-Dodecanal, Methylthiobutanal, Glutaraldehyde, Pentanedial, Glutaric aldehyde, Heptenal, cis or trans-Heptenal, Undecenal (2-, 10-), 2,4-octadienal, Nonenal (2-, 6-), Decenal (2-, 4-), 2,4-hexadienal, 2,4-Decadienal, 2,6-Nonadienal, Octenal, 2,6-dimethyl 5-heptenal, 2-isopropyl-5-methyl-2-hexenal, Trifernal, beta methyl Benzenepropanal, 2,6,6- Trimethyl-I-cyclohexene-I-acetaldehyde, phenyl Butenal (2-phenyl 2-butenal), 2.Methyl-3(p- isopropylphenyl)-propionaldehyde, 3-(p-isopropylphenyl)-propionaldehyde, p-Tolylacetaldehyde (4-methylphenylacetaldehyde), Anisaldehyde (p-methoxybenzene aldehyde), Benzaldehyde, Vernaldehyde (I-Methyl-4-(4-methylpentyl)-3-cyclohexenecarbaldehyde), Heliotropin (piperonal) 3,4-Methylene dioxy benzaldehyde, alpha-Amylcinnamic aldehyde, 2-pentyl-3- phenylpropenoic aldehyde, Vanillin (4-methoxy 3 -hydroxy benzaldehyde), Ethyl vanillin (3- ethoxy 4-hydroxybenzaldehyde), Hexyl Cinnamic aldehyde, Jasmonal H (alpha-n-hexylcinnamaldehyde), Floralozone (alpha,alpha-Dimethyl-p-ethylphenylpropanal), Acalea (p-methyl- alpha-pentylcinnamaldehyde), methylcinnamaldehyde, alpha-Methylcinnamaldehyde (2-methyl 3-pheny propenal), alpha-hexylcinnamaldehyde (2-hexyl 3-phenyl propenal), Salicylaldehyde (2-hydroxy benzaldehyde), 4-ethyl benzaldehyde, Cuminaldehyde (4-isopropyl benzaldehyde), Ethoxybenzaldehyde, 2,4-dimethylbenzaldehyde, Veratraldehyde (3,4-dimethoxybenzaldehyde), Syringaldehyde (3,5-dimethoxy 4-hydroxybenzaldehyde), Catechaldehyde (3,4-dihydroxybenzaldehyde), Safranal (2,6,6-trimethyl-I,3-diene methanal), Myrtenal (pin-2-ene-I-carbaldehyde), Perillaldehyde L-4(I-methylethenyl)-I-cyclohexene-I-carboxaldehyde), 2,4- Dimethyl-3-cyclohexene carboxaldehyde, 2-Methyl-2-pentenal, 2-methylpentenal, pyruvaldehyde, formyl Tricyclodecan, Mandarin aldehyde, Cyclemax, Pino acetaldehyde, Corps Iris, Maceal, and Corps 4322.

In one embodiment, the malodor control component includes a mixture of two or more volatile aldehydes selected from the group consisting of 2-ethoxy Benzylaldehyde, 2-isopropyl- 5-methyl-2-hexenal, 5-methyl Furfural, 5-methyl-thiophene-carboxaldehyde, Adoxal, p- anisaldehyde, Benzylaldehyde, Bourgenal, Cinnamic aldehyde, Cymal, Decyl aldehyde, Floral super (4,8-Dimethyldeca-4,9-dienal), Florhydral, Helional, Laurie aldehyde, Ligustral, Lyral, Melonal, o-anisaldehyde, Pino acetaldehyde, P.T. Bucinal, Thiophene carboxaldehyde, trans-4- Decenal, trans trans 2,4-Nonadienal, Undecyl aldehyde, and mixtures thereof.

**Surfactants**

The detergent composition may comprise one or more surfactants, which may be anionic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1% to 60% by weight, such as about 1% to about 40%, or about 3% to about 20%, or about 3% to about 10%. The surfactant(s) is chosen based on the desired cleaning application, and may include any conventional surfactant(s) known in the art.

When included therein, the detergent will usually contain from about 1% to about 40% by weight of an anionic surfactant, such as from about 5% to about 30%, including from about 5% to about 15%, or from about 15% to about 20%, or from about 20% to about 25% of an anionic surfactant. Non-limiting examples of anionic surfactants include sulfates and

sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof.

When included therein, the detergent will usually contain from about 0.2% to about 40% by weight of a nonionic surfactant, for example from about 0.5% to about 30%, in particular from about 1% to about 20%, from about 3% to about 10%, such as from about 3% to about 5%, from about 8% to about 12%, or from about 10% to about 12%. Non-limiting examples of nonionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or *N*-acyl *N*-alkyl derivatives of glucosamine (glucamides, GA, or fatty acid glucamides, FAGA), as well as products available under the trade names SPAN and TWEEN, and combinations thereof.

When included therein, the detergent will usually contain from about 0% to about 40% by weight of a semipolar surfactant. Non-limiting examples of semipolar surfactants include amine oxides (AO) such as alkyldimethylamineoxide, *N*-(coco alkyl)-*N*,*N*-dimethylamine oxide and *N*-(tallow-alkyl)-*N*,*N*-bis(2-hydroxyethyl)amine oxide, , and combinations thereof.

When included therein, the detergent will usually contain from about 0% to about 40% by weight of a zwitterionic surfactant. Non-limiting examples of zwitterionic surfactants include betaines such as alkyldimethylbetaines, sulfobetaines, and combinations thereof.

**Hydrotropes**

A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however the molecular structure of hydrotropes generally do not favor spontaneous self-aggregation, see e.g. review by Hodgdon and Kaler (2007), Current Opinion in Colloid & Interface Science 12: 121-128. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-10% by weight, for example 0-5% by weight, such as about 0.5 to about 5%, or about 3% to about 5%, of a hydrotrope. Any hydrotrope known in the art for use in detergents may be utilized. Non-limiting examples of hydrotropes include sodium benzenesulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

**Builders and Co-Builders**

The detergent composition may contain about 0-65% by weight, such as about 5% to about 50% of a detergent builder or co-builder, or a mixture thereof. The builder and/or co-builder may particularly be a chelating agent that forms water-soluble complexes with Ca and Mg. Any builder and/or co-builder known in the art for use in detergents may be utilized. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (e.g., SKS-6 from Hoechst), ethanolamines such as 2-aminoethan-1-ol (MEA), diethanolamine (DEA, also known as 2,2'-iminodiethan-1-ol), triethanolamine (TEA, also known as 2,2',2''-nitrilotriethan-1-ol), and (carboxymethyl)inulin (CMI), and combinations thereof.

The detergent composition may also contain 0-50% by weight, such as about 5% to about 30%, of a detergent co-builder. The detergent composition may include include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N*,*N*'-disuccinic acid (EDDS), methylglycinediacetic acid (MGDA), glutamic acid-N,N-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), N-(2-hydroxyethyl)iminodiacetic acid (EDG), aspartic acid-*N*-monoacetic acid (ASMA), aspartic acid-*N*,*N*-diacetic acid (ASDA), aspartic acid-*N*-monopropionic acid (ASMP), iminodisuccinic acid (IDA), N-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), *N*-methyliminodiacetic acid (MIDA), α-alanine-*N,N*-diacetic acid (α-ALDA), serine-*N*,*N*-diacetic acid (SEDA), isoserine-*N*,*N*-diacetic acid (ISDA), phenylalanine-*N*,*N*-diacetic acid (PHDA), anthranilic acid-*N,*N-diacetic acid (ANDA), sulfanilic acid-*N*,*N*-diacetic acid (SLDA) , taurine-N,N-diacetic acid (TUDA) and sulfomethyl-*N*,*N*-diacetic acid (SMDA), *N*-(2-hydroxyethyl)ethylenediamine-*N,N',N"*-triacetic acid (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, e.g., WO 09/102854, US 5977053

## Bleaching Systems

The detergent may contain 0-30% by weight, such as about 1% to about 20%, of a bleaching system. Any bleaching system known in the art for use in detergents may be utilized. Suitable bleaching system components include bleaching catalysts, photobleaches, bleach activators, sources of hydrogen peroxide such as sodium percarbonate, sodium perborates and hydrogen peroxide-urea (1:1), preformed peracids and mixtures thereof. Suitable preformed peracids include, but are not limited to, peroxycarboxylic acids and salts, diperoxydicarboxylic acids, perimidic acids and salts, peroxymonosulfuric acids and salts, for example, Oxone (R), and mixtures thereof. Non-limiting examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator. The term bleach activator is meant herein as a compound which reacts with hydrogen peroxide to form a peracid via perhydrolysis. The peracid thus formed constitutes the activated bleach. Suitable bleach activators to be used herein include those belonging to the class of esters, amides, imides or anhydrides. Suitable examples are tetraacetylethylenediamine (TAED), sodium 4-[(3,5,5-trimethylhexanoyl)oxy]benzene-1-sulfonate (ISONOBS), 4-(dodecanoyloxy)benzene-1-sulfonate (LOBS), 4-(decanoyloxy)benzene-1-sulfonate, 4-(decanoyloxy)benzoate (DOBS or DOBA), 4-(nonanoyloxy)benzene-1-sulfonate (NOBS), and/or those disclosed in WO98/17767. A particular family of bleach activators of interest was disclosed in EP624154 and particulary preferred in that family is acetyl triethyl citrate (ATC). ATC or a short chain triglyceride like triacetin has the advantage that it is environmentally friendly Furthermore acetyl triethyl citrate and triacetin have good hydrolytical stability in the product upon storage and are efficient bleach activators. Finally ATC is multifunctional, as the citrate released in the perhydrolysis reaction may function as a builder. Alternatively, the bleaching system may comprise peroxyacids of, for example, the amide, imide, or sulfone type. The bleaching system may also comprise peracids such as 6-(phthalimido) peroxyhexanoic acid (PAP). The bleaching system may also include a bleach catalyst. In some embodiments the bleach component may be an organic catalyst selected from the group consisting of organic catalysts having the following formulae:

(i)

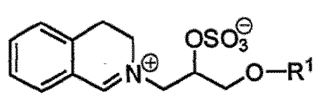

(ii)

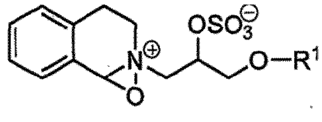

(iii) and mixtures thereof;

wherein each $R^1$ is independently a branched alkyl group containing from 9 to 24 carbons or linear alkyl group containing from 11 to 24 carbons, preferably each $R^1$ is independently a branched alkyl group containing from 9 to 18 carbons or linear alkyl group containing from 11 to 18 carbons, more preferably each $R^1$ is independently selected from the group consisting of 2-propylheptyl, 2-butyloctyl, 2-pentylnonyl, 2-hexyldecyl, dodecyl, tetradecyl, hexadecyl, octadecyl, isononyl, isodecyl, isotridecyl and isopentadecyl. Other exemplary bleaching systems are described, e.g. in WO2007/087258, WO2007/087244, WO2007/087259, EP1867708 (Vitamin K) and WO2007/087242. Suitable photobleaches may for example be sulfonated zinc or aluminium phthalocyanines.

Preferably the bleach component comprises a source of peracid in addition to bleach catalyst, particularly organic bleach catalyst. The source of peracid may be selected from (a) preformed peracid; (b) percarbonate, perborate or persulfate salt (hydrogen peroxide source) preferably in combination with a bleach activator; and (c) perhydrolase enzyme and an ester for forming peracid in situ in the presence of water in a textile treatment step.

**Polymers**

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-N-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated.

**Fabric hueing agents**

The detergent compositions may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO2005/03274, WO2005/03275, WO2005/03276 and EP1876226. The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g. WO 2007/087257 and WO2007/087243.

**Enzymes**

The detergent additive as well as the detergent composition may comprise one or more additional enzymes such as a protease, lipase, cutinase, an amylase, carbohydrase, cellulase, pectinase, mannanase, arabinase, galactanase, xylanase, oxidase, *e.g.*, a laccase, and/or peroxidase.

In general, the properties of the selected enzyme(s) should be compatible with the selected detergent, (*i.e.*, pH-optimum, compatibility with other enzymatic and non-enzymatic ingredients, etc.), and the enzyme(s) should be present in effective amounts.

**Cellulases:**

Suitable cellulases include those of bacterial or fungal origin. Chemically modified or protein engineered mutants

are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and WO99/001544.

Other cellulases are endo-beta-1,4-glucanase enzyme having a sequence of at least 97% identity to the amino acid sequence of position 1 to position 773 of SEQ ID NO:2 of WO 2002/099091 or a family 44 xyloglucanase, which a xyloglucanase enzyme having a sequence of at least 60% identity to positions 40-559 of SEQ ID NO: 2 of WO 2001/062903.

Commercially available cellulases include Celluzyme™, and Carezyme™ (Novozymes A/S) Carezyme Premium™ (Novozymes A/S), Celluclean ™ (Novozymes A/S), Celluclean Classic™ (Novozymes A/S), Cellusoft™ (Novozymes A/S), Whitezyme™ (Novozymes A/S), Clazinase™, and Puradax HA™ (Genencor International Inc.), and KAC-500(B)™ (Kao Corporation).

**Proteases:**

Suitable proteases include those of bacterial, fungal, plant, viral or animal origin e.g. vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from e.g. family M4 or other metalloprotease such as those from M5, M7 or M8 families.

The term "subtilases" refers to a sub-group of serine protease according to Siezen et al., Protein Engng. 4 (1991) 719-737 and Siezen et al. Protein Science 6 (1997) 501-523. Serine proteases are a subgroup of proteases characterized by having a serine in the active site, which forms a covalent adduct with the substrate. The subtilases may be divided into 6 sub-divisions, i.e. the Subtilisin family, the Thermitase family, the Proteinase K family, the Lantibiotic peptidase family, the Kexin family and the Pyrolysin family.

Examples of subtilases are those obtained from *Bacillus* such as *Bacillus lentus, B. alkalophilus, B. subtilis, B. amyloliquefaciens, Bacillus pumilus* and *Bacillus gibsonii* described in; US7262042 and WO09/021867, and *subtilisin lentus, subtilisin Novo, subtilisin Carlsberg, Bacillus licheniformis, subtilisin BPN', subtilisin 309, subtilisin 147* and *subtilisin 168* described in WO89/06279 and protease PD138 described in (WO93/18140). Other useful proteases may be those described in WO92/175177, WO01/016285, WO02/026024 and WO02/016547. Examples of trypsin-like proteases are trypsin (e.g. of porcine or bovine origin) and the *Fusarium* protease described in WO89/06270, WO94/25583 and WO05/040372, and the chymotrypsin proteases obtained from *Cellumonas* described in WO05/052161 and WO05/052146.

A further preferred protease is the alkaline protease from *Bacillus lentus* DSM 5483, as described for example in WO95/23221, and variants thereof which are described in WO92/21760, WO95/23221, EP1921147 and EP1921148.

Examples of metalloproteases are the neutral metalloprotease as described in WO07/044993 (Genencor Int.) such as those obtained from *Bacillus amyloliquefaciens.*

Examples of useful proteases are the variants described in: WO92/19729, WO96/034946, WO98/20115, WO98/20116, WO99/011768, WO01/44452, WO03/006602, WO04/03186, WO04/041979, WO07/006305, WO11/036263, WO11/036264, especially the variants with substitutions in one or more of the following positions: 3, 4, 9, 15, 27, 36, 57, 68, 76, 87, 95, 96, 97, 98, 99, 100, 101, 102, 103, 104, 106, 118, 120, 123, 128, 129, 130, 160, 167, 170, 194, 195, 199, 205, 206, 217, 218, 222, 224, 232, 235, 236, 245, 248, 252 and 274 using the BPN' numbering. More preferred the subtilase variants may comprise the mutations: S3T, V4I, S9R, A15T, K27R, *36D, V68A, N76D, N87S,R, *97E, A98S, S99G,D,A, S99AD, S101G,M,R S103A, V104I,Y,N, S106A, G118V,R, H120D,N, N123S, S128L, P129Q, S130A, G160D, Y167A, R170S, A194P, G195E, V199M, V205I, L217D, N218D, M222S, A232V, K235L, Q236H, Q245R, N252K, T274A (using BPN' numbering).

Suitable commercially available protease enzymes include those sold under the trade names Alcalase®, Duralase™, Durazym™, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Neutrase®, Everlase® and Esperase® (Novozymes A/S), those sold under the tradename Maxatase®, Maxacal®, Maxapem®, Purafect®, Purafect Prime®, Preferenz™, Purafect MA®, Purafect Ox®, Purafect OxP®, Puramax®, Properase®, Effectenz™, FN2®, FN3® , FN4®, Excellase®, Opticlean® and Optimase® (Danisco/DuPont), Axapem™ (Gist-Brocases N.V.), BLAP (sequence shown in Figure 29 of US5352604) and variants hereof (Henkel AG) and KAP *(Bacillus alkalophilus* subtilisin) from Kao.

**Lipases and Cutinases:**

Suitable lipases and cutinases include those of bacterial or fungal origin. Chemically modified or protein engineered mutant enzymes are included. Examples include lipase from *Thermomyces,* e.g. from *T. lanuginosus* (previously named *Humicola lanuginosa*) as described in EP258068 and EP305216, cutinase from *Humicola,* e.g. *H. insolens* (WO96/13580), lipase from strains of *Pseudomonas* (some of these now renamed to *Burkholderia*), e.g. *P. alcaligenes* or *P. pseudoalcaligenes* (EP218272), *P. cepacia* (EP331376), *P. sp.* strain SD705 (WO95/06720 & WO96/27002), *P. wisconsinensis* (WO96/12012), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and *S. pristinaespiralis* (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include include Lipolase™, Lipex™; Lipolex™ and Lipoclean™ (Novozymes A/S), Lumafast (originally from Genencor) and Lipomax (originally from Gist-Brocades).

Still other examples are lipases sometimes referred to as acyltransferases or perhydrolases, e.g. acyltransferases with homology to *Candida antarctica* lipase A (WO10/111143), acyltransferase from *Mycobacterium smegmatis* (WO05/56782), perhydrolases from the CE 7 family (WO09/67279), and variants of the M. *smegmatis* perhydrolase in particular the S54V variant used in the commercial product Gentle Power Bleach from Huntsman Textile Effects Pte Ltd (WO10/100028).

**Amylases:**

Suitable amylases which can be used together with the enzyme of the invention may be an alpha-amylase or a glucoamylase and may be of bacterial or fungal origin. Chemically modified or protein engineered mutants are included. Amylases include, for example, alpha-amylases obtained from *Bacillus, e.g.,* a special strain of *Bacillus licheniformis,* described in more detail in GB 1,296,839.

Suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193.

Other amylases which are suitable are hybrid alpha-amylase comprising residues 1-33 of the alpha-amylase obtained from *B. amyloliquefaciens* shown in SEQ ID NO: 6 of WO 2006/066594 and residues 36-483 of the *B. licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, I201, A209 and Q264. Most preferred variants of the hybrid alpha-amylase comprising residues 1-33 of the alpha-amylase obtained from *B. amyloliquefaciens* shown in SEQ ID NO: 6 of WO 2006/066594 and residues 36-483 of SEQ ID NO: 4 are those having the substitutions:

M197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476, using SEQ ID 2 of WO 96/023873 for numbering. More preferred variants are those having a deletion in two positions selected from 181, 182, 183 and 184, such as 181 and 182, 182 and 183, or positions 183 and 184. Most preferred amylase variants of SEQ ID NO: 1, SEQ ID NO: 2 or SEQ ID NO: 7 are those having a deletion in positions

183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:

N128C+K178L+T182G+Y305R+G475K;
N128C+K178L+T182G+F202Y+Y305R+D319T+G475K;
S125A+N128C+K178L+T182G+Y305R+G475K; or
S125A+N128C+T1311+T1651+K178L+T182G+Y305R+G475K wherein the variants are C-terminally truncated and optionally further comprises a substitution at position 243 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Duramyl™, Termamyl™, Fungamyl™, Stainzyme ™, Stainzyme Plus™, Natalase™, Liquozyme X and BAN™ (from Novozymes A/S), and Rapidase™, Purastar™/Effectenz™, Powerase and Preferenz S100 (from Genencor International Inc./DuPont).

**Peroxidases/Oxidases:**

A peroxidase is a peroxidase enzyme comprised by the enzyme classification EC 1.11.1.7, as set out by the Nomenclature Committee of the International Union of Biochemistry and Molecular Biology (IUBMB), or any fragment obtained therefrom, exhibiting peroxidase activity.

Suitable peroxidases include those of plant, bacterial or fungal origin. Chemically modified or protein engineered mutants are included. Examples of useful peroxidases include peroxidases from *Coprinopsis, e.g.,* from *C. cinerea* (EP 179,486), and variants thereof as those described in WO 93/24618, WO 95/10602, and WO 98/15257.

A peroxidase also includes a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions.

In an embodiment, the haloperoxidase is a chloroperoxidase. Preferably, the haloperoxidase is a vanadium haloperoxidase, *i.e*., a vanadate-containing haloperoxidase. In a preferred method the vanadate-containing haloperoxidase is combined with a source of chloride ion.

Haloperoxidases have been isolated from many different fungi, in particular from the fungus group dematiaceous hyphomycetes, such as *Caldariomyces, e.g., C. fumago, Alternaria, Curvularia, e.g., C. verruculosa* and *C. inaequalis, Drechslera, Ulocladium* and *Botrytis.*

Haloperoxidases have also been isolated from bacteria such as *Pseudomonas, e.g., P. pyrrocinia* and *Streptomyces, e.g., S. aureofaciens.*

In an preferred embodiment, the haloperoxidase is derivable from *Curvularia* sp., in particular *Curvularia verruculosa* or *Curvularia inaequalis,* such as *C. inaequalis* CBS 102.42 as described in WO 95/27046; or *C. verruculosa* CBS 147.63 or *C. verruculosa* CBS 444.70 as described in WO 97/04102; or from *Orechslera hartlebii* as described in WO 01/79459, *Dendryphiella salina* as described in WO 01/79458, *Phaeotrichoconis crotalarie* as described in WO 01/79461, or *Geniculosporium* sp. as described in WO 01/79460.

An oxidase includes, in particular, any laccase enzyme comprised by the enzyme classification EC 1.10.3.2, or any fragment obtained therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

Preferred laccase enzymes are enzymes of microbial origin. The enzymes may be obtained from plants, bacteria or fungi (including filamentous fungi and yeasts).

Suitable examples from fungi include a laccase derivable from a strain of *Aspergillus, Neurospora, e.g., N. crassa, Podospora, Botrytis, Collybia, Fomes, Lentinus, Pleurotus, Trametes, e.g., T. villosa* and *T. versicolor, Rhizoctonia, e.g., R. solani, Coprinopsis, e.g., C. cinerea, C. comatus, C. friesii,* and *C. plicatilis, Psathyrella, e.g., P. condelleana, Panaeolus, e.g., P. papilionaceus, Myceliophthora, e.g., M. thermophila, Schytalidium, e.g., S. thermophilum, Polyporus, e.g., P. pinsitus, Phlebia, e.g., P. radiata* (WO 92/01046), or *Coriolus, e.g., C. hirsutus* (JP 2238885).

Suitable examples from bacteria include a laccase derivable from a strain of *Bacillus.*

A laccase obtained from *Coprinopsis* or *Myceliophthora* is preferred; in particular a laccase obtained from *Coprinopsis cinerea,* as disclosed in WO 97/08325; or from *Myceliophthora thermophila,* as disclosed in WO 95/33836.

The detergent enzyme(s) may be included in a detergent composition by adding separate additives containing one or more enzymes, or by adding a combined additive comprising all of these enzymes. A detergent additive, *i.e.*, a separate additive or a combined additive, can be formulated, for example, as a granulate, liquid, slurry, etc. Preferred detergent additive formulations are granulates, in particular non-dusting granulates, liquids, in particular stabilized liquids, or slurries.

Non-dusting granulates may be produced, e.g. as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

## Other materials

Any detergent components known in the art for use in detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in detergents may be utilized. The choice of such ingredients is well within the skill of the artisan.

## Dispersants

The detergent compositions can also contain dispersants. In particular powdered detergents may comprise dispersants. Suitable water-soluble organic materials include the homo- or copolymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

## Dye Transfer Inhibiting Agents

The detergent compositions may also include one or more dye transfer inhibiting agents. Suitable polymeric dye transfer inhibiting agents include, but are not limited to, polyvinylpyrrolidone polymers, polyamine *N*-oxide polymers, copolymers of *N*-vinylpyrrolidone and *N*-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

## Fluorescent whitening agent

The detergent compositions will preferably also contain additional components that may tint articles being cleaned, such as fluorescent whitening agent or optical brighteners. Where present the brightener is preferably at a level of

about 0.01% to about 0.5%. Any fluorescent whitening agent suitable for use in a laundry detergent composition may be used in the composition. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2*H*-naphtho[1,2-*d*][1,2,3]triazol-2-yl)-2-[(*E*)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%.

**Soil release polymers**

The detergent compositions may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523. Furthermore random graft co-polymers are suitable soil release polymers. Suitable graft co-polymers are described in more detail in WO 2007/138054, WO 2006/108856 and WO 2006/113314. Other soil release polymers are substituted polysaccharide structures especially substituted cellulosic structures such as modified cellulose deriviatives such as those described in EP 1867808 or WO 2003/040279. Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof. The soil release polymer is different from DNase.

**Anti-redeposition agents**

The detergent compositions may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents. The anti redeposition polymer is different from DNase

**Rheology Modifiers**

The detergent compositions may also include one or more rheology modifiers, structurants or thickeners, as distinct from viscosity reducing agents. The rheology modifiers are selected from the group consisting of non-polymeric crystalline, hydroxy-functional materials, polymeric rheology modifiers which impart shear thinning characteristics to the aqueous liquid matrix of a liquid detergent composition. The rheology and viscosity of the detergent can be modified and adjusted by methods known in the art, for example as shown in EP 2169040.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

**Formulation of detergent products**

The detergent composition may be in any convenient form, e.g., a bar, a homogenous tablet, a tablet having two or more layers, a pouch having one or more compartments, a regular or compact powder, a granule, a paste, a gel, or a

regular, compact or concentrated liquid.

Pouches can be configured as single or multicompartments. It can be of any form, shape and material which is suitable for hold the composition, e.g. without allowing the release of the composition to release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be of blended compositions comprising hydrolytically degradable and water soluble polymer blends such as polylactide and polyvinyl alcohol (known under the Trade reference M8630 as sold by MonoSol LLC, Indiana, USA) plus plasticisers like glycerol, ethylene glycerol, propylene glycol, sorbitol and mixtures thereof. The pouches can comprise a solid laundry cleaning composition or part components and/or a liquid cleaning composition or part components separated by the water soluble film. The compartment for liquid components can be different in composition than compartments containing solids: US2009/0011970 A1.

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

A liquid or gel detergent , which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent.

A liquid or gel detergent may be non-aqueous.

**Laundry soap bars**

The DNase of the invention may be added to laundry soap bars and used for hand washing laundry, fabrics and/or textiles. The term laundry soap bar includes laundry bars, soap bars, combo bars, syndet bars and detergent bars. The types of bar usually differ in the type of surfactant they contain, and the term laundry soap bar includes those containing soaps from fatty acids and/or synthetic soaps. The laundry soap bar has a physical form which is solid and not a liquid, gel or a powder at room temperature. The term solid is defined as a physical form which does not significantly change over time, i.e. if a solid object (e.g. laundry soap bar) is placed inside a container, the solid object does not change to fill the container it is placed in. The bar is a solid typically in bar form but can be in other solid shapes such as round or oval.

The laundry soap bar may contain one or more additional enzymes, protease inhibitors such as peptide aldehydes (or hydrosulfite adduct or hemiacetal adduct), boric acid, borate, borax and/or phenylboronic acid derivatives such as 4-formylphenylboronic acid, one or more soaps or synthetic surfactants, polyols such as glycerine, pH controlling compounds such as fatty acids, citric acid, acetic acid and/or formic acid, and/or a salt of a monovalent cation and an organic anion wherein the monovalent cation may be for example $Na^+$, $K^+$ or $NH_4^+$ and the organic anion may be for example formate, acetate, citrate or lactate such that the salt of a monovalent cation and an organic anion may be, for example, sodium formate.

The laundry soap bar may also contain complexing agents like EDTA and HEDP, perfumes and/or different type of fillers, surfactants e.g. anionic synthetic surfactants, builders, polymeric soil release agents, detergent chelators, stabilizing agents, fillers, dyes, colorants, dye transfer inhibitors, alkoxylated polycarbonates, suds suppressers, structurants, binders, leaching agents, bleaching activators, clay soil removal agents, anti-redeposition agents, polymeric dispersing agents, brighteners, fabric softeners, perfumes and/or other compounds known in the art.

The laundry soap bar may be processed in conventional laundry soap bar making equipment such as but not limited to: mixers, plodders, e.g a two stage vacuum plodder, extruders, cutters, logo-stampers, cooling tunnels and wrappers. A premix may be added to the soap at different stages of the process. For example, the premix containing a soap, DNase, optionally one or more additional enzymes, a protease inhibitor, and a salt of a monovalent cation and an organic anion may be prepared and and the mixture is then plodded. The DNase and optional additional enzymes may be added at the same time as the protease inhibitor for example in liquid form. Besides the mixing step and the plodding step, the process may further comprise the steps of milling, extruding, cutting, stamping, cooling and/or wrapping.

**Formulation of enzyme in co-granule**

The DNase may be formulated as a granule for example as a co-granule that combines one or more enzymes.

Each enzyme will then be present in more granules securing a more uniform distribution of enzymes in the detergent. This also reduces the physical segregation of different enzymes due to different particle sizes. Methods for producing multi-enzyme co-granulates for the detergent industry are disclosed in the IP.com disclosure IPCOM000200739D.

Another example of formulation of enzymes by the use of co-granulates are disclosed in WO 2013/188331, which relates to a detergent composition comprising (a) a multi-enzyme co- granule; (b) less than 10 wt zeolite (anhydrous basis); and (c) less than 10 wt phosphate salt (anhydrous basis), wherein said enzyme co-granule comprises from 10 to 98 wt% moisture sink component and the composition additionally comprises from 20 to 80 wt% detergent moisture sink component. WO 2013/188331 also relates to a method of treating and/or cleaning a surface, preferably a fabric surface comprising the steps of (i) contacting said surface with the detergent composition as claimed and described herein in an aqueous wash liquor, (ii) rinsing and/or drying the surface.

The multi-enzyme co-granule may comprise a DNase and (a) one or more enzymes selected from the group consisting of first- wash lipases, cleaning cellulases, xyloglucanases, perhydrolases, peroxidases, lipoxygenases, laccases and mixtures thereof; and (b) one or more enzymes selected from the group consisting of hemicellulases, proteases, care cellulases, cellobiose dehydrogenases, xylanases, phospho lipases, esterases, cutinases, pectinases, mannanases, pectate lyases, keratinases, reductases, oxidases, phenoloxidases, ligninases, pullulanases, tannases, pentosanases, lichenases glucanases, arabinosidases, hyaluronidase, chondroitinase, amylases, and mixtures thereof.

## Assays and detergent compositions

### Detergent compositions

The below mentioned detergent composition can be used in combination with the polypeptide of the invention for preventing or reducing static electricity.

### Biotex black (liquid)

5-15% Anionic surfactants, <5% Nonionic surfactants, perfume, enzymes, DMDM and hydantoin.

### Composition of Ariel Sensitive White & Color, liquid detergent composition:

Aqua, Alcohol Ethoxy Sulfate, Alcohol Ethoxylate, Amino Oxide, Citrid Acid, C12-18 topped palm kernel fatty acid, Protease, Glycosidase, Amylase, Ethanol, 1,2 Propanediol, Sodium Formate, Calcium Chloride, Sodium hydroxide, Silicone Emulsion, Trans-sulphated EHDQ (the ingredients are listed in descending order).

### Composition of WFK IEC-A model detergent (powder)

Ingredients: Linear sodium alkyl benzene sulfonate 8,8 %, Ethoxylated fatty alcohol C12-18 (7 EO) 4,7 %, Sodium soap 3,2 %, Anti foam DC2-4248S 3,9 %, Sodium aluminium silicate zeolite 4A 28,3 %, Sodium carbonate 11,6 %, Sodium salt of a copolymer from acrylic and maleic acid (Sokalan CP5) 2,4 %, Sodium silicate 3,0 %, Carboxymethylcellulose 1,2 %, Dequest 2066 2,8 %, Optical whitener 0,2 %, Sodium sulfate6,5 %, Protease 0,4 %.

### Composition of model detergent A (liquid)

Ingredients: 12% LAS, 11% AEO Biosoft N25-7 (NI), 7% AEOS (SLES), 6% MPG (monopropylene glycol), 3% ethanol, 3% TEA, 2.75% cocoa soap, 2.75% soya soap, 2% glycerol, 2% sodium hydroxide, 2% sodium citrate, 1% sodium formiate, 0.2% DTMPA and 0.2% PCA (all percentages are w/w)

### Composition of Ariel Actilift (liquid)

Ingredients: 5-15% Anionic surfactants; <5% Non-ionic surfactants, Phosphonates, Soap; Enzymes, Optical brighteners, Benzisothiazolinone, Methylisothiazolinone, Perfumes, Alpha-isomethyl ionone, Citronellol, Geraniol, Linalool.

### Composition of Ariel Actilift Colour&Style (liquid)

Ingredients: 5-15% Anionic surfactants; <5% Non-ionic surfactants, Phosphonates, Soap; Enzymes, Perfumes, Benzisothiazolinone, Methylisothiazolinone, Alpha-isomethyl ionone, Butylphenyl methylpropional, Citronellol, Geraniol, Linalool.

**Composition of Ariel Actilift Colour & Style**

Aqua, Sodium Dodecylbenzenesulfonate, C14-C15 Pareth-7, Sodium Citrate, Propylene Glycol, Sodium Palm Kernelate, Sodium Laureth Sulfate, MEA Dodecylbenzenesulfonage, Sulfated Ethoxylated Hexamethylenediamine Quaternized, Sodium Cumenesulfonate, Perfume, Co-polymer of PEG/Vinyl Acetate, Sodium formate, Hydrogenated Castor Oil, Sodium Diethylenetriamine Pentamethylene Phosphonate, PEG/PPG-10/2 Propylheptyl Ether, Butyophenyl Methylpropional, Polyvinylpyridine-N-Oxide, Sorbitol, Glycerin, Ethanolamine, Sodium Hydroxide, Alpha-Isomethyl Ionone, Protease, Calcium Chloride, Geraniol, Linalool, Citronelllol, Tripropylene Glycol, Glycosidase, Benzisothiazolinone, Dimethicone, Glycosidase, Sodium Acetate, Cellulase, Colorant, Glyceryl Stearate, Hydroxyethylcellulose, Silica.

**Composition of Ariel Actilift Colour & Style, new pack**

Ingredients: Aqua, Sodium Laureth Sulfate, Propylene Glycol, C14-C15 Pareth-7, Sodium citrate, Sodium Palm Kernelate, Alcohol, Sodium Formate, Sulfated Ethoxylated Hexamethylenediamine Quaternized, Sodium Hydroxide, Perfume, Polyvinylpyridine-N-Oxide, Sorbitol, Calcium Chloride, protease, Glycerin, Glucosidase, Glycosidase, Sodium Acetate, Colorant, Cellulase.

**Composition of Ariel Actilift Whites & Colours Coolclean, new pack**

Ingredients: Aqua, Sodium Laureth Sulfate, Propylene Glycol, C14-C15 Pareth-7, Sodium citrate, Sodium Palm Kernelate, Alcohol, Sodium Formate, Sulfated Ethoxylated Hexamethylenediamine Quaternized, Sodium Hydroxide, Perfume, Sorbitol, Calcium Chloride, protease, Glycerin, Glucosidase, Glycosidase, Sodium Acetate, Colorant, Cellulase.

**Composition of Ariel Sensitive White & Color**

Ingredients: Aqua, Sodium Laureth Sulfate, Propylene Glycol, C14-C15 Pareth-7, Sodium citrate, Sodium Palm Kernelate, Alcohol, Sodium Formate, Sulfated Ethoxylated Hexamethylenediamine Quaternized, Sodium Hydroxide, , Sorbitol, Calcium Chloride, protease, Glycerin, Glycosidase, Sodium Acetate, Cellulase, Silica.

**Composition of Ariel Actilift, regular**

Aqua, Sodium Dodecylbenzenesulfonate, C14-C15 Pareth-7, Sodium Citrate, Propylene Glycol, Sodium Palm Kernelate, Sodium Laureth Sulfate, MEA Dodecylbenzenesulfonage, Sulfated Ethoxylated Hexamethylenediamine Quaternized, Sodium Cumenesulfonate, Perfume, Co-polymer of PEG/Vinyl Acetate, Sodium formate, C12-C14 Pareth-7, Hydrogenated Castor Oil, Sodium Diethylenetriamine Pentamethylene Phosphonate, PEG/PPG-10/2 Propylheptyl Ether, Butyophenyl Methylpropional, Fluorescent Brightener 9, Sorbitol, Glycerin, Ethanolamine, Sodium Hydroxide, Alpha-Isomethyl Ionone, Protease, Calcium Chloride, Geraniol, Linalool, Citronelllol, Tripropylene Glycol, Sodium Chloride, Glycosidase, Benzisothiazolinone, Dimethicone, Glycosidase, Sodium Acetate, Cellulase, Colorant, Glyceryl Stearate, Hydroxyethylcellulose, Silica.

**Composition of Persil Small & Mighty (liquid)**

Ingredients: 15-30% Anionic surfactants, Non-ionic surfacts, 5-15% Soap, < 5% Polycarboxylates, Perfume, Phosphates, Optical Brighteners

**Persil 2 in1 with Comfort Passion Flower Powder**

Sodium sulfate, Sodium carbonate, Sodium Dodecylbenzenesulfonate, Bentonite, Sodium Carbonate Peroxide, Sodium Silicate, Zeolite, Aqua, Citric acid, TAED, C12-15 Pareth-7, Stearic Acid, Parfum, Sodium Acrylic Acid/MA Copolymer, Cellulose Gum, Corn Starch Modified, Sodium chloride, Tetrasodium Etidronate, Calcium Sodium EDTMP, Disodium Anilinomorpholinotriazinylaminostilbenesulfonate, Sodium bicarbonate, Phenylpropyl Ethyl Methicone, Butylphenyl Methylpropional, Glyceryl Stearates, Calcium carbonate, Sodium Polyacrylate, Alpha-Isomethyl Ionone, Disodium Distyrylbiphenyl Disulfonate, Cellulose, Protease, Limonene, PEG-75, Titanium dioxide, Dextrin, Sucrose, Sodium Polyaryl Sulphonate, CI 12490, CI 45100, CI 42090, Sodium Thiosulfate, CI 61585.

**Persil Biological Powder**

Sucrose, Sorbitol, Aluminum Silicate, Polyoxymethylene Melamine, Sodium Polyaryl Sulphonate, CI 61585, CI 45100, Lipase, Amylase, Xanthan gum, Hydroxypropyl methyl cellulose, CI 12490, Disodium Distyrylbiphenyl Disulfonate, Sodium Thiosulfate, CI 42090, Mannanase, CI 11680, Etidronic Acid, Tetrasodium EDTA.

**Persil Biological Tablets**

Sodium carbonate, Sodium Carbonate Peroxide, Sodium bicarbonate, Zeolite, Aqua, Sodium Silicate, Sodium Lauryl Sulfate, Cellulose, TAED, Sodium Dodecylbenzenesulfonate, Hemicellulose, Lignin, Lauryl Glucoside, Sodium Acrylic Acid/MA Copolymer, Bentonite, Sodium chloride, Parfum, Tetrasodium Etidronate, Sodium sulfate, Sodium Polyacrylate, Dimethicone, Disodium Anilinomorpholinotriazinylaminostilbenesulfonate, Dodecylbenzene Sulfonic Acid, Trimethylsiloxysilicate, Calcium carbonate, Cellulose, PEG-75, Titanium dioxide, Dextrin, Protease, Corn Starch Modified, Sucrose, CI 12490, Sodium Polyaryl Sulphonate, Sodium Thiosulfate, Amylase, Kaolin,

**Persil Colour Care Biological Powder**

Subtilisin, Imidazolidinone, Hexyl Cinnamal, Sucrose, Sorbitol, Aluminum Silicate, Polyoxymethylene Melamine, CI 61585, CI 45100, Lipase, Amylase, Xanthan gum, Hydroxypropyl methyl cellulose, CI 12490, Disodium Distyrylbiphenyl Disulfonate, Sodium Thiosulfate, CI 42090, Mannanase, CI 11680, Etidronic Acid, Tetrasodium EDTA.

**Persil Colour Care Biological Tablets**

Sodium bicarbonate, Sodium carbonate, Zeolite, Aqua, Sodium Silicate, Sodium Lauryl Sulfate, Cellulose Gum, Sodium Dodecylbenzenesulfonate, Lauryl Glucoside, Sodium chloride, Sodium Acrylic Acid/MA Copolymer, Parfum, Sodium Thioglycolate, PVP, Sodium sulfate, Tetrasodium Etidronate, Sodium Polyacrylate, Dimethicone, Bentonite, Dodecylbenzene Sulfonic Acid, Trimethylsiloxysilicate, Calcium carbonate, Cellulose, PEG-75, Titanium dioxide, Dextrin, Protease, Corn Starch Modified, Sucrose, Sodium Thiosulfate, Amylase, CI 74160, Kaolin.

**Persil Dual Action Capsules Bio**

MEA-Dodecylbenzenesulfonate, MEA-Hydrogenated Cocoate, C12-15 Pareth-7, Dipropylene Glycol, Aqua, Tetrasodium Etidronate, Polyvinyl Alcohol, Glycerin, Aziridine, homopolymer ethoxylated, Propylene glycol, Parfum, Sodium Diethylenetriamine Pentamethylene Phosphonate, Sorbitol, MEA-Sulfate, Ethanolamine, Subtilisin, Glycol, Butylphenyl Methylpropional, Boronic acid, (4-formylphenyl), Hexyl Cinnamal, Limonene, Linalool, Disodium Distyrylbiphenyl Disulfonate, Alpha-Isomethyl Ionone, Geraniol, Amylase, Polymeric Blue Colourant, Polymeric Yellow Colourant, Talc, Sodium chloride, Benzisothiazolinone, Mannanase, Denatonium Benzoate.

**Persil 2 in1 with Comfort Sunshiny Days Powder**

Sodium sulfate, Sodium carbonate, Sodium Dodecylbenzenesulfonate, Bentonite, Sodium Carbonate Peroxide, Sodium Silicate, Zeolite, Aqua, Citric acid, TAED, C12-15 Pareth-7, Parfum, Stearic Acid, Sodium Acrylic Acid/MA Copolymer, Cellulose Gum, Com Starch Modified, Sodium chloride, Tetrasodium Etidronate, Calcium Sodium EDTMP, Disodium Anilinomorpholinotriazinylaminostilbenesulfonate, Sodium bicarbonate, Phenylpropyl Ethyl Methicone, Butylphenyl Methylpropional, Glyceryl Stearates, Calcium carbonate, Sodium Polyacrylate, Geraniol, Disodium Distyrylbiphenyl Disulfonate, Cellulose, Protease, PEG-75, Titanium dioxide, Dextrin, Sucrose, Sodium Polyaryl Sulphonate, CI 12490, CI 45100, CI 42090, Sodium Thiosulfate, CI 61585.

**Persil Small & Mighty 2in1 with Comfort Sunshiny Days**

Aqua, C12-15 Pareth-7, Sodium Dodecylbenzenesulfonate, Propylene glycol, Sodium Hydrogenated Cocoate, Triethanolamine, Glycerin, TEA-Hydrogenated Cocoate, Parfum, Sodium chloride, Polyquaternium-10, PVP, Polymeric Pink Colourant, Sodium sulfate, Disodium Distyrylbiphenyl Disulfonate, Butylphenyl Methylpropional, Styrene/Acrylates Copolymer, Hexyl Cinnamal, Citronellol, Eugenol, Polyvinyl Alcohol, Sodium acetate, Isopropyl alcohol, Polymeric Yellow Colourant, Sodium Lauryl Sulfate.

**Persil Small & Mighty Bio**

Aqua, MEA-Dodecylbenzenesulfonate, Propylene glycol, Sodium Laureth Sulfate, C12-15 Pareth-7, TEA-Hydrogenated Cocoate, MEA-Citrate, Aziridine homopolymer ethoxylated, MEA-Etidronate, Triethanolamine, Parfum, Acrylates Copolymer, Sorbitol, MEA-Sulfate, Sodium Sulfite, Disodium Distyrylbiphenyl Disulfonate, Butylphenyl Methylpropional, Styrene/Acrylates Copolymer, Citronellol, Sodium sulfate, Peptides, salts, sugars from fermentation (process), Subtilisin, Glycerin, Boronic acid, (4-formylphenyl), Geraniol, Pectate Lyase, Amylase, Sodium Lauryl Sulfate, Mannanase, CI 42051.

**Persil Small & Mighty Capsules Biological**

MEA-Dodecylbenzenesulfonate, MEA-Hydrogenated Cocoate, C12-15 Pareth-7, Dipropylene Glycol, Aqua, Glycerin, Polyvinyl Alcohol, Parfum, Aziridine homopolymer ethoxylated, Sodium Diethylenetriamine Pentamethylene Phosphonate, Propylene glycol, Sorbitol, MEA-Sulfate, Ethanolamine, Subtilisin, Glycol, Butylphenyl Methylpropional, Hexyl Cinnamal, Starch, Boronic acid, (4-formylphenyl), Limonene, Linalool, Disodium Distyrylbiphenyl Disulfonate, Alpha-Isomethyl Ionone, Geraniol, Amylase, Talc, Polymeric Blue Colourant, Sodium chloride, Benzisothiazolinone, Denatonium Benzoate, Polymeric Yellow Colourant, Mannanase.

**Persil Small & Mighty Capsules Colour Care**

MEA-Dodecylbenzenesulfonate, MEA-Hydrogenated Cocoate, C12-15 Pareth-7, Dipropylene Glycol, Aqua, Glycerin, Polyvinyl Alcohol, Parfum, Aziridine homopolymer ethoxylated, Sodium Diethylenetriamine Pentamethylene Phosphonate, Propylene glycol, MEA-Sulfate, Ethanolamine, PVP, Sorbitol, Butylphenyl Methylpropional, Subtilisin, Hexyl Cinnamal, Starch, Limonene, Linalool, Boronic acid, (4-formylphenyl), Alpha-Isomethyl Ionone, Geraniol, Talc, Polymeric Blue Colourant, Denatonium Benzoate, Polymeric Yellow Colourant.

**Persil Small & Mighty Colour Care**

Aqua, MEA-Dodecylbenzenesulfonate, Propylene glycol, Sodium Laureth Sulfate, C12-15 Pareth-7, TEA-Hydrogenated Cocoate, MEA-Citrate, Aziridine homopolymer ethoxylated, MEA-Etidronate, Triethanolamine, Parfum, Acrylates Copolymer, Sorbitol, MEA-Sulfate, Sodium Sulfite, Glycerin, Butylphenyl Methylpropional, Citronellol, Sodium sulfate, Peptides, salts, sugars from fermentation (process),Styrene/Acrylates Copolymer, Subtilisin, Boronic acid, (4-formylphenyl), Geraniol, Pectate Lyase, Amylase, Sodium Lauryl Sulfate, Mannanase, CI 61585, CI 45100.

**Composition of Fairy Non Bio (liquid)**

Ingredients: 15-30% Anionic Surfactants,5-15% Non-Ionic Surfactants, Soap, Benzisothiazolinone, Methylisothiazolinone, Perfumes

**Composition of Model detergent T (powder)**

Ingredients: 11% LAS, 2% AS/AEOS, 2% soap, 3% AEO, 15.15% sodium carbonate, 3% sodium slilcate, 18.75% zeolite, 0.15% chelant, 2% sodium citrate, 1.65% AA/MA copolymer, 2.5% CMC and 0.5% SRP (all percentages are w/w).

**Composition of Model detergent X (powder)**

Ingredients: 16.5% LAS, 15% zeolite, 12% sodium disilicate, 20% sodium carbonate, 1% sokalan, 35.5% sodium sulfate (all percentages are w/w).

**Composition of Ariel Actilift Colour & Style (powder)**

Ingredients: 15-30% Anionic surfactants, <5% Non-ionic surfactants, Phosphonates, Polycarboxylates, Zeolites; Enzymes, Perfumes, Hexyl cinnamal.

**Composition of Ariel Actilift (powder)**

Ingredients: 5-15% Anionic surfactants, Oxygen-based bleaching agents, <5% Non-ionic surfactants, Phosphonates, Polycarboxylates, Zeolites, Optical brightners, Enzymes, Perfumes, Butylphenyl Methylpropional, Coumarin, Hexyl Cinnamal

**Composition of Persil Megaperls (powder)**

Ingredients: 15 - 30 % of the following: anionic surfactants, oxygen-based bleaching agent and zeolites, less than 5 % of the following: non-ionic surfactants, phosphonates, polycarboxylates, soap, Further ingredients: Perfumes, Hexyl cinnamal, Benzyl salicylate, Linalool, optical brighteners, Enzymes and Citronellol.

**Gain Liquid, Original:**

Ingredients: Water, Alcohol Ethoxysulfate, Diethylene Glycol, Alcohol Ethoxylate, Ethanolamine, Linear Alkyl Benzene Sulfonate, Sodium Fatty Acids, Polyethyleneimine Ethoxylate, Citric Acid, Borax, Sodium Cumene Sulfonate, Propylene Glycol, DTPA, Disodium Diaminostilbene Disulfonate, Dipropylethyl Tetramine, Sodium Hydroxide, Sodium Formate, Calcium Formate, Dimethicone, Amylase, Protease, Liquitint™, Hydrogenated Castor Oil, Fragrance

**Tide Liquid, Original:**

Ingredients: Linear alkylbenzene sulfonate , propylene glycol, citric acid, sodium hydroxide, borax, ethanolamine, ethanol , alcohol sulfate, polyethyleneimine ethoxylate, sodium fatty acids, diquaternium ethoxysulfate, protease, diethylene glycol, laureth-9, alkyldimethylamine oxide, fragrance, amylase, disodium diaminostilbene disulfonate, DTPA, sodium formate, calcium formate, polyethylene glycol 4000, mannanase, Liquitint™ Blue, dimethicone.

**Liquid Tide, Free and Gentle:** Water, sodium alcoholethoxy sulfate, propylene glycol, borax, ethanol, linear alkylbenzene sulfonate sodium, salt, polyethyleneimine ethoxylate, diethylene glycol, trans sulfated & ethoxylated hexamethylene diamine, alcohol ethoxylate, linear alkylbenzene sulfonate, MEA salt, sodium formate, sodium alkyl sulfate, DTPA, amine oxide, calcium formate, disodium diaminostilbene, disulfonate, amylase, protease, dimethicone, benzisothiazolinone

**Tide Coldwater Liquid, Fresh Scent:** Water, alcoholethoxy sulfate, linear alkylbenzene sulfonate, diethylene glycol, propylene glycol, ethanolamine, citric acid, Borax, alcohol sulfate, sodium hydroxide, polyethyleneimine, ethoxylate, sodium fatty acids, ethanol, protease, Laureth-9, diquaternium ethoxysulfate, lauramine oxide, sodium cumene, sulfonate, fragrance, DTPA, amylase, disodium, diaminostilbene, disulfonate, sodium formate, disodium distyrylbiphenyl disulfonate, calcium formate, polyethylene glycol 4000, mannanase, pectinase, Liquitint™ Blue, dimethicone

**Tide TOTALCARE™ Liquid, Cool Cotton:** Water, alcoholethoxy sulfate, propylene glycol, sodium fatty acids, laurtrimonium chloride, ethanol, sodium hydroxide, sodium cumene sulfonate, citric acid, ethanolamine, diethylene glycol, silicone polyether, borax, fragrance, polyethyleneimine ethoxylate, protease, Laureth-9,

DTPA, polyacrylamide quaternium chloride, disodium diaminostilbene disulfonate, sodium formate, Liquitint™ Orange, dipropylethyl tetraamine, dimethicone, cellulase,

**Liquid Tide Plus Bleach Alternative™, Vivid White and Bright, Original and Clean Breeze:** Water, sodium alcoholethoxy sulfate, sodium alkyl sulfate, MEA citrate, linear alkylbenzene sulfonate, MEA salt, propylene glycol, diethylene glycol, polyethyleneimine ethoxylate, ethanol, sodium fatty acids, ethanolamine, lauramine oxide, borax, Laureth-9, DTPA, sodium cumene sulfonate, sodium formate, calcium formate, linear alkylbenzene sulfonate, sodium salt, alcohol sulfate, sodium hydroxide, diquaternium ethoxysulfate, fragrance, amylase, protease, mannanase, pectinase, disodium diaminostilbene disulfonate, benzisothiazolinone, Liquitint™ Blue, dimethicone, dipropylethyl tetraamine.

**Liquid Tide HE, Original Scent:** Water, Sodium alcoholethoxy sulfate, MEA citrate, Sodium Alkyl Sulfate, alcohol ethoxylate, linear alkylbenzene sulfonate, MEA salt, sodium fatty acids, polyethyleneimine ethoxylate, diethylene glycol, propylene glycol, diquaternium ethoxysulfate, borax, polyethyleneimine, ethoxylate propoxylate, ethanol, sodium cumene sulfonate, fragrance, DTPA, disodium diaminostilbene disulfonate, Mannanase, cellulase, amylase, sodium formate, calcium formate, Lauramine oxide, Liquitint™ Blue, Dimethicone / polydimethyl silicone.

**Tide TOTALCARE HE Liquid, renewing Rain:**Water, alcoholethoxy sulfate, linear alkylbenzene sulfonate, alcohol ethoxylate, citric acid, Ethanolamine, sodium fatty acids, diethylene glycol, propylene glycol, sodium hydroxide, borax, polyethyleneimine ethoxylate, silicone polyether, ethanol, protease, sodium cumene sulfonate, diquaternium ethoxysulfate, Laureth-9, fragrance, amylase, DTPA, disodium diaminostilbene disulfonate, disodium distyrylbiphenyl disulfonate, sodium formate, calcium formate, mannanase, Liquitint™ Orange, dimethicone, polyacrylamide quaternium chloride, cellulase, dipropylethyl tetraamine.

**Tide liquid HE Free:** Water, alcoholethoxy sulfate, diethylene glycol, monoethanolamine citrate, sodium formate, propylene glycol, linear alkylbenzene sulfonates, ethanolamine, ethanol, polyethyleneimine ethoxylate, amylase, benzisothiazolin, borax, calcium formate, citric acid, diethylenetriamine pentaacetate sodium, dimethicone, diquaternium ethoxysulfate, disodium diaminostilbene disulfonate, Laureth-9, mannanase, protease, sodium cumene sulfonate, sodium fatty acids.

**Tide Coldwater HE Liquid, Fresh Scent:** Water, alcoholethoxy sulfate, MEA Citrate, alcohol sulfate, Alcohol ethoxylate, Linear alkylbenzene sulfonate MEA, sodium fatty acids, polyethyleneimine ethoxylate, diethylene glycol, propylene glycol, diquaternium ethoxysulfate, borax, polyethyleneimine ethoxylate propoxylate, ethanol, sodium cumene sulfonate, fragrance, DTPA, disodium diaminostilbene disulfonate, protease, mannanase, cellulase, amylase, sodium formate, calcium formate, lauramine oxide, Liquitint™ Blue, dimethicone.

**Tide for Coldwater HE Free Liquid:** Water, sodium alcoholethoxy sulfate, MEA Citrate, Linear alkylbenzene sulfonate: sodium salt, Alcohol ethoxylate, Linear alkylbenzene sulfonate: MEA salt, sodium fatty acids, polyethyleneimine ethoxylate, diethylene glycol, propylene glycol, diquaternium ethoxysulfate, Borax, protease, polyethyleneimine ethoxylate propoxylate, ethanol, sodium cumene sulfonate, Amylase, citric acid, DTPA, disodium diaminostilbene disulfonate, sodium formate, calcium formate, dimethicone.

**Tide Simply Clean & Fresh:** Water, alcohol ethoxylate sulfate, linear alkylbenzene sulfonate Sodium/Mea salts, propylene glycol, diethylene glycol, sodium formate, ethanol, borax, sodium fatty acids, fragrance, lauramine oxide, DTPA, Polyethylene amine ethoxylate, calcium formate, disodium diaminostilbene disulfonate, dimethicone, tetramine, Liquitint™ Blue.

**Tide Pods, Ocean Mist, Mystic Forest, Spring Meadow:** Linear alkylbenzene sulfonates, C12-16 Pareth-9, propylene glycol, alcoholethoxy sulfate, water, polyethyleneimine ethoxylate, glycerine, fatty acid salts, PEG-136 polyvinyl acetate, ethylene Diamine disuccinic salt, monoethanolamine citrate, sodium bisulfite, diethylenetriamine pentaacetate sodium, disodium distyrylbiphenyl disulfonate, calcium formate, mannanase, exyloglucanase, sodium formate, hydrogenated castor oil, natalase, dyes, termamyl, subtilisin, benzisothiazolin, perfume.

**Tide to Go:** Deionized water, Dipropylene Glycol Butyl Ether, Sodium Alkyl Sulfate, Hydrogen Peroxide, Ethanol, Magnesium Sulfate, Alkyl Dimethyl Amine Oxide, Citric Acid, Sodium Hydroxide, Trimethoxy Benzoic Acid, Fragrance.

**Tide Stain Release Liquid:** Water, Alkyl Ethoxylate, Linear Alkylbenzenesulfonate, Hydrogen Peroxide, Diquaternium Ethoxysulfate, Ethanolamine, Disodium Distyrylbiphenyl Disulfonate, tetrabutyl Ethylidinebisphenol, F&DC Yellow 3, Fragrance.

**Tide Stain Release Powder:** Sodium percarbonate, sodium sulfate, sodium carbonate, sodium aluminosilicate, nonanoyloxy benzene sulfonate, sodium polyacrylate, water, sodium alkylbenzenesulfonate, DTPA, polyethylene glycol, sodium palmitate, amylase, protease, modified starch, FD&C Blue 1, fragrance.

**Tide Stain Release, Pre Treater Spray:**

Water, Alkyl Ethoxylate, MEA Borate, Linear Alkylbenzenesulfonate, Propylene Glycol, Diquaternium Ethoxysulfate, Calcium Chlorideenzyme, Protease, Ethanolamine, Benzoisothiazolinone, Amylase, Sodium Citrate, Sodium Hydroxide, Fragrance.

**Tide to Go Stain Eraser:** Water, Alkyl Amine Oxide, Dipropylene Glycol Phenyl Ether, Hydrogen Peroxide, Citric Acid, Ethylene Diamine Disuccinic Acid Sodium salt, Sodium Alkyl Sulfate, Fragrance.

**Tide boost with Oxi:**

Sodium bicarbonate, sodium carbonate, sodium percarbonate, alcohol ethoxylate, sodium chloride, maleic/acrylic copolymer, nonanoyloxy benzene sulfonate, sodium sulfate, colorant, diethylenetriamine pentaacetate sodium salt, hydrated aluminosilicate (zeolite), polyethylene glycol, sodium alkylbenzene sulfonate, sodium palmitate, starch, water, fragrance.

**Tide Stain Release boost Duo Pac:**

Polyvinyl Alcoholpouch film, wherein there is packed a liquid part and a powder part:

**Liquid Ingredients:** Dipropylene Glycol, diquaternium Ethoxysulfate, Water, Glycerin, LiquitintTM Orange, **Powder Ingredients:** sodium percarbonate, nonanoyloxy benzene sulfonate, sodium carbonate, sodium sulfate, sodium aluminosilicate, sodium polyacrylate, sodium alkylbenzenesulfonate, maleic/acrylic copolymer, water, amylase, polyethylene glycol, sodium palmitate, modified starch, protease, glycerine, DTPA, fragrance.

**Tide Ultra Stain Release:** Water, sodium alcoholethoxy sulfate, linear alkyl benzene sulfonate, sodium/MEA salts, MEA citrate, propylene glycol, polyethyleneimine ethoxylate, ethanol, diethylene glycol, polyethyleneimine propoxyethoxylate, sodium fatty acids, protease, borax, sodium cumene sulfonate, DTPA, fragrance, amylase, disodium diaminostilbene disulfonate, calcium formate, sodium formate, gluconase, dimethicone, Liquitint™ Blue, mannanase.

**Ultra Tide with a Touch of Downy® Powdered Detergent, April Fresh/Clean Breeze/April Essence:** Sodium Carbonate, Sodium Aluminosilicate, Sodium Sulfate, Linear Alkylbenzene Sulfonate, Bentonite, Water, Sodium Percarbonate, Sodium Polyacrylate, Silicate, Alkyl Sulfate, Nonanoyloxybenzenesulfonate, DTPA, Polyethylene Glycol 4000,

Silicone, Ethoxylate, fragrance, Polyethylene Oxide, Palmitic Acid, Disodium Diaminostilbene Disulfonate, Protease, Liquitint™ Red, FD&C Blue 1, Cellulase.

**Ultra Tide with a Touch of Downy Clean Breeze:** Water, sodium alcoholethoxy sulfate, MEA citrate, linear alkyl benzene sulfonate: sodium/MEA salts, propylene glycol, polyethyleneimine ethoxylate, ethanol, diethylene glycol, polyethyleneimine, propoxyethoxylate, diquaternium ethoxysulfate, alcohol sulfate, dimethicone, fragrance, borax, sodium fatty acids, DTPA, protease, sodium bisulfite, disodium diaminostilbene disulfonate, amylase, gluconase, castor oil, calcium formate, MEA, styrene acrylate copolymer, sodium formate, Liquitint™ Blue.

**Ultra Tide with Downy Sun Blossom:** Water, sodium alcoholethoxy sulfate, MEA citrate, linear alkyl benzene sulfonate: sodium/MEA salts, propylene glycol, ethanol, diethylene glycol, polyethyleneimine propoxyethoxylate, polyethyleneimine ethoxylate, alcohol sulfate, dimethicone, fragrance, borax, sodium fatty acids, DTPA, protease, sodium bisulfite, disodium diaminostilbene disulfonate, amylase, castor oil, calcium formate, MEA, styrene acrylate copolymer, propanaminium propanamide, gluconase, sodium formate, Liquitint™ Blue.

**Ultra Tide with Downy April Fresh/ Sweet Dreams:** Water, sodium alcoholethoxy sulfate, MEA citrate, linear alkyl benzene sulfonate: sodium/MEA salts, propylene glycol, polyethyleneimine ethoxylate, ethanol, diethylene glycol, polyethyleneimin propoxyethoxylate, diquaternium ethoxysulfate, alcohol sulfate, dimethicone, fragrance, borax, sodium fatty acids, DTPA, protease, sodium bisulfite, disodium diaminostilbene disulfonate, amylase, gluconase, castor oil, calcium formate, MEA, styrene acrylate copolymer, propanaminium propanamide, sodium formate, Liquitint™ Blue.

**Ultra Tide Free Powdered Detergent:** Sodium Carbonate, Sodium Aluminosilicate, Alkyl Sulfate, Sodium Sulfate, Linear Alkylbenzene Sulfonate, Water, Sodium polyacrylate, Silicate, Ethoxylate, Sodium percarbonate, Polyethylene Glycol 4000, Protease, Disodium Diaminostilbene Disulfonate, Silicone, Cellulase.

**Ultra Tide Powdered Detergent, Clean Breeze/Spring Lavender/mountain Spring:** Sodium Carbonate, Sodium Aluminosilicate, Sodium Sulfate, Linear Alkylbenzene Sulfonate, Alkyl Sulfate, Sodium Percarbonate, Water, Sodium Polyacrylate, Silicate, Nonanoyloxybenzenesulfonate, Ethoxylate, Polyethylene Glycol 4000, Fragrance, DTPA, Disodium Diaminostilbene Disulfonate, Palmitic Acid, Protease, Silicone, Cellulase.

**Ultra Tide HE (high Efficiency) Pwdered Detergent, Clean Breeze:** Sodium Carbonate, Sodium Aluminosilicate, Sodium Sulfate, Linear Alkylbenzene Sulfonate, Water, Nonanoyloxybenzenesulfonate, Alkyl Sulfate, Sodium Polyacrylate, Silicate, Sodium Percarbonate, Ethoxylate, Polyethylene Glycol 4000, Fragrance, DTPA, Palmitic Acid, Disodium Diaminostilbene Disulfonate, Protease, Silicone, Cellulase.

**Ultra Tide Coldwater Powdered Detergent, Fresh Scent:** Sodium Carbonate, Sodium Aluminosilicate, Sodium Sulfate, Sodium Percarbonate, Alkyl Sulfate, Linear Alkylbenzene Sulfonate, Water, Nonanoyloxybenzenesulfonate, Sodium Polyacrylate, Silicate, Ethoxylate, Polyethylene Glycol 4000, DTPA, Fragrance, Natalase, Palmitic Acid, Protease, Disodium, Diaminostilbene Disulfonate, FD&C Blue 1, Silicone, Cellulase, Alkyl Ether Sulfate.

**Ultra Tide with bleach Powdered Detergent, Clean Breeze:** Sodium Carbonate, Sodium Aluminosilicate, Sodium Sulfate, Linear Alkylbenzene Sulfonate, Sodium Percarbonate, Nonanoyloxybenzenesulfonate, Alkyl Sulfate, Water, Silicate, Sodium Polyacrylate, Ethoxylate, Polyethylene Glycol 4000, Fragrance, DTPA, Palmitic Acid, Protease, Disodium Diaminostilbene Disulfonate, Silicone, FD&C Blue 1, Cellulase, Alkyl Ether Sulfate.

**Ultra Tide with Febreeze Freshness™ Powdered Detergent, Spring Renewal:**

Sodium Carbonate, Sodium Aluminosilicate, Sodium Sulfate, Linear Alkylbenzene Sulfonate, Sodium Percarbonate , Alkyl Sulfate, Water, Sodium Polyacrylate, Silicate, Nonanoyloxybenzenesulfonate, Ethoxylate, Polyethylene Glycol 4000, DTPA, Fragrance, Cellulase, Protease, Disodium Diaminostilbene Disulfonate, Silicone, FD&C Blue 1.

**Liquid Tide Plus with Febreeze Freshness - Sport HE Active Fresh:**

Water, Sodium alcoholethoxy sulfate, MEA citrate, linear alkylbenzene sulfonate, sodium salt, linear alkylbenzene sulfonate: MEA salt, alcohol ethoxylate, sodium fatty acids, propylene glycol, diethylene glycol, polyethyleneimine ethoxylate propoxylate, diquaternium ethoxysulfate,
Ethanol, sodium cumene sulfonate, borax, fragrance, DTPA, Sodium bisulfate, disodium diaminostilbene disulfonate, Mannanase, cellulase, amylase, sodium formate, calcium formate, Lauramine oxide, Liquitint™ Blue, Dimethicone / polydimethyl silicone.

**Tide Plus Febreeze Freshness Spring & Renewal:**

Water, sodium alcoholethoxy sulfate, linear alkyl benzene sulfonate: sodium/MEA salts, MEA citrate, propylene glycol, polyethyleneimine ethoxylate, fragrance, ethanol, diethylene glycol, polyethyleneimine propoxyethoxylate, protease, alcohol sulfate, borax, sodium fatty acids, DTPA, disodium diaminostilbene disulfonate, MEA, mannanase, gluconase, sodium formate, dimethicone, Liquitint™ Blue, tetramine.

**Liquid Tide Plus with Febreeze Freshness, Sport HE Victory Fresh:** Water, Sodium alcoholethoxy sulfate, MEA citrate, linear alkylbenzene sulfonate, sodium salt, linear alkylbenzene sulfonate: MEA salt, alcohol ethoxylate, sodium fatty acids, propylene glycol, diethylene glycol, polyethyleneimine ethoxylate propoxylate, diquaternium ethoxysulfate, ethanol, sodium cumene sulfonate, borax, fragrance, DTPA, Sodium bisulfate, disodium diaminostilbene disulfonate, Mannanase, cellulase, amylase, sodium formate, calcium formate, Lauramine oxide, Liquitint™ Blue, Dimethicone / polydimethyl silicone.

**Tide Vivid White + Bright Powder, Original:**

Sodium Carbonate, Sodium Aluminosilicate, Sodium Sulfate, Linear Alkylbenzene Sulfonate, Sodium Percarbonate, Nonanoyloxybenzenesulfonate, Alkyl Sulfate, Water, Silicate, Sodium Polyacrylate Ethoxylate, Polyethylene Glycol 4000, Fragrance, DTPA, Palmitic Acid, Protease, Disodium Diaminostilbene Disulfonate, Silicone, FD&C Blue 1, Cellulase, Alkyl Ether Sulfate.

**HEY SPORT TEX WASH Detergent**

Aqua, dodecylbenzenesulfonsäure, laureth-11, peg-75 lanolin, propylene glycol, alcohol denat., potassium soyate, potassium hydroxide, disodium cocoamphodiacetate, ethylendiamine triacetate cocosalkyl acetamide, parfum, zinc ricinoleate, sodium chloride, benzisothiazolinone, methylisothiazolinone, ci 16255, benzyl alcohol.

The products named Tide, Ariel, Gain and Fairy are commercially available products supplied by Procter & Gamble. The products named Persil are commercially available products supplied by Unilever and Henkel. The products named Hey Sport are commercially available products supplied by Hey Sport.

| Ingredient | Amount (in wt %) |
|---|---|
| Anionic detersive surfactant (such as alkyl benzene sulphonate, alkyl ethoxylated sulphate and mixtures | from 8 wt % to 15 wt % thereof) |
| Non-ionic detersive surfactant (such as alkyl ethoxylated alcohol) | from 0.5 wt % to 4 wt % |
| Cationic detersive surfactant (such as quaternary ammonium compounds) | from 0 to 4 wt % |
| Other detersive surfactant (such as zwiterionic detersive surfactants, amphoteric surfactants and mixtures thereof) | from 0 wt % to 4 wt % |
| Carboxylate polymer (such as co-polymers of maleic acid and acrylic acid) | from 1 wt % to 4 wt % |
| Polyethylene glycol polymer (such as a polyethylene glycol polymer comprising poly vinyl acetate side chains) | from 0.5 wt % to 4 wt % |
| Polyester soil release polymer (such as Repel-o-tex from and/or Texcare polymers) | 0.1 to 2 wt % |
| Cellulosic polymer (such as carboxymethyl cellulose, methyl cellulose and combinations thereof) | from 0.5 wt % to 2 wt % |
| Other polymer (such as amine polymers, dye transfer inhibitor polymers, hexamethylenediamine derivative polymers, and mixtures thereof) | from 0 wt % to 4 wt % |
| Zeolite builder and phosphate builder (such as zeolite 4A and/or sodium tripolyphosphate) | from 0 wt % to 4 wt% |
| Other builder (such as sodium citrate and/or citric acid) | from 0 wt % to 3 wt % |
| Carbonate salt (such as sodium carbonate and/or sodium bicarbonate) | from 15 wt % to 30 wt % |
| Silicate salt (such as sodium silicate) | from 0 wt % to 10 wt % |
| Filler (such as sodium sulphate and/or bio-fillers) | from 10 wt % to 40 wt % |
| Source of available oxygen (such as sodium percarbonate) | from 10 wt % to 20 wt % |
| Bleach activator (such as tetraacetylethylene diamine (TAED) and/or nonanoyloxybenzenesulphonate (NOBS) | from 2 wt % to 8 wt % |
| Bleach catalyst (such as oxaziridinium-based bleach catalyst and/or transition metal bleach catalyst) | from 0 wt % to 0.1 wt % |

(continued)

| Ingredient | Amount (in wt %) |
| --- | --- |
| Other bleach (such as reducing bleach and/or pre- formed peracid) | from 0 wt % to 10 wt % |
| Chelant (such as ethylenediamine-N'N'-disuccinic acid (EDDS) and/or hydroxyethane diphosphonic acid(HEDP) | from 0.2 wt % to1 wt % |
| Photobleach (such as zinc and/or aluminium sulphonated phthalocyanine) | from 0 wt % to 0.1 wt % |
| Hueing agent (such as direct violet 99, acid red 52, acid blue 80, direct violet 9, solvent violet 13 and any combination thereof) | from 0 wt % to 1 wt % |
| Brightener (such as brightener 15 and/or brightener 49) | from 0.1 wt % to 0.4 wt % |
| Protease (such as Savinase, Savinase Ultra, Purafect, FN3, FN4 and any combination thereof) | from 0.1 wt % to 0.4 wt % |
| Amylase (such as Termamyl, Termamyl ultra Natalase, Optisize, Stainzyme, Stainzyme Plus, and any combination thereof) | from 0.05 wt % to 0.2 wt % |
| Cellulase (such as Carezyme and/or Celluclean) | from 0.05 wt % to 0.2 wt % |
| Lipase (such as Lipex, Lipolex, Lipoclean and any combination thereof) | from 0.2 to 1 wt % |
| Other enzyme (such as xyloglucanase, cutinase, pectate lyase, mannanase, bleaching enzyme) | from 0 wt % to 2 wt % |
| Fabric softener (such as montmorillonite clay and/or polydimethylsiloxane (PDMS) | from 0 wt % to 4 wt % |
| Flocculant (such as polyethylene oxide) | from 0 wt % to 1 wt % |
| Suds suppressor (such as silicone and/or fatty acid) | from 0 wt % to 0.1 wt % |
| Perfume (such as perfume microcapsule, spray-on perfume, starch encapsulated perfume accords, perfume loaded zeolite, and any combination thereof) | from 0.1 wt % to 1 wt % |
| Aesthetics (such as coloured soap rings and/or coloured speckles/noodles) | from 0 wt % to 1 wt % |
| Miscellaneous | balance |

| Ingredient | Amount |
| --- | --- |
| Carboxyl group-containing polymer (comprising from about 60% to about 70% by mass of an acrylic acid-based monomer (A); and from about 30% to about 40%) by mass of a sulfonic acid group-containing monomer (B); and wherein the average molecular weight is from about 23,000 to about 50,000 preferably in the range of from about 25,000 to about 38,000 as described in WO2014032269. | from about 0.5 wt% to about 1.5 wt% |
| Amylase (Stainzyme Plus(R), having an enzyme activity of 14 mg active enzyme/ g) | from about 0.1wt% to about 0.5 wt% |
| Anionic detersive surfactant (such as alkyl benzene sulphonate, alkyl ethoxylated sulphate and mixtures thereof) | from about 8 wt% to about 15 wt% |
| Non-ionic detersive surfactant (such as alkyl ethoxylated alcohol) | from about 0.5 wt% to 4 wt% |
| Cationic detersive surfactant (such as quaternary ammonium compounds) | from about 0 wt% to about 4 wt% |
| Other detersive surfactant (such as zwiterionic detersive surfactants, amphoteric surfactants and mixtures thereof) | from about 0 wt% to 4 wt% |
| Carboxylate polymer (such as co-polymers of maleic acid and acrylic acid) | from about 1 wt% to about 4 wt% |
| Polyethylene glycol polymer (such as a polyethylene glycol polymer comprising poly vinyl acetate side chains) | from about 0 wt% to about 4 wt% |

(continued)

| Ingredient | Amount |
|---|---|
| Polyester soil release polymer (such as Repel-O- Tex(R) and/or Texcare(R) polymers) | from about 0.1 wt% to about 2 wt% |
| Cellulosic polymer (such as carboxymethyl cellulose, methyl cellulose and combinations thereof) | from about 0.5wt% to about 2 wt% |
| Other polymer (such as amine polymers, dye transfer inhibitor polymers, hexamethylenediamine derivative polymers, and mixtures thereof) | from about 0 wt% to about 4 wt% |
| Zeolite builder and phosphate builder (such as zeolite 4A and/or sodium tripolyphosphate) | from about 0 wt% to about 4 wt% |
| Other builder (such as sodium citrate and/or citric acid) | from about 0 wt% to about 3 wt% |
| Carbonate salt (such as sodium carbonate and/or sodium bicarbonate) | from about 15 t% to about 30 wt% |
| Silicate salt (such as sodium silicate) | from about 0 wt% to about 10 wt% |
| Filler (such as sodium sulphate and/or bio-fillers) | from about 10 wt% to about 40 wt% |
| Source of available oxygen (such as sodium percarbonate) | from about 10wt% to about 20 wt% |
| Bleach activator (such as tetraacetylethylene diamine (TAED) and/or nonanoyloxybenzenesulphonate (NOBS) | from about 2 wt% to about 8 wt% |
| Bleach catalyst (such as oxaziridinium-based bleach catalyst and/or transition metal bleach catalyst) | from about 0 wt% to about 0.1 wt% |
| Other bleach (such as reducing bleach and/or pre formed peracid) | from about 0 wt% to about 10 wt% |
| Chelant (such as ethylenediamine-N'N'-disuccinic acid (EDDS) and/or hydroxyethane diphosphonic acid (HEDP) | from about 0.2wt% to about 1wt% |
| Photobleach (such as zinc and/or aluminium sulphonated phthalocyanine) | from about 0 wt% to about 0.1 wt% |
| Hueing agent (such as direct violet 99, acid red 52, acid blue 80, direct violet 9, solvent violet 13 and any combination thereof) | from about 0 wt% to about 0.5 wt% |
| Brightener (such as brightener 15 and/or brightener 49) | from about 0.1wt% to about 0.4 wt% |
| Protease (such as Savinase, Polarzyme, Purafect, FN3, FN4 and any combination thereof, typically having an enzyme activity of from about 20 mg to about 100mg active enzyme/g) | from about 0.1wt% to about 1.5 wt% |
| Amylase (such as Termamyl(R), Termamyl Ultra(R), Natalase(R), Optisize HT Plus(R), Powerase(R), Stainzyme(R) and any combination thereof, typically having an enzyme activity of from about 10 mg to about 50 mg active enzyme/ g) | from about 0.05 wt% to about 0.2 wt% |
| Cellulase (such as Carezyme(R), Celluzyme(R) and/or Celluclean(R), typically having an enzyme activity of about from 10 to 50mg active enzyme/ g) | from about 0.05 wt% to 0.5 wt% |
| Lipase (such as Lipex(R), Lipolex(R), Lipoclean(R) and any combination thereof, typically having an enzyme activity of from about 10 mg to about 50 mg active enzyme/ g) | from about 0.2 wt% to about 1 wt% |
| Other enzyme (such as xyloglucanase (e.g., Whitezyme(R)), cutinase, pectate lyase, mannanase, bleaching enzyme, typically having an enzyme activity of from about 10 mg to about 50 mg active enzyme/g) | from 0 wt% to 2 wt% |
| Fabric softener (such as montmorillonite clay and/or polydimethylsiloxane (PDMS)) | from 0 wt% to 15 wt% |

(continued)

| Ingredient | Amount |
|---|---|
| Flocculant (such as polyethylene oxide) | from 0 wt% to 1 wt% |
| Suds suppressor (such as silicone and/or fatty acid) | from 0 wt% to 0.1wt% |
| Perfume (such as perfume microcapsule, spray-on perfume, starch encapsulated perfume accords, perfume loaded zeolite, and any combination thereof) | from 0.1 wt% to 1 wt% |
| Aesthetics (such as colored soap rings and/or colored speckles/noodles) | from 0 wt% to 1wt% |
| Miscellaneous | Balance |

All enzyme levels expressed as rug active enzyme protein per 100 g detergent composition. Surfactant ingredients can be obtained from BASF, Ludwigshafen, Germany (Lutensol(R)); Shell Chemicals, London, UK; Stepan, Northfield, III, USA; Huntsman, Huntsman, Salt Lake City, Utah, USA; Clariant, Sulzbach, Germany (Praepagen(R)).

Sodium tripolyphosphate can be obtained from Rhodia, Paris, France. Zeolite can be obtained from Industrial Zeolite (UK) Ltd, Grays, Essex, UK. Citric acid and sodium citrate can be obtained from Jungbunzlauer, Basel, Switzerland. NOBS is sodium nonanoyloxybenzenesulfonate, supplied by Eastman, Batesville, Ark., USA.

TAED is tetraacetylethylenediamine, supplied under the Peractive(R) brand name by Clariant GmbH, Sulzbach, Germany.

Sodium carbonate and sodium bicarbonate can be obtained from Solvay, Brussels, Belgium.

Polyacrylate, polyacrylate/maleate copolymers can be obtained from BASF, Ludwigshafen, Germany.

Repel-O-Tex(R) can be obtained from Rhodia, Paris, France.

Texcare(R) can be obtained from Clariant, Sulzbach, Germany. Sodium percarbonate and sodium carbonate can be obtained from Solvay, Houston, Tex., USA.

Na salt of Ethylenediamine-N,N'-disuccinic acid, (S,S) isomer (EDDS) was supplied by Octel, Ellesmere Port, UK.

Hydroxy ethane di phosphonate (HEDP) was supplied by Dow Chemical, Midland, Mich., USA.

Enzymes Savinase(R), Savinase(R) Ultra, Stainzyme(R) Plus, Lipex(R), Lipolex(R), Lipoclean(R), Celluclean(R), Carezyme(R), Natalase(R), Stainzyme(R), Stainzyme(R) Plus, Termamyl(R), Termamyl(R) ultra, and Mannaway(R) can be obtained from Novozymes, Bagsvaerd, Denmark.

Enzymes Purafect(R), FN3, FN4 and Optisize can be obtained from Genencor International Inc., Palo Alto, California, US.

Direct violet 9 and 99 can be obtained from BASF DE, Ludwigshafen, Germany. Solvent violet 13 can be obtained from Ningbo Lixing Chemical Co., Ltd. Ningbo, Zhejiang, China. Brighteners can be obtained from Ciba Specialty Chemicals, Basel, Switzerland. All percentages and ratios are calculated by weight unless otherwise indicated. All percentages and ratios are calculated based on the total composition unless otherwise indicated. It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

## Wash assays

### Launder-O-Meter (LOM) Model Wash System

The Launder-O-Meter (LOM) is a medium scale model wash system that can be applied to test up to 20 different wash conditions simultaneously. A LOM is basically a large temperature controlled water bath with 20 closed metal beakers rotating inside it. Each beaker constitutes one small washing machine and during an experiment, each will contain a solution of a specific detergent/enzyme system to be tested along with the soiled and unsoiled fabrics it is tested on. Mechanical stress is achieved by the beakers being rotated in the water bath and by including metal balls in the beaker.

The LOM model wash system is mainly used in medium scale testing of detergents and enzymes at European wash conditions. In a LOM experiment, factors such as the ballast to soil ratio and the fabric to wash liquor ratio can be varied. Therefore, the LOM provides the link between small scale experiments, such as AMSA and mini-wash, and the more time consuming full scale experiments in front loader washing machines.

### Mini Launder-O-Meter (MiniLOM) Model Wash System

MiniLOM is a modified mini wash system of the Launder-O-Meter (LOM), which is a medium scale model wash system that can be applied to test up to 20 different wash conditions simultaneously. A LOM or is basically a large temperature controlled water bath with 20 closed metal beakers rotating inside it. Each beaker constitutes one small washing machine and during an experiment, each will contain a solution of a specific detergent/enzyme system to be tested along with the soiled and unsoiled fabrics it is tested on. Mechanical stress is achieved by the beakers being rotated in the water bath and by including metal balls in the beaker.

The LOM model wash system is mainly used in medium scale testing of detergents and enzymes at European wash conditions. In a LOM experiment, factors such as the ballast to soil ratio and the fabric to wash liquor ratio can be varied. Therefore, the LOM provides the link between small scale experiments, such as AMSA and mini-wash, and the more time consuming full scale experiments in front loader washing machines.

In miniLOM, washes are performed in 50 ml test tubes placed in Stuart rotator.

### Terg-O-timeter (TOM) wash assay

The Tergo-To-Meter (TOM) is a medium scale model wash system that can be applied to test 12 different wash conditions simultaneously. A TOM is basically a large temperature controlled water bath with up to 12 open metal beakers submerged into it. Each beaker constitutes one small top loader style washing machine and during an experiment, each of them will contain a solution of a specific detergent/enzyme system and the soiled and unsoiled fabrics its performance is tested on. Mechanical stress is achieved by a rotating stirring arm, which stirs the liquid within each beaker. Because the TOM beakers have no lid, it is possible to withdraw samples during a TOM experiment and assay for information on-line during wash.

The TOM model wash system is mainly used in medium scale testing of detergents and enzymes at US or LA/AP wash conditions. In a TOM experiment, factors such as the ballast to soil ratio and the fabric to wash liquor ratio can be varied. Therefore, the TOM provides the link between small scale experiments, such as AMSA and mini-wash, and the more time consuming full scale experiments in top loader washing machines.

Equipment: The water bath with 12 steel beakers and 1 rotating arm per beaker with capacity of 500 or 1200mL of detergent solution. Temperature ranges from 5 to 80°C. The water bath has to be filled up with deionised water. Rotational speed can be set up to 70 to 120rpm/min.

Set temperature in the Terg-O-Tometer and start the rotation in the water bath. Wait for the temperature to adjust (tolerance is +/- 0,5°C). All beakers shall be clean and without traces of prior test material.

The wash solution with desired amount of detergent, temperature and water hardness is prepared in a bucket. The detergent is allowed to dissolve during magnet stirring for 10 min. Wash solution shall be used within 30 to 60 min after preparation.

800ml wash solution is added into a TOM beaker. The wash solution is agitated at 120rpm and optionally one or more enzymes are added to the beaker. The swatches are sprinkled into the beaker and then the ballast load. Time measurement starts when the swatches and ballast are added to the beaker. The swatches are washed for 20 minutes after which agitation is terminated. The wash load is subsequently transferred from the TOM beaker to a sieve and rinse with cold tap water. The soid swatches are separated from the ballast load. The soil swatches are transferred to a 5L beaker with cold tap water under running water for 5 minuttes. The ballast load is kept separately for the coming inactivation. The water is gently pressed out of the swatches by hand and placed on a tray covered with a paper. Another paper is palced on top of the swatches. The swatches are allowed to dry overnight before subjecting the swatches to analysis, such as assaying for odor removal as described in Example 7.

### Full scale wash

This is the test method used to test the wash performance of DNAse in full scale wash under EU conditions (washing in a front loader washing machine)..

The real items (T-shirts) and ballast are added to each wash together with detergent and enzyme. After wash, the real items (T-shirts) are dried. After drying, round swatches are cut out and washed with detergent added soil (dirty detergent) in miniLOM. Color difference is measured on a MacBeth Color Eye spectrophotometer.

The enzymes are added on basis of weight percent of the detergent dosage in each wash,

<u>Equipment used:</u>

- Washing machine: Miele Softtronic W2445
- Water meters and automatically data collection system
- MacBeth Color Eye spectrophotometer

For the preparation and adjustment of water hardness the following ingredients are needed:

- Calcium chloride ($CaCl_2$• $2H_2O$)
- Magnesium chloride ($MgCL_2$• $6H_2O$)
- Sodium Hydrogen Carbonate ($NaHCO_3$)

Ballast

The ballast consists of clean white cloth without optical whitener made of cotton, polyester or cotton/polyester. The composition of the ballast is a mix of different items at a cotton/polyester ratio of 65/35 based on weight. The ballast weight, dryness and item composition must be the same in each wash.

After each wash the ballast is inactivated in an industrial washer at 85°C/15 min or in a 95°C wash (EU machine) without detergent

Ballast Example: (Standard EU ballast composition, total 3kg)

- 3 T-shirts (100% cotton)
- 10 shirts, short sleeves (55% cotton 45% polyester)
- 4 pillow cases (35% cotton, 65%polyester), 110x75cm
- 1 small bed sheets, size 100x75cm (100% cotton)
- 3 Tea towels (100% cotton)
- Socks (80% cotton 20% polyester) as balance

Wash conditions

- Temperature: 30°C.
- Washing programme: Normal cotton wash without pre-wash: "Cottons".
- Water level 13-14L with "water plus"
- Water hardness: Standard EU conditions: 15°dH, Ca2+:Mg2+:HCO3 = 4:1:7.5
- DNAse dosage: 1 ppm.

Detailed steps to carry out full scale wash trial

1. Select wash program as in study plan.
2. The detergent and DNAse are placed in the wash drum in a "washing ball" (both liquid and powder detergents). Place it at the bottom.
3. Place the real items (T-shirts) and ballast in the wash drum.
4. Start digital water meter
5. Start the washer by pressing the knob START
6. After wash, take out real items (T-shirts) and ballast, put real items into drying room.

Drying procedure

Put stains on tray or hang in line and dry at room temperature. The room has a de-humidifier working for 24h per day to keep the room dry

Measurement

Round swatches from T-shirts (armpits, front, back and edge) are cut out and washed in miniLOM with dirty detergent added soil. Swatches are evaluated by measurement of Color difference (L values) was measured using a Color Eye (Macbeth Color Eye 7000 reflectance spectrophotometer). The measurements were made without UV in the incident light, and the L value from the CIE Lab color space was extracted.

**Enzyme assays**

**Assay I**

Testing of DNase activity

DNase activity was determined on DNase Test Agar with Methyl Green (BD, Franklin Lakes, NJ, USA), which was prepared according to the manual from supplier. Briefly, 21 g of agar was dissolved in 500 ml water and then autoclaved for 15 min at 121°C. Autoclaved agar was temperated to 48°C in water bath, and 20 ml of agar was poured into petridishes with and allowed to solidify by incubation o/n at room temperature. On solidified agar plates, 5 μl of enzyme solutions are added, and DNase activity are observed as colorless zones around the spotted enzyme solutions.

**Assay II**

Analysis of E-2-nonenal on textile using an electronic nose

One way of testing for the presence of malodor on textiles is by using E-2-Nonenal as a marker for the malodor, as this compound contributes to the malodor on laundry.
Add a solution of E-2-nonenal to a 5 cm x 5 cm textile swatch and place the swatch in a 20 mL glass vial for GC analysis and cap the vial. Analyze 5 mL headspace from the capped vials in a Heracles II Electronic nose from Alpha M.O.S., France (double column gas chromatograph with 2 FIDs, column 1: MXT5 and column 2: MXT1701) after 20 minutes incubation at 40°C.

**Examples**

**Example 1**

**Detection of deep-cleaning effects on T-shirts for running**

Two white T-shirts for running made of 100% polyester were washed (full scale wash)in a washing machine using 3.33 g/L of model detergent A.
The two T-shirts were worn by a test person (male), one T-shirt at the time. The test person wore each of the the T-shirts during physical activity for one hour. After wearing, one T-shirt was washed in a washing machine using 3.33 g/L of model detergent A with the DNAse of SEQ ID NO: 7 (1 ppm), whereas the second T-shirt was washed in a washing machine using 3.33 g/L of model detergent A without the DNAse (0 ppm). The T-shirts were washed as described in the full scale wash described above and for washing, 15 L of tap water was used. Both T-shirts were worn during physical activity and then washed. This wear and wash cycle was repeated 10 times.
For evaluation of the cleaning effect (deep-cleaning effect), five circular swatches (diameter of 2 cm) were cut out from armpit, back (upper back, between shoulders), front (breast) and lower front edge of the T-shirts. Five swatches from armpit, back, front and edge, respectively were mixed with five sterile Polyester WFK30A swatches in a 50 mL test tube and added 10 mL of wash liquor prepared by adding 3.33 g/l in water of a model detergent A added 0.7 g/L soil (Pigmentschmutz, 09V, wfk, Krefeld, Germany). Test tubes were placed in a Stuart rotator (miniLOM) for 1 hour at 30°C and washed in accordance with the miniLOM described above. Swatches were rinsed twice with 10 ml of tap water and dried on filter paper over night.
Color difference (L value) was measured using a Color Eye (Macbeth Color Eye 7000 reflectance spectrophotometer). The measurements were made without UV in the incident light, and the L value from the CIE Lab color space was extracted. A high L value reflects a white textile. Thus, the higher ΔL, the whiter textile.
The result below shows that washing with DNase during 10 washes prevents deposition of soil in a subsequent laundering process. In addition the results show that washing with DNase during 10 washes improves the whiteness of the textile.

| | Color difference | | |
|---|---|---|---|
| | L with DNAse | L without DNAse | ΔL |
| Left armpit | 91.6 | 83.5 | 8.1 |
| Right armpit | 89.0 | 82.1 | 6.9 |
| Front | 92.6 | 86.7 | 5.9 |
| Back | 92.9 | 86.2 | 6.7 |
| Edge | 91.7 | 90.3 | 1.4 |

**Example 2**

**Deep-cleaning performance of DNAse in liquid detergent over 2 washes**

One strain of *Brevundimonas* sp. isolated from laundry was used in the present example. *Brevundimonas* sp. was pre-grown on Tryptone Soya Agar (TSA) (pH 7.3) (CM0131; Oxoid Ltd, Basingstoke, UK) for 2-5 days at 30°C. From a single colony, a loop-full was transferred to 10 mL of TSB (Tryptone Soya broth, Oxoid) and incubated for 16 hours at 30°C with shaking (240 rpm). After **propagation,** *Brevundimonas* sp. was pelleted by centrifugation (Sigma Laboratory Centrifuge 6K15) (3000 g at 21°C in 7 min) and resuspended in 10 mL of TSB diluted twice with milliQ water. Optical density (OD) at 600 nm was measured using a spectrophotometer (POLARstar Omega (BMG Labtech, Ortenberg, Germany). Fresh TSB diluted twice with sterile milliQ water was inoculated with Brevundimonas sp. to $OD_{600nm}$ 0.03, and 1.6 mL was added into each well of a 12-well polystyrene flat-bottom microplate (3512; Corning Incorporated, Coming, NY, USA), in which round swatches (diameter 2 cm) of sterile Polyester WFK30A was placed. After 24 h and 72 h incubation, respectively, at 15°C with shaking (100 rpm), growth media was removed, and swatches were rinsed twice with 0.9% (w/v) NaCl.

In wash 1, five rinsed swatches with *Brevundimonas* sp. as prepared above (donor swatches) were mixed with five sterile Polyester WFK30A swatches (tracer swatches) in a 50 mL test tube and added 10 mL of wash liquor prepared by adding 3.33 g/l in water of a model detergent A and DNAse of SEQ ID NO: 2 (0.04 ppm). Washes with model detergent A without DNAse added were made in parallel. Test tubes were placed in a Stuart rotator (miniLOM) for 1 hour at 30°C and washed in accordance with the miniLOM described above. Swatches were rinsed twice with tap water and dried on filter paper over night.

In wash 2, the five dried donor and tracer swatches from wash 1 were washed in a 50 mL test tube added 10 mL of wash liquor prepared by adding 3.33 g/l in water of a model detergent A and 0.7 g/L soil (Pigmentschmutz, 09V, wfk, Krefeld, Germany). Test tubes were placed in a Stuart rotator for 1 hour at 30°C and washed in accordance with the miniLOM described above. Swatches were rinsed twice with tap water and dried on filter paper over night.

Color difference (L values) was measured using a Color Eye (Macbeth Color Eye 7000 reflectance spectrophotometer). The measurements were made without UV in the incident light, and the L value from the CIE Lab color space was extracted. A high L value reflects a white textile. Thus, the higher ΔL, the whiter textile.

The results in the table below shows that the L-values measured after wash 2. The results show that washing with DNase in wash 1 prevents deposition of soil in a subsequent laundering process with no DNase present. In addition the results show that washing with DNase during 10 washes improves the whiteness of the textile.

| | Color difference | | | | | |
|---|---|---|---|---|---|---|
| | $L_{Donor+enz.}$ | $L_{Donor-enz.}$ | $(\Delta L_{donor})$ | $L_{Tracer+enz.}$ | $L_{Tracer-enz.}$ | $(\Delta L_{tracer})$ |
| 24 h Brevundimonas stain | 92.2 | 86.7 | 5.5 | 92.9 | 91.2 | 1.7 |
| 72 h Brevundimonas stain | 87.3 | 85.0 | 2.3 | 92.2 | 86.5 | 5.7 |

**Example 3** (not encompassed by the wording of the claims)

**Detection of sweaty odor on T-shirts for running**

Two white T-shirts for running made of 100% polyester were washed in a washing machine (full scale wash) using 3.33 g/L of model detergent A.

The two T-shirts were worn by a test person (male), one T-shirt at the time. The test person wore each of the the T-shirts during physical activity for one hour. After wearing, one T-shirt was washed in a washing machine using 3.33 g/L of model detergent A with the DNAse of SEQ ID NO: 7 (1 ppm), whereas the second T-shirt was washed in a washing machine using 3.33 g/L of model detergent A without the DNAse (0 ppm). The T-shirts were washed as described in the full scale wash described above and for washing, 15 L of tap water was used. Both T-shirts were worn during physical activity and then washed. This wear and wash cycle was repeated 10 times

For evaluation of odor, a trained test person investigated T-shirts prior to use for sweaty odor (malodor) by smelling to T-shirts. If a sweaty odor was detected, a mark (X) was added in the table below. The observations below show that washing with DNase during 10 washes prevents accumulation of odor in a subsequent laundering process where no DNase is used.

Table 1.

| Number of use | 1 | 2 | 3 | 4 | 5 | 6 | 7 | 8 | 9 | 10 |
|---|---|---|---|---|---|---|---|---|---|---|
| T-shirt washed without DNase | - | - | - | - | - | x | x | x | x | x |
| T-shirt washed with DNase | - | - | - | - | - | - | - | - | - | - |

**Example 4** (not encompassed by the wording of the claims)

A wash experiment was conducted on a collection of running clothes (sweatshirts and running T-shirts), which had been worn during training exercises for at least 50 times. During the training exercises the running clothes had become damp of sweat but generally not soiled. The running clothes had previous been washed in a Miele Softtronic W5825 at 30°C (low filling of the machine) using BioTex Black (Unilever) in a recommended standard dose.

Before conducting the present experiment, the sweatshirt and running T-shirt (100 % polyester) was evaluated by a trained odor panelist, who found a pronounced odor of sour sweat (malodor), in particular present in the armpit of the clothes. The malodor was present after washing the clothes using BioTex Black and the malodor accumulated with the number of times the running clothes were used.

The running clothes was washed in a Miele Softtronic W5825 at 30°C (low filling of the machine) using standard dose of BioTex black and a dose of A. *oryzae* DNase (0.4 ppm).

Between each training exercises, the running clothes were subjected to another round of assessment by the odor panelist. The odor panelist found that the malodor was significantly reduced and hardly detectable after 1 to 3 washing cycles in the presence of the A. *oryzae* DNAse of the present invention. In an extending study, the running clothes were washed in the presence of the A. *oryzae* DNAse of the present invention every time the clothes were washed. The odor panelist found that when the running clothes had been washed in the presence of the A. *oryzae* DNAse it could be worn for at least two training exercises (allowing the clothes to dry without washing the clothes between the training exercises) before malodor was clearly detectable.

**Example 5** (not encompassed by the wording of the claims)

Odor removal by DNase (E-nose)

Preparation of DNA swatches with E-2-nonenal

Chromosomal DNA from *Pseudomonas sp.* was isolated by QIAamp DNA Blood Mini Kit following the manufactures instructions (Qiagen, Hilden, Germany). Polyester swatches (WFK30A) (2 cm in diameter) were sterilized in a Holm & Halby Systec DB-23 autoclave for 60 minutes at 121°C. 100μl of purified *Pseudomonas sp.* DNA diluted to a final concentration of 1 ng/μl was added on each of the autoclaved swatches and swatches were dried under continuous flow in a Laminar Air Flow Bench for 2 hours. After drying, 10 swatches with DNA were placed in a sterile 25 mL NUNC tube (364238; Thermo Scientific), and 10 ml of 0.2 mM E-2-Nonenal (255653; Sigma-Aldrich) was added (Assay II). The tube was placed in a Stuart rotator (miniLOM) (20 rpm at room temperature for 20 min). The 10 swatches were transferred to a 50.000 MWCO centrifugal tube (VS203, Vivaspin 20, Satorius). The tubes were centrifuged at 3000 g at 21°C in 1 min, and the 10 swatches were split into 2 sterile NUNC tubes (364238; Thermo Scientific) with 5 swatches in each tube. Additionally, 5 sterile polyester swatches (WFK30A) (2 cm in diameter) without DNA and E-2-nonenal were added to each of the tubes. The swatches without DNA were marked allowing discrimination from swatches with DNA/ E-2-nonenal.

Washing procedure of DNA swatches with E-2-nonenal

Wash liquor of Ariel Actilift powder Style&Color and Ariel Actilift powder White were prepared by dissolving 5.0 g

of detergent in 1000 ml of sterile MilliQ water with a hardness of 15°dH (EU conditions). Wash liquor of Tide Pods was prepared by dissolving 1.8 g of the white phase detergent in 1000 ml of sterile milliQ water with a hardness of 6°dH. Wash liquors were left on a magnetic stirrer for 20 min prior to use.

Wash liquor (10 ml) was added to each of the two identical tubes with 5 DNA swatches with E-2-nonenal and 5 sterile swatches, 10 μl of *Apergillus oryzae* DNAse of the present invention resulting in a final concentration of 5 ppm was added to one of the tubes. The tubes were placed in a Stuart rotor (20 rpm at 30°C for 60 min). Wash liquor was poured off, and swatches were rinsed twice with 20 mL of sterile milliQ water with hardness 15°dH. Swatches from each tube were transferred to a 50.000 MWCO centrifugal tube (VS203, Vivaspin 20, Satorius) and centrifuged at 3000 g at 21°C in 1 min. Each swatch with E-2-nonenal was then transferred to a 20 mL GC headspace vial, using clean, sterile tweezers to place each swatch one swatch in each vial. They were then analyzed in an Alpha MOS HERACLES Flash Gas Chromatography Electronic Nose, equipped with a Restek MXT-5 capillary column, with an HS100 autosampler and an FID detector.

Table 2. Intensity of E-2-Nonenal measured with E-nose.

| Detergent | Peak area (no DNAse) | Peak area (with DNAse) | % reduction |
|---|---|---|---|
| Ariel Actilift powder White | 108325 | 83177 | 23% |
| Ariel Actilift powder Style&Color | 167288 | 122868 | 27% |
| Tide pods | 128945 | 80895 | 37% |

Conclusion: DNA-trapped odor on textile can be removed with the *Apergillus oryzae* DNAse, thus resulting in odor reduction.

**Example 6** (not encompassed by the wording of the claims)

Odor removal by DNase (Sensory analysis)

Polyester and cotton swatches (2 cm in diameter) were soiled with *Pseudomonas* sp. DNA and 0.5 mM E-2-nonenal (255653; Sigma Aldrich) (see Example 5for details on prepration of the DNA swatches) and washed in Stuart rotator (miniLOM) 60 min at 30°C in model detergent A (prepared by dissolving 3.33 g in 1 liter of milliQ water with hardness 15°dH) in the presence or absence of *Aspergillus oryzae* DNAse of the present invention (5 ppm). After washing, swatches were rinsed twice in 20 ml of MilliQ water with hardness 15°dH. The swatches were evaluated for the perceived intensity of E-2-nonenal by four odor panelist (samples where blinded). For evaluation a perceived intensity scale going from 0 to 9 was used. 0 indicated no perceived intensity of E-2-Nonenal, whereas 9 indicated the highest perceived intensity of E-2-Nonenal. Results showed consensus within all four odor panelists.

Table 3

| Type of textile | DNAse addition | Average score of four odor panelist |
|---|---|---|
| Cotton | - | 5.0 |
| Cotton | + | 1.3 |
| Polyester | - | 6.3 |
| Polyester | + | 2.8 |

DNA-trapped odor on textile can be removed with the *Apergillus oryzae* DNAse and thus result in odor reduction.

**Example 7** (not encompassed by the wording of the claims)

Assay for visual determination of odor removal

*Brevundimonas* sp. was pre-grown on Tryptone Soya Agar (TSA) (pH 7.3) (CM0131; Oxoid Ltd, Basingstoke, UK) for 2-5 days at 30°C. From a single colony, a loop-full was transferred to 10 mL of TSB and incubated for 20 hours at 30°C with shaking (240 rpm). After propagation, *Brevundimonas* sp. was pelleted by centrifugation (Sigma Laboratory Centrifuge 6K15) (3000 g at 21°C in 7 min) and resuspended in 10 mL of TSB diluted twice with water. Optical density (OD) at 600 nm was measured using a spectophometer (POLARstar Omega (BMG Labtech, Ortenberg, Germany). Fresh TSB diluted twice with water was inoculated to an OD600nm of 0.03, and 20 mL was added to a petri dish, in which 5 cm x 5 cm

swatch of sterile Polyester WFK30A was placed. After incubation (24 h at 15°C with shaking (75 rpm), swatches were rinsed twice with 0.9% (w/v) NaCl. Swatches were washed in TOM either directly after rinsing or after drying for 24 hours in a LAF bench.

The visual indicating agent Phenol red in solution was prepared by dissolving 0.1 g in 100 ml 99% Ethanol. Indicator solution (50 μL) was added to a filter paper (diameter 2 cm) and dried overnight in fume hood to form an odour capturing agent including the visual indicating agent (Phenol red). Four washed swatches were placed in the bottom of a glass container (one container for each swatch) and 500 μL of 17% TSB was added. As a control, washed clean textile polyester was placed in the bottom of another glass container and 500 μL of 17% TSB was added. The containers with the swatches were incubated at incubated at 37°C. The containers were monitored up to 48 hours to detect change of colour of the Phenol red tracer.

Table 4. Incubation results

| Detergent | No enzyme | DNase (10 ppm) |
|---|---|---|
| Tide Original | Red (24h) | Yellow (24h) |
| Ariel sensitive White&Color | Red (48h) | Yellow (48h) |
| Model T | Red (24h) | Yellow (24h) |

Hours in table indicate time points for detected colour change.

## Claims

1. Use of a polypeptide having DNase activity for preventing or reducing redeposition of soil on an item during a subsequent cleaning or laundering process, wherein the item is a textile, and wherein no polypeptide having DNase activity is present in the subsequent cleaning or laundering process.

2. Use according to claim 1, for maintaining or improving whiteness of the item.

3. Use according to claim 1 or 2, wherein the polypeptide having DNase activity is used for cleaning or laundering the item at least one time before the subsequent cleaning or laundering process in which no polypeptide having DNase activity is present.

4. Use according to claim 3, wherein the polypeptide having DNase activity is used for cleaning or laundering the item at least two times, three times, four times, five times, six times, seven times, eight times, nine times or ten times before the subsequent cleaning or laundering process in which no polypeptide having DNase activity is present.

5. Use according to any of claims 1-4, wherein the polypeptide having DNase activity has at least 80% sequence identity to the mature polypeptide of SEQ ID NO: 2 or SEQ ID NO: 3.

6. Use according to claim 5, wherein the polypeptide having DNase activity has at least 90% sequence identity to the mature polypeptide of SEQ ID NO: 2 or SEQ ID NO: 3.

7. Use according to any of claims 1-4, wherein the polypeptide having DNase activity is a variant of the mature polypeptide of SEQ ID NO: 2 or SEQ ID NO: 3 having a substitution, deletion and/or insertion at 1, 2, 3, 4, 5, 6, 7, 8, 9 or 10 positions.

## Patentansprüche

1. Verwendung eines Polypeptids mit DNase-Aktivität zum Verhindern oder Verringern einer Wiederablagerung von Schmutz auf einem Gegenstand während eines nachfolgenden Reinigungs- oder Waschverfahrens, wobei der Gegenstand ein Textil ist, und wobei kein Polypeptid mit DNase-Aktivität im nachfolgenden Reinigungs- oder Waschverfahren vorhanden ist.

2. Verwendung nach Anspruch 1, zum Erhalten oder Verbessern des Weißgrads des Gegenstands.

3. Verwendung nach Anspruch 1 oder 2, wobei das Polypeptid mit DNase-Aktivität mindestens einmal vor dem nachfolgenden Reinigungs- oder Waschverfahren, in dem kein Polypeptid mit DNase-Aktivität vorhanden ist, zum Reinigen oder Waschen des Gegenstands verwendet wird.

4. Verwendung nach Anspruch 3, wobei das Polypeptid mit DNase-Aktivität mindestens zweimal, dreimal, viermal, fünfmal, sechsmal, siebenmal, achtmal, neunmal oder zehnmal vor dem nachfolgenden Reinigungs- oder Waschverfahren, in dem kein Polypeptid mit DNase-Aktivität vorhanden ist, zum Reinigen oder Waschen des Gegenstands verwendet wird.

5. Verwendung nach einem beliebigen der Ansprüche 1-4, wobei das Polypeptid mit DNase-Aktivität mindestens 80% Sequenzidentität zum reifen Polypeptid von SEQ ID NO: 2 oder SEQ ID NO: 3 aufweist.

6. Verwendung nach Anspruch 5, wobei das Polypeptid mit DNase-Aktivität mindestens 90% Sequenzidentität zum reifen Polypeptid von SEQ ID NO: 2 oder SEQ ID NO: 3 aufweist.

7. Verwendung nach einem beliebigen der Ansprüche 1-4, wobei das Polypeptid mit DNase-Aktivität eine Variante des reifen Polypeptids von SEQ ID NO: 2 oder SEQ ID NO: 3 mit einer Substitution, Deletion und/oder Insertion an 1, 2, 3, 4, 5, 6, 7, 8, 9 oder 10 Positionen ist.

## Revendications

1. Utilisation d'un polypeptide ayant une activité de DNase pour prévenir ou réduire la redéposition de salissure sur un article pendant un processus de nettoyage ou lessivage ultérieur, dans laquelle l'article est un textile, et dans laquelle aucun polypeptide ayant une activité de DNase n'est présent lors du processus de nettoyage ou lessivage ultérieur.

2. Utilisation selon la revendication 1, pour maintenir ou améliorer la blancheur de l'article.

3. Utilisation selon la revendication 1 ou 2, dans laquelle le polypeptide ayant une activité de DNase est utilisé pour le nettoyage ou le lessivage de l'article au moins une fois avant le processus de nettoyage ou lessivage ultérieur dans lequel aucun polypeptide ayant une activité de DNase n'est présent.

4. Utilisation selon la revendication 3, dans laquelle le polypeptide ayant une activité de DNase est utilisé pour le nettoyage ou le lessivage de l'article au moins deux fois, trois fois, quatre fois, cinq fois, six fois, sept fois, huit fois, neuf fois ou dix fois avant le processus de nettoyage ou lessivage ultérieur dans lequel aucun polypeptide ayant une activité de DNase n'est présent.

5. Utilisation selon l'une quelconque des revendications 1 à 4, dans laquelle le polypeptide ayant une activité de DNase a une identité de séquence d'au moins 80 % avec le polypeptide mature de SEQ ID NO : 2 ou de SEQ ID NO : 3.

6. Utilisation selon la revendication 5, dans laquelle le polypeptide ayant une activité de DNase a une identité de séquence d'au moins 90 % avec le polypeptide mature de SEQ ID NO : 2 ou de SEQ ID NO : 3.

7. Utilisation selon l'une quelconque des revendications 1 à 4, dans laquelle le polypeptide ayant une activité de DNase est un variant du polypeptide mature de SEQ ID NO : 2 ou de SEQ ID NO : 3 ayant une substitution, une délétion et/ou une insertion au niveau de 1, 2, 3, 4, 5, 6, 7, 8, 9 ou 10 positions.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011098579 A
- WO 2014087011 A
- WO 9901534 A
- WO 9724177 A
- EP 14164424
- EP 14164429
- WO 9517413 A
- WO 9522625 A
- US 5223409 A
- WO 9206204 A
- WO 9219709 A
- WO 9219708 A
- US 3426 A
- US 011 A, Parmerter
- US 3453257 A
- US 3453258 A
- US 3453259 A
- US 3453260 A, Parmerter
- US 3459731 A, Gramera
- US 3553191 A, Parmerter
- US 3565887 A, Parmerter
- US 4535152 A, Szejtli
- US 4616008 A, Hirai
- US 4678598 A, Ogino
- US 4638058 A, Brandt
- US 4746734 A, Tsuchiyama
- US 4009 A
- US 253 A
- US 4187 A
- US 251 A
- US 4719 A
- US 105 A
- US 5441 A
- US 727 A
- US 5861 A
- US 371 A
- US 5676163 A
- WO 0049120 A
- WO 0107095 A
- JP 02284997 A
- JP 04030855 B
- US 4325939 A
- US 4469674 A
- US 6358469 A
- US 6015547 A
- US 3172817 A, Leupold
- WO 2012097034 A
- WO 09102854 A
- US 5977053 A
- WO 9817767 A
- EP 624154 A
- WO 2007087258 A
- WO 2007087244 A
- WO 2007087259 A
- EP 1867708 A
- WO 2007087242 A
- WO 2006130575 A
- WO 200503274 A
- WO 200503275 A
- WO 200503276 A
- EP 1876226 A
- WO 2007087257 A
- WO 2007087243 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- WO 99001544 A
- WO 2002099091 A
- WO 2001062903 A
- US 7262042 B
- WO 09021867 A
- WO 8906279 A
- WO 9318140 A
- WO 92175177 A
- WO 01016285 A
- WO 02026024 A
- WO 02016547 A
- WO 8906270 A
- WO 9425583 A
- WO 05040372 A
- WO 05052161 A
- WO 05052146 A
- WO 9523221 A
- WO 9221760 A

- EP 1921147 A
- EP 1921148 A
- WO 07044993 A
- WO 9219729 A
- WO 96034946 A
- WO 9820115 A
- WO 9820116 A
- WO 99011768 A
- WO 0144452 A
- WO 03006602 A
- WO 0403186 A
- WO 04041979 A
- WO 07006305 A
- WO 11036263 A
- WO 11036264 A
- US 5352604 A
- EP 258068 A
- EP 305216 A
- WO 9613580 A
- EP 218272 A
- EP 331376 A
- WO 9506720 A
- WO 9627002 A
- WO 9612012 A
- WO 10065455 A
- WO 10107560 A
- US 5389536 A
- WO 11084412 A
- WO 11084417 A
- WO 11084599 A
- WO 11150157 A
- WO 12137147 A
- EP 407225 A
- WO 9205249 A
- WO 9401541 A
- WO 9425578 A
- WO 9514783 A
- WO 9530744 A
- WO 9535381 A
- WO 9522615 A
- WO 9600292 A
- WO 9704079 A
- WO 9707202 A
- WO 0034450 A
- WO 0060063 A
- WO 0192502 A
- WO 0787508 A
- WO 09109500 A
- WO 10111143 A
- WO 0556782 A
- WO 0967279 A
- WO 10100028 A
- GB 1296839 A
- WO 9510603 A
- WO 9402597 A
- WO 9418314 A
- WO 9743424 A
- WO 99019467 A
- WO 02010355 A
- WO 2006066594 A
- WO 96023873 A
- WO 08153815 A
- WO 0166712 A
- WO 09061380 A
- WO 2011098531 A
- WO 2013001078 A
- WO 2013001087 A
- EP 179486 A
- WO 9324618 A
- WO 9510602 A
- WO 9815257 A
- WO 9527046 A
- WO 9704102 A
- WO 0179459 A
- WO 0179458 A
- WO 0179461 A
- WO 0179460 A
- WO 9201046 A
- JP 2238885 A
- WO 9708325 A
- WO 9533836 A
- US 4106991 A
- US 4661452 A
- GB 1483591 A
- EP 238216 A
- WO 2009087523 A
- WO 2007138054 A
- WO 2006108856 A
- WO 2006113314 A
- EP 1867808 A
- WO 2003040279 A
- EP 2169040 A
- US 20090011970 A1
- WO 2013188331 A
- WO 2014032269 A

**Non-patent literature cited in the description**


- **MUNK et al.** Microbial survival and odor in laundry. *Journal of Surfactants and Detergents*, October 2001, vol. 4 (4), 385-394
- **AMBERG et al.** Odour formation on textiles - Why do textiles accumulate Malodour?. *8th International Fresenius Conference 'Detergents and Cleaning products*, February 2014
- **NEEDLEMAN** ; **WUNSCH**. *J. Mol. Biol.*, 1970, vol. 48, 443-453

- **RICE et al.** EMBOSS: The European Molecular Biology Open Software Suite. *Trends Genet.*, 2000, vol. 16, 276-277
- **RICE et al.** *EM-BOSS: The European Molecular Biology Open Software Suite*, 2000
- **SAMBROOK et al.** Molecular Cloning, A Laboratory Manual. Cold Spring Harbor, 1989
- **H. NEURATH** ; **R.L. HILL**. The Proteins. Academic Press, 1979
- **CUNNINGHAM** ; **WELLS**. *Science*, 1989, vol. 244, 1081-1085
- **HILTON et al.** *J. Biol. Chem.*, 1996, vol. 271, 4699-4708
- **DE VOS et al.** *Science*, 1992, vol. 255, 306-312
- **SMITH et al.** *J. Mol. Biol.*, 1992, vol. 224, 899-904
- **WLODAVER et al.** *FEBS Lett.*, 1992, vol. 309, 59-64
- **REIDHAAR-OLSON** ; **SAUER**. *Science*, 1988, vol. 241, 53-57
- **BOWIE** ; **SAUER**. *Proc. Natl. Acad. Sci. USA*, 1989, vol. 86, 2152-2156
- **LOWMAN et al.** *Biochemistry*, 1991, vol. 30, 10832-10837
- **DERBYSHIRE et al.** *Gene*, 1986, vol. 46, 145
- **NER et al.** *DNA*, 1988, vol. 7, 127
- **NESS et al.** *Nature Biotechnology*, 1999, vol. 17, 893-896
- **COOPER et al.** *EMBO J.*, 1993, vol. 12, 2575-2583
- **DAWSON et al.** *Science*, 1994, vol. 266, 776-779
- **MARTIN et al.** *J. Ind. Microbiol. Biotechnol.*, 2003, vol. 3, 568-576
- **SVETINA et al.** *J. Biotechnol.*, 2000, vol. 76, 245-251
- **RASMUSSEN-WILSON et al.** *Appl. Environ. Microbiol.*, 1997, vol. 63, 3488-3493
- **WARD et al.** *Biotechnology*, 1995, vol. 13, 498-503
- **CONTRERAS et al.** *Biotechnology*, 1991, vol. 9, 378-381
- **EATON et al.** *Biochemistry*, 1986, vol. 25, 505-512
- **COLLINS-RACIE et al.** *Biotechnology*, 1995, vol. 13, 982-987
- **CARTER et al.** *Proteins: Structure, Function, and Genetics*, 1989, vol. 6, 240-248
- **STEVENS**. *Drug Discovery World*, 2003, vol. 4, 35-48
- **F. DIEDAINI-PILARD** ; **B. PERLY**. Optimal Performances with Minimal Chemical Modification of Cyclodextrins. *The 7th International Cyclodextrin Symposium Abstracts*, April 1994, 49
- **V. T. D'SOUZA** ; **K. B. LIPKOWITZ**. CHEMICAL REVIEWS: CYCLODEXTRINS. American Chemical Society, July 1998, vol. 98
- **STEFFEN ARCTANDER**. *Perfume and Flavor Chemicals (Aroma Chemicals)*, 1969
- **A. LEO**. Comprehensive Medicinal Chemistry. Pergamon Press, 1990, vol. 4, 295
- **HODGDON** ; **KALER**. *Current Opinion in Colloid & Interface Science*, 2007, vol. 12, 121-128
- **SIEZEN et al.** *Protein Engng.*, 1991, vol. 4, 719-737
- **SIEZEN et al.** *Protein Science*, 1997, vol. 6, 501-523
- Powdered Detergents, Surfactant science. Marcel Dekker, Inc, vol. 71